(19) Europäisches Patentamt
European Patent Office
Office européen des brevets

(11) EP 4 729 616 A1

(12) EUROPEAN PATENT APPLICATION
published in accordance with Art. 153(4) EPC

(43) Date of publication:
**22.04.2026 Bulletin 2026/17**

(21) Application number: **24823460.1**

(22) Date of filing: **14.06.2024**

(51) International Patent Classification (IPC):
***C12N 15/113*** (2010.01) ***A61K 31/7088*** (2006.01)
***A61K 47/54*** (2017.01) ***A61K 47/60*** (2017.01)
***A61K 47/61*** (2017.01) ***A61K 47/64*** (2017.01)
***A61K 47/68*** (2017.01) ***A61K 48/00*** (2006.01)
***A61P 21/00*** (2006.01) ***A61P 25/00*** (2006.01)
***A61P 25/14*** (2006.01) ***A61P 25/18*** (2006.01)
***A61P 43/00*** (2006.01)

(52) Cooperative Patent Classification (CPC):
**A61K 31/7088; A61K 47/54; A61K 47/60; A61K 47/61; A61K 47/64; A61K 47/68; A61K 48/00; A61P 21/00; A61P 25/00; A61P 25/14; A61P 25/18; A61P 43/00; C12N 15/113**

(86) International application number:
**PCT/JP2024/021613**

(87) International publication number:
**WO 2024/257847 (19.12.2024 Gazette 2024/51)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **16.06.2023 JP 2023099439**

(71) Applicants:
- **Sumitomo Pharma Co., Ltd.**
  **Osaka-shi, Osaka 541-0045 (JP)**
- **Luxna Biotech Co., Ltd.**
  **Suita-shi, Osaka 565-0871 (JP)**

(72) Inventors:
- **AOYAMA, Tomohiko**
  **Osaka-shi, Osaka 554-0022 (JP)**
- **ASANO, Shigehiro**
  **Osaka-shi, Osaka 554-0022 (JP)**
- **KAKUTANI, Tomomi**
  **Osaka-shi, Osaka 554-0022 (JP)**
- **KAWANOBE, Takaaki**
  **Suita-shi, Osaka 565-0871 (JP)**
- **SHRESTHA, Ajaya Ram**
  **Suita-shi, Osaka 565-0871 (JP)**
- **SUZUKI, Takao**
  **Suita-shi, Osaka 565-0871 (JP)**

(74) Representative: **J A Kemp LLP**
**80 Turnmill Street**
**London EC1M 5QU (GB)**

Remarks:
The complete document including Reference Table(s) and the Sequence Listing(s) can be downloaded from the EPO website

# (54) ANTISENSE OLIGONUCLEOTIDE CAPABLE OF REGULATING EXPRESSION AND/OR FUNCTION OF RPS25 GENE

(57) A single-stranded antisense oligonucleotide, or a pharmaceutically acceptable salt thereof, capable of modulating expression and/or function of RPS25 gene, wherein nucleosides of the single-stranded antisense oligonucleotide are bonded to each other via a phosphate group and/or a modified phosphate group, the single-stranded antisense oligonucleotide includes a gap region, a 3' wing region bonded to a 3' end of the gap region, and a 5' wing region bonded to a 5' end of the gap region, the gap region is a deoxyribose-based nucleic acid , which may contain a nucleic acid having a modified sugar moiety, the gap region includes at least one 5'-CP nucleic acid, each of the 3' wing region and the 5' wing region is a modified nucleic acid having a substituent at 2' position, the single-stranded antisense oligonucleotide is composed of 12 to 30 bases, and a base sequence of the



EP 4 729 616 A1

single-stranded antisense oligonucleotide is: a base sequence with a sequence identity of 90% to 100% to a base sequence complementary to at least one target region having the same base length as the single-stranded antisense oligonucleotide present in a base sequence as set forth in SEQ ID NO: 1; a base sequence complementary to a base sequence of the target region with deletion, substitution, insertion, or addition of one or several bases; or a base sequence capable of hybridizing under stringent conditions with an oligonucleotide having the target region.

FIG.1

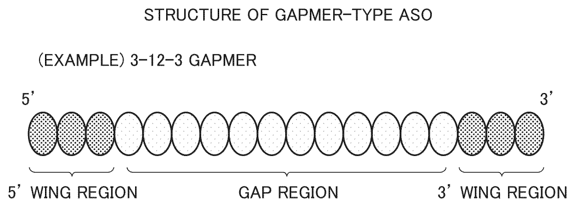

## Description

TECHNICAL FIELD

The present invention relates to an antisense oligonucleotide capable of modulating expression and/or function of RPS25 gene, as well as to an agent for modulating expression and/or function of RPS25 gene comprising the same.

BACKGROUND ART

Ribosomal Protein S25 (RPS25) gene is a gene that codes for one of the constituent proteins of ribosomal 40S subunit. The constituent protein (RPS25 protein) coded for by RPS25 gene has already been determined in terms of its conformation when it is present in the ribosome 40S subunit. (NPL 1)

RPS25 protein plays a role in protein synthesis process by controlling translation by means of binding to an RNA element that is capable of initiating cap-independent translation. The RNA element to which RPS25 protein binds is called Internal Ribosome Entry Site (IRES). IRES is one of the cap-independent translation mechanisms often found in viruses in particular. (NPL 2)

Repeat associated non-ATG translation (RAN translation) was first reported in a patient with spinocerebellar ataxia type 8 in 2011 (NPL 3). RAN translation refers to a mechanism where repeat expansion of a particular sequence brings about ATG-independent translation into a protein (such as a dipeptide repeat (DPR)). After this, more reports followed, suggesting the involvement of RAN translation in a plurality of repeat diseases (diseases caused by repeat expansion of a particular gene sequence) including amyotrophic lateral sclerosis (ALS) with mutation in C9orf72 gene (which may also be expressed as "C9orf72 ALS" hereinafter), Huntington's disease, and myotonic dystrophy. Many research were conducted to investigate the correlation between the DPR produced by RAN translation and the pathology, and it was reported that DPR removal was effective for alleviating the pathology. (NPL 4, NPL 5)

In 2019, a report identified RPS25 protein as a molecule that significantly contributes to the dipeptide repeat production attributable to RAN translation. RPS25 gene knockdown reduced RAN-translation-dependent DPR production attributable to GGGGCC repeats or CAG repeats. The GGGGCC repeat is known as abnormal expansion mutation of C9orf72 gene, a familial mutation in amyotrophic lateral sclerosis. The CAG repeat is known as abnormal expansion mutation of huntingtin gene and ATXN2 gene. In motor neurons derived from induced pluripotent stem cells (iPSC) established from a patient with C9orf72 gene mutation, it was demonstrated that RPS25 gene knockdown reduced DPR production attributable to GGGGCC repeats and also reduced death of the motor neurons. (NPL 6)

The antisense oligonucleotide for RPS25 gene disclosed by NPL 6 is a gapmer that has its wing moiety modified with 2'-O-methylated RNA (2'-OMe nucleic acid) and also has phosphorothioate bonds between its nucleosides. The base sequence of the antisense oligonucleotide includes a partial base sequence of the sense strand of the RPS25 gene.

The inventors of the present application conducted intensive research and reported a single-stranded antisense oligonucleotide or a pharmaceutically acceptable salt thereof capable of binding to RPS25 gene and effectively modulating the expression of the RPS25 gene. (PTL 7)

Antisense oligonucleotides generally referred to as gapmers are compounds that have a gap region composed of deoxyribose as well as a wing region having modified nucleic acids developed for the purpose of enhancing nuclease resistance and improving binding affinity and specificity etc. for target RNA, and a gapmer exhibits its effect by hybridizing with a target RNA sequence and inhibiting the gene expression. To ensure clinical application of gapmers, various modified nucleic acids have been developed, and many compounds (gapmers) containing such modified nucleic acids have been developed and put on the market. Nucleic acid drugs containing a gapmer, due to its nature, are known to accumulate in some organs such as the liver at the time of systemic exposure; in the course of research and development, issues related to toxicity caused by nucleic acid drugs such as hepatotoxicity came to attention, and efforts have been made to address these issues (attempts to elucidate the mechanism, and/or search for ways to avoid toxicity, for example). On the other hand, potential toxicity of gapmers due to topical administration that can occur at sites of high exposure such as eyes, brain, and skin is not fully understood, and issues are left to be addressed such as elucidation of the mechanism of toxicity manifestation and development of methods for avoiding toxicity.

Toxicity of antisense oligonucleotides can be classified into so-called "toxicity due to hybridization with non-target RNA (off-target toxicity in a narrow sense)" and "toxicity not caused by hybridization with RNA (off-target toxicity in a broad sense)", and to avoid such toxicity, various approaches have been adopted. As for off-target toxicity in a broad sense, extensive research has been conducted aiming at toxicity in the liver where, as mentioned above, the substance can be accumulated. In recent years, antisense oligonucleotides for intrathecal injection intended for treating central nervous system diseases have been actively developed, and new issues have emerged related to toxicity to the central nervous system (which may also be called "central nervous system toxicity" hereinafter).

Regarding the relationship between antisense oligonucleotides and central toxicity, it is known that symptoms related to the central nervous system such as convulsion can be observed within one hour after exposure of the central

nervous system, and correlation between changes of intracellular free calcium concentrations in neurons and central toxicity is disclosed. (PTL 8, NPL 7) Moreover, there is a paper that assessed central toxicity attributable to phosphorothioate modification in antisense oligonucleotides, and it discloses that central toxicity was reduced by replacing phosphorothioate bonds in the wing portions of a phosphorothioate-modified gapmer with phosphodiester bonds. (NPL 8)

Regarding central toxicity observed immediately after exposure of the central nervous system to antisense oligonucleotides (which may also be called "acute central nervous system toxicity" hereinafter; which is also expressed as "in vivo acute neuroTox" in the present specification), PTL 9 demonstrates that the central toxicity can be reduced when the modified nucleic acid has a substituent at 5' carbon atom of the sugar moiety of its nucleoside. Besides acute central toxicity, another type of central toxicity is known, which appears after a certain time period following administration (which may also be called "delayed central toxicity" hereinafter; which is also expressed as "in vivo delayed neuroTox" in the present specification); on the day following exposure of the central nervous system, or later, symptoms such as a decrease in locomotor activity, abnormal gait and abnormal hindlimb function, tremor, weakness in hindlimbs and tail, loss of hindlimb reflex, and body weight loss are observed. To the knowledge of the inventors, there are no specific solutions reported for delayed central toxicity.

CITATION LIST

PATENT LITERATURE

PTL 1: International Patent Laying-Open No. WO 2019/084068
PTL 2: International Patent Laying-Open No. WO 2011/052436
PTL 3: International Patent Laying-Open No. WO 2014/046212
PTL 4: International Patent Laying-Open No. WO 2015/125783
PTL 5: International Patent Laying-Open No. WO 2020/158910
PTL 6: International Patent Laying-Open No. WO 99/14226
PTL 7: International Patent Laying-Open No. WO 2022/097727
PTL 8: International Patent Laying-Open No. WO 2016/127000
PTL 9: International Patent Laying-Open No. WO 2022/234855


NON PATENT LITERATURE

NPL 1: Science (2011) 331 (6018):730-736
NPL 2: Genes Dev. (2009) 23 (23):2753-2764
NPL 3: Proc. Natl. Acad. Sci. USA (2011) 108 (1):260-265
NPL 4: Neuron (2015) 88:667-677
NPL 5: Neuron (2020) 105:645-662
NPL 6: Nat. Neurosci. (2019) 22 (9):1383-1388
NPL 7: Mol. Ther. Nucleic Acids (2023) 31:182-196
NPL 8: BioRxiv.doi:https://doi.org/10.1101/2021.02.14.431096 (2021)
NPL 9: Mol. Gen. Genet. (1979) 169:1-6
NPL 10: Curr. Opin. Struct. Biol. (2014) 24:165-169
NPL 11: J. Med. Chem. (2016) 59:9645-9667
NPL 12: Nature (2015) 518 (7539):409-412


SUMMARY OF INVENTION

TECHNICAL PROBLEM

Although PTL 1 recites that RPS25 gene inhibition reduces DPR production, it is unclear whether the same effect

can be exhibited by an approach which uses a nucleic acid such as an antisense nucleic acid. In addition, the RPS25 gene expression-reducing action of the antisense oligonucleotide recited by NPL 5 is limited. In PTL 7, the inventors of the present invention reported a single-stranded antisense oligonucleotide or a pharmaceutically acceptable salt thereof capable of effectively modulating the expression of RPS25 gene, but for pharmaceutical application, it is necessary to develop RPS25 gene antisense oligonucleotides with reduced toxicity (for example, delayed central toxicity).

The present invention has been devised in light of the above-described circumstances, and an objective of the present invention is to provide a single-stranded antisense oligonucleotide that is capable of modulating expression and/or function of RPS25 gene and reduce delayed central toxicity, as well as an agent for modulating expression and/or function of RPS25 gene comprising the same.

SOLUTION TO PROBLEM

The inventors of the present invention have conducted intensive research to achieve the above objective and, as a result, have found that it is possible to reduce delayed central toxicity by placing a modified nucleic acid having a cyclopropane ring at 5' position of the sugar moiety of the nucleoside in the gap region of a single-stranded antisense oligonucleotide or a pharmaceutically acceptable salt thereof (which may also be called "the antisense oligonucleotide according to the present invention" hereinafter) capable of binding to RPS25 gene and effectively modulating the expression of the RPS25 gene. Thus, the present invention has been completed. In the following, more specific description of the present invention will be given.

[1] An antisense oligonucleotide according to a first aspect of the present invention is a single-stranded antisense oligonucleotide, or a pharmaceutically acceptable salt thereof, capable of modulating expression and/or function of RPS25 gene, wherein

nucleosides of the single-stranded antisense oligonucleotide are bonded to each other via a phosphate group and/or a modified phosphate group,
the single-stranded antisense oligonucleotide includes a gap region, a 3' wing region bonded to a 3' end of the gap region, and a 5' wing region bonded to a 5' end of the gap region,
the gap region is a deoxyribose-based nucleic acid, which may contain a nucleic acid having a modified sugar moiety,
the gap region includes at least one 5'-CP nucleic acid,
the 3' wing region and the 5' wing region are modified nucleic acids having a substituent at the 2' position,
the single-stranded antisense oligonucleotide is composed of 12 to 30 bases, and
a base sequence of the single-stranded antisense oligonucleotide is:

a base sequence with a sequence identity of 90% to 100% to a base sequence complementary to at least one target region having the same base length as the single-stranded antisense oligonucleotide present in a base sequence as set forth in SEQ ID NO: 1;
a base sequence complementary to a base sequence of the target region with deletion, substitution, insertion, or addition of one or several bases; or
a base sequence capable of hybridizing under stringent conditions with an oligonucleotide having the target region.

[2] An antisense oligonucleotide according to a second aspect of the present invention is a single-stranded antisense oligonucleotide, or a pharmaceutically acceptable salt thereof, capable of modulating expression and/or function of RPS25 gene, wherein

nucleosides of the single-stranded antisense oligonucleotide are bonded to each other via a phosphate group and/or a modified phosphate group,
the single-stranded antisense oligonucleotide includes a gap region, a 3' wing region bonded to a 3' end of the gap region, and a 5' wing region bonded to a 5' end of the gap region,
the gap region is a deoxyribose-based nucleic acid, which may contain a nucleic acid having a modified sugar moiety,
the gap region includes at least one 5'-CP nucleic acid,
the 3' wing region and the 5' wing region are modified nucleic acids having a substituent at 2' position,
the single-stranded antisense oligonucleotide is composed of 12 to 30 bases, and
a base sequence of the single-stranded antisense oligonucleotide is:

a base sequence with a sequence identity of 90% to 100% to a base sequence complementary to at least one target region having the same base length as the single-stranded antisense oligonucleotide present in a base sequence as set forth in SEQ ID NO: 1;
a base sequence complementary to a base sequence of the target region with deletion, substitution, insertion, or addition of one or several bases; or
a base sequence capable of hybridizing under stringent conditions with an oligonucleotide having the target region

(except for single-stranded antisense oligonucleotides of Reference Examples 1 to 31).

[3] An antisense oligonucleotide according to a third aspect of the present invention is a single-stranded antisense oligonucleotide, or a pharmaceutically acceptable salt thereof, capable of modulating expression and/or function of RPS25 gene, wherein

nucleosides of the single-stranded antisense oligonucleotide are bonded to each other via a phosphate group and/or a modified phosphate group,
the single-stranded antisense oligonucleotide includes a gap region, a 3' wing region bonded to a 3' end of the gap region, and a 5' wing region bonded to a 5' end of the gap region,
the gap region is a deoxyribose-based nucleic acid , which may contain a nucleic acid having a modified sugar moiety,
the gap region includes at least one 5'-CP nucleic acid,
each of the 3' wing region and the 5' wing region is a modified nucleic acid having a substituent at 2' position,
the single-stranded antisense oligonucleotide is composed of 12 to 30 bases, and
a base sequence of the single-stranded antisense oligonucleotide is:

a base sequence with a sequence identity of 90% to 100% to a base sequence complementary to at least one target region having the same base length as the single-stranded antisense oligonucleotide present in a base sequence as set forth in SEQ ID NO: 1;
a base sequence complementary to a base sequence of the target region with deletion, substitution, insertion, or addition of one or several bases; or
a base sequence capable of hybridizing under stringent conditions with an oligonucleotide having the target region

(except for single-stranded antisense oligonucleotides of Reference Examples 1 to 31 and Design Examples 1 to 18).

The antisense oligonucleotide according to the present invention has modified nucleic acids in the gap region, the 5' wing region, and the 3' wing region. The gap region includes at least one 5'-cyclopropyl nucleic acid (5'-CP nucleic acid). As the modified nucleic acid having a substituent at 2' position that is placed in the 5' wing region and the 3' wing region, when it is a non-bridged modified nucleic acid, it is 2'-O-methoxyethyl nucleic acid (2'-MOE nucleic acid), 2'-O-methyl nucleic acid (2'-OMe nucleic acid), and/or 2'-O-(2-N-methylcarbamoyl)ethyl nucleic acid (MCE), and when it is a bridged modified nucleic acid, it is 2',4'-Bridged Nucleic Acid/Locked Nucleic Acid (2',4'-BNA/LNA; hereinafter Locked Nucleic Acid may also be called "LNA"), Amido-bridged nucleic acid (AmNA), Guanidino-bridged nucleic acid (GuNA), and/or 2'-O,4'-C-Spirocyclopropylene bridged nucleic acid (scpBNA); preferably, at least one of these modified nucleic acids is included. With this configuration, the antisense oligonucleotide according to the present invention is expected to have a high binding affinity for RPS25 mRNA or mRNA precursor while being reduced in delayed central toxicity.
In addition, the antisense oligonucleotide according to the present invention, which is a so-called gapmer-type single-stranded antisense oligonucleotide, functions as a catalyst in RNase-driven RPS25 gene degradation reaction, which is described below. Because of this, administration of only a small amount is expected to exhibit a certain level of sustained effect.

[4] In any one of [1] to [3] above, the base sequence of the single-stranded antisense oligonucleotide is:
a base sequence with a sequence identity of 95% to 100% to a base sequence complementary to at least one target region having the same base length as the single-stranded antisense oligonucleotide present in the base sequence as set forth in SEQ ID NO: 1.
[5] In any one of [1] to [4] above, the base sequence of the single-stranded antisense oligonucleotide is:
a base sequence complementary to at least one target region having the same base length as the single-stranded antisense oligonucleotide present in the base sequence as set forth in SEQ ID NO: 1.

[6] In any one of [1] to [5] above,

the gap region is composed of 5 to 20 bases,
the 3' wing region is a modified nucleic acid composed of 3 to 5 bases having a substituent at 2' position, and
the 5' wing region is a modified nucleic acid composed of 3 to 5 bases having a substituent at 2' position.

[7] In any one of [1] to [6] above, the 5'-CP nucleic acid is at least located at position 2 counted from the 5' end of the gap region.
[8] In any one of [1] to [7] above, the 5'-CP nucleic acid comprises two or more 5'-CP nucleic acids in the gap region.
[9] In any one of [1] to [8] above, the 5'-CP nucleic acid comprises two to five 5'-CP nucleic acids in the gap region.
[10] In any one of [1] to [7] above, the 5'-CP nucleic acid occupies one ninth or more of the gap region.
[11] In [10] above, the 5'-CP nucleic acid occupies one fifth or more of the gap region.
[12] In any one of [1] to [11] above, the 5'-CP nucleic acid comprises consecutive two to four 5'-CP nucleic acids located at at least one position in the gap region.
[13] In any one of [1] to [12] above, the 5'-CP nucleic acid is located on the 5' end side in the gap region.
[14] In any one of [1] to [13] above, the 5'-CP nucleic acid is located on the 5' end side and the 3' end side in the gap region.
[15] In any one of [1] to [14] above, the single-stranded antisense oligonucleotide is composed of 15 to 20 bases.
[16] In any one of [1] to [15] above, the single-stranded antisense oligonucleotide is composed of 18 to 20 bases.
[17] In any one of [1] to [16] above,

the modified nucleic acid having a substituent at 2' position in the 3' wing region includes at least one selected from the group consisting of 2'-MOE nucleic acid, LNA, AmNA, GuNA, and scpBNA, and
the modified nucleic acid having a substituent at 2' position in the 5' wing region includes at least one selected from the group consisting of 2'-MOE nucleic acid, LNA, AmNA, GuNA, and scpBNA.

[18] In any one of [1] to [17] above,

the modified nucleic acid having a substituent at 2' position in the 3' wing region is composed of a modified nucleic acid selected from the group consisting of 2'-MOE nucleic acid, LNA, AmNA, GuNA, and scpBNA, and
the modified nucleic acid having a substituent at 2' position in the 5' wing region is composed of a modified nucleic acid selected from the group consisting of 2'-MOE nucleic acid, LNA, AmNA, GuNA, and scpBNA.

[19] In any one of [1] to [18] above,

the modified nucleic acid having a substituent at 2' position in the 3' wing region is composed of a modified nucleic acid selected from the group consisting of 2'-MOE nucleic acid, AmNA, GuNA, and scpBNA, and
the modified nucleic acid having a substituent at 2' position in the 5' wing region is composed of a modified nucleic acid selected from the group consisting of 2'-MOE nucleic acid, AmNA, GuNA, and scpBNA.

[20] In any one of [1] to [19] above, at least one nucleoside-nucleoside bond in the single-stranded antisense oligonucleotide is a phosphorothioate bond.
[21] In any one of [1] to [20] above, at least one nucleoside-nucleoside bond in the single-stranded antisense oligonucleotide is a phosphodiester bond.
[22] In any one of [1] to [21] above, a proportion of phosphorothioate bonds among all of nucleoside-nucleoside bonds in the single-stranded antisense oligonucleotide is from 50% to 80%.
[23] In [22] above, a proportion of phosphorothioate bonds among all of nucleoside-nucleoside bonds in the single-stranded antisense oligonucleotide is from 50% to 70%.
[24] In any one of [1] to [23] above, in the gap region, a bond between the 5'-CP nucleic acid and a nucleoside located adjacent to a 3' end of the 5'-CP nucleic acid is a phosphorothioate bond (except for a case where the 5'-CP nucleic acid is located at the 3' end of the gap region).
[25] In any one of [1] to [24] above, in the gap region, a bond between the 5'-CP nucleic acid and a nucleoside located adjacent to a 3' end of the 5'-CP nucleic acid is a phosphorothioate bond (except for a case where the 5'-CP nucleic acid is located at the 3' end of the gap region and a case where the nucleoside located adjacent to the 3' end of the 5'-CP nucleic acid is a 5'-CP nucleic acid).
[26] In any one of [1] to [25] above, in the gap region, a bond between the 5'-CP nucleic acid and a nucleoside located adjacent to a 5' end of the 5'-CP nucleic acid is a phosphodiester bond.
[27] In any one of [1] to [26] above, the base sequence of the single-stranded antisense oligonucleotide is:

a base sequence with a sequence identity of 90% to 100% to a base sequence complementary to a target region present in the base sequence as set forth in SEQ ID NO: 1 composed of consecutive 14 to 22 bases starting from a base located at one of position 123, position 124, position 185, position 186, position 263, position 264, position 324, position 325, position 442, position 443, and position 447 to position 453 counted from a 5' end;
a base sequence complementary to a base sequence of the target region with deletion, substitution, insertion, or addition of one or several bases; or
a base sequence capable of hybridizing under stringent conditions with an oligonucleotide having the target region.

[28] In any one of [1] to [27] above, the base sequence of the single-stranded antisense oligonucleotide is:

a base sequence with a sequence identity of 90% to 100% to a base sequence complementary to a target region present in the base sequence as set forth in SEQ ID NO: 1 composed of consecutive 14 to 22 bases starting from a base located at one of position 324, position 325, and position 448 to position 453 counted from a 5' end;
a base sequence complementary to a base sequence of the target region with deletion, substitution, insertion, or addition of one or several bases; or
a base sequence capable of hybridizing under stringent conditions with an oligonucleotide having the target region.

[29] In any one of [1] to [28] above,

the base sequence of the single-stranded antisense oligonucleotide is a base sequence with a sequence identity of 90% to 100% to a base sequence complementary to a target region present in the base sequence as set forth in SEQ ID NO: 1 composed of consecutive 18 to 22 bases starting from a base located at one of position 448 to position 453 counted from a 5' end,
the 3' wing region is composed of 3 to 5 bases, and
the 5' wing region is composed of 3 to 5 bases.

[30] In any one of [1] to [29] above, the base sequence of the single-stranded antisense oligonucleotide is a base sequence with a sequence identity of 90% to 100% to a base sequence complementary to a target region present in the base sequence as set forth in SEQ ID NO: 1 composed of consecutive 18 to 20 bases starting from a base located at one of position 450 to position 451 and position 453 counted from a 5' end.
[31] In any one of [1] to [30] above, the base sequence of the single-stranded antisense oligonucleotide is a base sequence selected from the group consisting of base sequences as set forth in SEQ ID NOs: 5 to 47 and 49 to 56.
[32] In any one of [1] to [31] above, the base sequence of the single-stranded antisense oligonucleotide is a base sequence selected from the group consisting of base sequences as set forth in SEQ ID NOs: 6, 9, 12, 15 to 18, 22, 24, 27 to 29, 31 to 36, 38, 40 to 42, 47, and 49 to 54.
[33] In any one of [1] to [32] above, the base sequence of the single-stranded antisense oligonucleotide is a base sequence selected from the group consisting of base sequences as set forth in SEQ ID NOs: 41, 47, 50, and 53 to 55.
[34] An antisense oligonucleotide complex according to the present invention is an antisense oligonucleotide complex, or a pharmaceutically acceptable salt thereof, comprising:

the single-stranded antisense oligonucleotide or a pharmaceutically acceptable salt thereof according to any one of [1] to [33] above; and
an additional substance bonded to the single-stranded antisense oligonucleotide, wherein
the additional substance is selected from the group consisting of a polyethylene glycol, a peptide, an alkyl chain, a nucleic acid, a ligand compound, an antibody, a protein, and a sugar chain.

[35] A pharmaceutical according to the present invention comprises the single-stranded antisense oligonucleotide or a pharmaceutically acceptable salt thereof according to any one of [1] to [33] above or the antisense oligonucleotide complex or a pharmaceutically acceptable salt thereof according to [34] above, as an active ingredient.
[36] In [35] above, the single-stranded antisense oligonucleotide or a pharmaceutically acceptable salt thereof or the antisense oligonucleotide complex or a pharmaceutically acceptable salt thereof is administered in such a manner that the central nervous system is exposed thereto.
[37] In [35] or [36] above, the single-stranded antisense oligonucleotide or a pharmaceutically acceptable salt thereof or the antisense oligonucleotide complex or a pharmaceutically acceptable salt thereof is administered to a subject susceptible to delayed central toxicity.
[38] An agent for modulating expression and/or function of RPS25 gene according to the present invention comprises

the single-stranded antisense oligonucleotide or a pharmaceutically acceptable salt thereof according to any one of [1] to [33] above or the antisense oligonucleotide complex or a pharmaceutically acceptable salt thereof according to [34] above, as an active ingredient.

[39] An agent for inhibiting dipeptide repeat production attributable to RAN translation according to the present invention comprises the single-stranded antisense oligonucleotide or a pharmaceutically acceptable salt thereof according to any one of [1] to [33] above or the antisense oligonucleotide complex or a pharmaceutically acceptable salt thereof according to [34] above, as an active ingredient.

[40] An agent for treating a repeat disease according to the present invention comprises the single-stranded antisense oligonucleotide or a pharmaceutically acceptable salt thereof according to any one of [1] to [33] above or the antisense oligonucleotide complex or a pharmaceutically acceptable salt thereof according to [34] above, as an active ingredient.

[41] An agent for preventing a repeat disease according to the present invention comprises the single-stranded antisense oligonucleotide or a pharmaceutically acceptable salt thereof according to any one of [1] to [33] above or the antisense oligonucleotide complex or a pharmaceutically acceptable salt thereof according to [34] above, as an active ingredient.

[42] In the treatment agent according to [40] above or the preventing agent according to [41] above, the repeat disease is at least one selected from the group consisting of C9orf72 ALS, C9orf72 FTLD, Huntington’s disease, spinocerebellar ataxia, dentatorubral-pallidoluysian atrophy, spinal and bulbar muscular atrophy, Friedreich ataxia, fragile X-associated tremor/ataxia syndrome, and myotonic dystrophy.

[43] The method of treating or preventing a repeat disease according to the present invention comprises administering the single-stranded antisense oligonucleotide or a pharmaceutically acceptable salt thereof according to any one of [1] to [33] above or the antisense oligonucleotide complex or a pharmaceutically acceptable salt thereof according to [34] above, to an individual.

[44] In the method of treating or preventing a repeat disease according to [43] above, the repeat disease is at least one selected from the group consisting of C9orf72 ALS, C9orf72 FTLD, Huntington’s disease, spinocerebellar ataxia, dentatorubral-pallidoluysian atrophy, spinal and bulbar muscular atrophy, Friedreich ataxia, fragile X-associated tremor/ataxia syndrome, and myotonic dystrophy.

[45] The present invention provides the single-stranded antisense oligonucleotide or a pharmaceutically acceptable salt thereof according to any one of [1] to [33] above or the antisense oligonucleotide complex or a pharmaceutically acceptable salt thereof according to [34] above, for use for treating or preventing C9orf72 ALS.

[46] The present invention provides the single-stranded antisense oligonucleotide or a pharmaceutically acceptable salt thereof according to any one of [1] to [33] above or the antisense oligonucleotide complex or a pharmaceutically acceptable salt thereof according to [34] above, for use for producing an agent for treating or preventing C9orf72 ALS.

## ADVANTAGEOUS EFFECTS OF INVENTION

The present invention makes it possible to provide a single-stranded antisense oligonucleotide that is capable of modulating expression and/or function of RPS25 gene and reduced in delayed central toxicity, as well as an agent for modulating expression and/or function of RPS25 gene comprising the same.

## BRIEF DESCRIPTION OF DRAWINGS

[Fig. 1] Fig. 1 is a schematic view of an example configuration of a single-stranded antisense oligonucleotide according to the present embodiment.

[Fig. 2] Fig. 2 is a schematic view describing the mechanism of reduction of RPS25 gene expression when the single-stranded antisense oligonucleotide according to the present embodiment is used.

## DESCRIPTION OF EMBODIMENTS

In the following, a description will be given of an embodiment of the present invention (which may also be expressed as "the present embodiment" hereinafter). It should be noted that the present embodiment is not limited to the description below. Herein, the expression "from I to J" means the upper limit and the lower limit of a range (that is, it means "not less than I and not more than J"). When I is not accompanied by unit expression and J is accompanied by unit expression, the unit of I is the same as the unit of J.

<<Single-Stranded Antisense Oligonucleotide Capable of Modulating Expression and/or Function of RPS25 Gene>>

A single-stranded antisense oligonucleotide according to the present embodiment is a single-stranded antisense oligonucleotide, or a pharmaceutically acceptable salt thereof, capable of modulating expression and/or function of RPS25 gene, wherein

nucleosides of the single-stranded antisense oligonucleotide are bonded to each other via a phosphate group and/or a modified phosphate group,
the single-stranded antisense oligonucleotide includes a gap region, a 3' wing region bonded to a 3' end of the gap region, and a 5' wing region bonded to a 5' end of the gap region,
the gap region is a deoxyribose-based nucleic acid , which may contain a nucleic acid having a modified sugar moiety,
the gap region includes at least one 5'-CP nucleic acid,
each of the 3' wing region and the 5' wing region is a modified nucleic acid having a substituent at 2' position,
the single-stranded antisense oligonucleotide is composed of 12 to 30 bases, and
a base sequence of the single-stranded antisense oligonucleotide is:

a base sequence with a sequence identity of 90% to 100% to a base sequence complementary to at least one target region having the same base length as the single-stranded antisense oligonucleotide present in a base sequence as set forth in SEQ ID NO: 1;
a base sequence complementary to a base sequence of the target region with deletion, substitution, insertion, or addition of one or several bases; or
a base sequence capable of hybridizing under stringent conditions with an oligonucleotide having the target region. In the following, a detailed description will be given.

<Definitions of Terms, etc.>

First, definitions and the like of terms used in the present specification are given below.

(RPS25 Gene)

Definition of "RPS25 gene" herein can be found in Mol. Gen. Genet. (1979) 169:1-6 (NPL 9) and Curr. Opin. Struct. Biol. (2014) 24:165-169 (NPL 10). Examples of synonyms of "RPS25" include 40S ribosomal protein S25, ribosomal protein S25, Small ribosomal subunit protein eS25, Rps25, 2810009D21Rik, ribosomal protein s25, Ribosomal Protein S25, S25, eS25, and ribosomal protein.

(Single-Stranded Antisense Oligonucleotide)

Herein, "single-stranded antisense oligonucleotide" or "antisense oligonucleotide" (which may also be called "ASO" hereinafter) means an oligonucleotide that is complementary to mRNA, mRNA precursor, or ncRNA (noncoding RNA) of the target gene (hereinafter, these three may also be collectively called "target RNA"), or a pharmacologically acceptable salt of the oligonucleotide. The antisense oligonucleotide is composed of DNA, RNA, and/or an analog thereof. The antisense oligonucleotide forms a double strand with the target mRNA, mRNA precursor, or ncRNA to reduce the action of the target mRNA, mRNA precursor, or ncRNA. The antisense oligonucleotide includes the following: one having a base sequence fully complementary to the base sequence of the target mRNA, mRNA precursor, or ncRNA; one having this complementary base sequence with deletion, substitution, insertion, or addition of one or several bases; and one including, in its base sequence, a base that is capable of forming a wobble base pair.

Moreover, the antisense oligonucleotide according to the present invention may further include, in addition to "a modified nucleic acid whose sugar moiety is modified sugar" (a modified nucleotide having sugar modification) described below, other modified nucleotides that are known in the field. Examples of the modified nucleotides that are known in the field, other than the modified nucleotides having sugar modification, include modified nucleotides having phosphate modification, modified nucleotides having nucleobase modification (both of which are described below), and the like.

The structure of either terminal of the antisense oligonucleotide according to the present embodiment is not particularly limited, and may be either -OH or -OR (where R represents an alkyl chain, a phosphate, or an additional substance described below), for example.

Moreover, the single-stranded antisense oligonucleotide according to the present embodiment may be in the form of a single strand, or may be hybridized with a second oligonucleotide (which is described below) to form a double strand. A double-stranded oligonucleotide that is composed of the single-stranded antisense oligonucleotide and the second oligonucleotide hybridized with the single-stranded antisense oligonucleotide may also be called "a double-stranded antisense oligonucleotide".

(Oligonucleotide)

Herein, "oligonucleotide" means a polymer of 2 to 30 nucleosides (which are the same as or different from each other) bonded to each other via phosphodiester bond or other bonds. Another way of understanding it is that the oligonucleotide is composed of a nucleobase moiety, a phosphate moiety, and a sugar moiety, as shown by a structural formula below.

[Chem. 1]

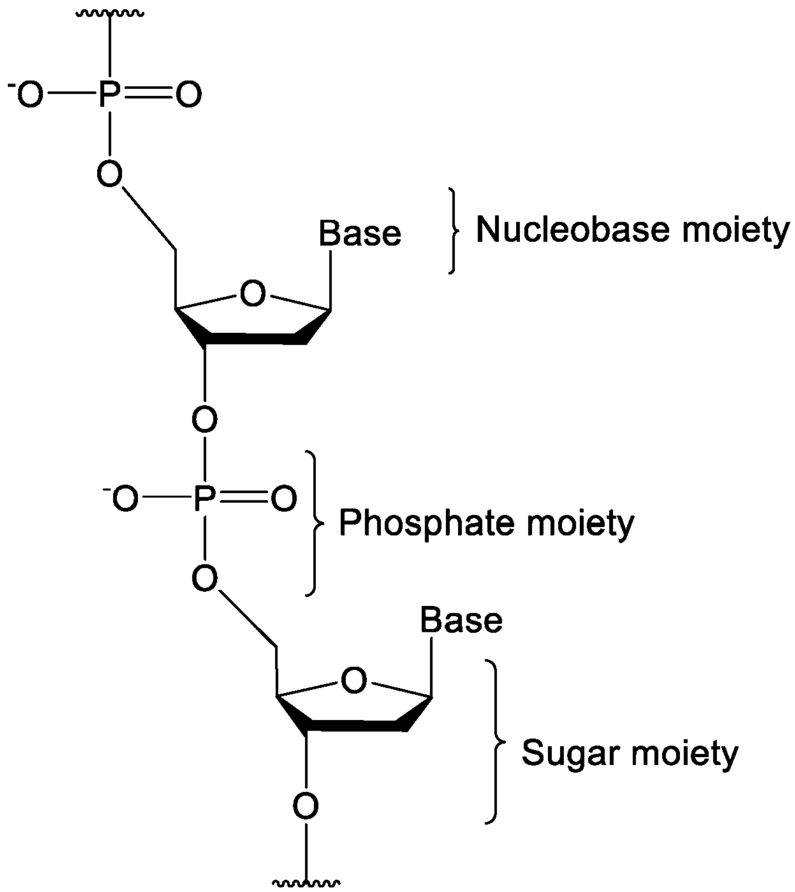


The oligonucleotide is broadly classified into natural oligonucleotide and non-natural oligonucleotide. "Natural oligonucleotide" means an oligonucleotide formed of naturally-occurring nucleotides. "Non-natural oligonucleotide" means an oligonucleotide that includes at least one modified nucleotide (which is described below) as a structural unit. Preferable examples of the "non-natural oligonucleotide" include sugar-modified derivatives in which the sugar moiety is modified; phosphorothioate derivatives in which one non-bridging oxygen atom of the phosphodiester bond is substituted with a sulfur atom; phosphorodithioate derivatives in which two non-bridging oxygen atoms of the phosphodiester bond are substituted with sulfur atoms; ester derivatives in which the phosphodiester bond is converted into the triester form; phosphoamide derivatives in which the phosphodiester bond is amidated; boranophosphate derivatives in which the phosphodiester bond is converted into the boronate ester form; alkylphosphonate (such as methylphosphonate or methoxypropylphosphonate, for example) derivatives in which a non-bridging oxygen atom of the phosphodiester bond is substituted with an alkyl group; amide derivatives in which the phosphodiester bond is substituted with an amide bond; and modified base derivatives in which the nucleobase is modified. Further preferable examples of the non-natural oligonucleotide include bridged sugar-modified derivatives in which the sugar moiety is modified; phosphorothioate derivatives in which one non-bridging oxygen atom of the phosphodiester bond is substituted with a sulfur atom; ester derivatives in which the phosphodiester bond is converted into the ester form; alkylphosphonated derivatives in which the sugar moiety is modified with modified sugar (which is described below) (such as bridged sugar, for example), and in which one non-bridging oxygen atom of the phosphodiester bond is substituted with a sulfur atom or a non-bridging oxygen atom of the phosphodiester bond is substituted with an alkyl group; and the like.

(Nucleoside)

Herein, "nucleoside" means a compound that is composed of a purine base or a pyrimidine base and a sugar which are bonded to each other. A naturally-occurring nucleoside may also be called "a natural nucleoside". A non-naturally-occurring modified nucleoside may also be called "a modified nucleoside". A modified nucleoside whose sugar moiety is modified, in particular, may also be called "a sugar-modified nucleoside".

(Nucleotide)

Herein, "nucleotide" means a compound that is composed of an above-defined nucleoside and a phosphate group bonded to the sugar. A naturally-occurring nucleotide may also be called "a natural nucleotide". A modified nucleotide that does not naturally occur may also be called "a modified nucleotide" or "a modified nucleic acid". Examples of the "modified nucleotide" or the "modified nucleic acid" include compounds that are composed of an above-defined modified nucleoside and a phosphate group bonded to the sugar, compounds that are composed of an above-defined modified nucleoside and a modified phosphate group (which is described below) bonded to the sugar, compounds that are composed of a natural nucleoside and a modified phosphate group (which is described below) bonded to the sugar, and the like.

(Sugar Modification, Modified Sugar)

Herein, "sugar modification" means modification to the sugar moiety of an above-defined nucleotide. A modified sugar moiety, in particular, may also be called "a modified sugar". A modified nucleotide with sugar modification can be used as a modified nucleic acid, and examples thereof include LNA, AmNA, GuNA, scpBNA, 2'-O-alkyl (such as 2'-O-methyl nucleic acid and 2'-MOE nucleic acid, for example), 2'-F, 5'-methyl-DNA, ENA (2'-O,4'-C-Ethylene-Bridged Nucleic Acid), S-cEt (2',4'-constrained Ethyl Nucleic Acid), 5'-CP nucleic acid (5'-cyclopropyl Nucleic Acid), and the like.

Examples of LNA include those that include structures represented by symbols "A (L)", "5 (L)", "G (L)", "T (L)" which are described below. Examples of AmNA include those that include structures represented by symbols "A (Y)", "5 (Y)", "G (Y)", "T (Y)" which are described below. Examples of GuNA include those that include structures represented by symbols "A (Gx)", "5 (Gx)", "G (Gx)", "T (Gx)" which are described below. Examples of scpBNA include those that include structures represented by symbols "A (S)", "5 (S)", "G (S)", "T (S)" which are described below. Examples of 2'-MOE nucleic acid include those that include structures represented by symbols "A (m)", "5 (m)", "G (m)", "T (m)" which are described below. Examples of 5'-CP nucleic acid include those that include structures represented by symbols "A (5'-CP)", "5 (5'-CP)", "G (5'-CP)", "T (5'-CP)" which are described below. Examples of 2'-OMe nucleic acid include those that include structures represented by symbols "A(M)", "5(M)", "G(M)", "U(M)", "T(M)" which are described below. Examples of MCE nucleic acid include those that include structures represented by symbols "A(Mx)", "5(Mx)", "G(Mx)", "U(Mx)" which are described below.

(Modified Nucleic Acid Having Substituent at 2' Position)

Herein, "modified nucleic acid having a substituent at 2' position " means a modified nucleic acid having a substituent at 2' position of the sugar moiety of an above-defined nucleotide. Examples of a non-bridged modified nucleic acid include 2'-O-alkyl (such as 2'-O-methyl nucleic acid, 2'-MOE nucleic acid, and MCE nucleic acid, for example), 2'-F, and the like, and examples of a bridged modified nucleic acid include LNA, AmNA, GuNA, scpBNA, ENA, S-cEt, and the like.

(Nucleotide Modification Known in Field Other Than Sugar Modification)

A nucleotide modification that is known in the field other than the above-defined sugar modification can be used for a modified nucleic acid that is to be used for producing the single-stranded antisense oligonucleotide according to the present invention. As nucleotide modifications of this type, phosphate modification and nucleobase modification are known, which are described below. Examples of the nucleotide modifications of this type include nucleotide modification described in W. Brad Wan et. Al. J. Med. Chem. (2016) 59:9645-9667. (NPL 11), for example. Such nucleotide modifications can be carried out based on a method known in the field described in a document cited by the above document.

(Phosphate Group)

Herein, "phosphate group" means a phosphate moiety of an above-defined nucleotide whose bonding is a

naturally-occurring phosphodiester bond (which is a bond represented by symbol "-" described below).

(Phosphate Modification, Modified Phosphate Group)

Herein, "phosphate modification" means modification to the phosphate moiety of an above-defined nucleotide. A modified phosphate moiety, in particular, may also be called "a modified phosphate group". Examples of the bonding that includes this modified phosphate group include a phosphorothioate bond (a bond represented by symbol "^" described below), a phosphorodithioate bond, a phosphoramidate bond (a bond represented by symbol "=" described below), a boranophosphate bond (a bond represented by symbol "×" described below), alkylphosphonate, and the like.

(Nucleobase Modification, Modified Nucleobase)

Herein, "nucleobase modification" means modification to the nucleobase moiety of an above-defined nucleotide. A modified nucleobase moiety, in particular, may also be called "a modified nucleobase". Examples of the modified nucleobase include 5-methylcytosine, 5-hydroxymethylcytosine, 5-propynylcytosine, and the like.

(DNA or RNA Analog)

The above-mentioned DNA or RNA analog means a molecule whose structure is similar to that of DNA or RNA. Examples thereof include peptide nucleic acids (pNA), morpholino nucleic acids, and the like.

(ncRNA)

Herein, "ncRNA" is a generic name for RNAs that are not involved in protein translation. Examples of this ncRNA include ribosomal RNA, transfer RNA, miRNA, Natural Antisense Transcript (NAT), and the like.

(Nucleobase Moiety of Oligonucleotide)

Examples of the nucleobase moiety of an above-defined oligonucleotide include thyminyl group, cytosinyl group, adeninyl group, guaninyl group, 5-methylcytosinyl group, uracilyl group, 2-oxo-4-hydroxy-5-methyl-1,2-dihydropyrimidin-1-yl group, 2-oxo-4-amino-1,2-dihydropyrimidin-1-yl group, 4-amino-5-methyl-2-oxo-1,2-dihydropyrimidin-1-yl group, 2-oxo-4-hydroxy -1 ,2-dihydropyrimidin-1-yl group, and the like. Preferable examples of the nucleobase moiety include thyminyl group, cytosinyl group, adeninyl group, guaninyl group, 5-methylcytosinyl group, uracilyl group, and the like. Among these nucleobases, uracil (U) and thymine (T) are interchangeable. Both uracil (U) and thymine (T) are capable of forming a base pair with adenine (A) in the complementary strand. Cytosine (C) and 5-methylcytosine (5(x)) are interchangeable, and both are capable of forming a base pair with guanine (G) in the complementary strand. The same is true for the nucleobase moiety of an antisense oligonucleotide.

(Target RNA)

Herein, "target RNA" means an RNA whose function is reduced by an above-defined single-stranded antisense oligonucleotide binding thereto. In other words, the target RNA according to the present embodiment means RPS25 mRNA and mRNA precursor. Examples of the target RNA include human RPS25 mRNA (which may also be called "hRPS25" hereinafter) having the base sequence as set forth in SEQ ID NO: 1, monkey RPS25 mRNA (which may also be called "cRPS25" hereinafter) having the base sequence as set forth in SEQ ID NO: 2, mouse RPS25 mRNA (which may also be called "mRPS25" hereinafter) having the base sequence as set forth in SEQ ID NO: 3, rat RPS25 mRNA (which may also be called "rRPS25") having the base sequence as set forth in SEQ ID NO: 4, and the like.

(Binding to Target RNA)

Herein, "binding to target RNA" means that the nucleobase of the single-stranded antisense oligonucleotide forms a double-stranded nucleic acid with the nucleobase of target RNA due to complementation to the target RNA. The double-stranded nucleic acid needs to be formed only with at least part of the target RNA. The strength of the bonding to the target RNA can be measured with the use of a thermal stability index, for example. Examples of the thermal stability index include the melting temperature (Tm value) of the double-stranded nucleic acid, and the like. The Tm value is preferably from 40 to 90°C, more preferably from 50 to 70°C.

(Target Region)

The above-mentioned target region means a region in RPS25 mRNA and mRNA precursor capable of binding to the single-stranded antisense oligonucleotide. The target region includes a target region composed of the above-described base sequence as well as a region of RPS25 mRNA precursor.

(mRNA Precursor)

The mRNA precursor means a primary RNA transcript transcribed from DNA. That is, the mRNA precursor is an RNA that includes an exon region, an intron region, and an untranslated region (UTR). Another way of understanding it is that the mRNA precursor is an RNA prior to post-transcriptional splicing. The mRNA precursor is spliced to become an mRNA.

(Binding to Target Region)

Binding to the target region means that the single-stranded antisense oligonucleotide according to the present invention forms a double strand with the target region. The single-stranded antisense oligonucleotide according to the present invention does not necessarily form a double strand with the entire target region; it only needs to form a double strand with a part of the target region. In other words, the single-stranded antisense oligonucleotide according to the present invention is preferably fully complementary to the target region, but, as long as it is capable of binding to RPS25 target RNA, it is only necessary to be complementary to at least part of the target region.

(Part of Target Region)

The above-mentioned part of the target region means a region of the target region consisting of 10 to 15 nucleotide bases.

(Complementary to At Least Part of Target Region)

The expression "complementary to at least part of the target region" means being complementary to the base(s) of at least part of the target region of the target RNA. This expression also includes being complementary to the base(s) of mRNA or mRNA precursor corresponding to the at least part of the region.

<Base Sequence of Single-Stranded Antisense Oligonucleotide>

Preferably, the base sequence of the single-stranded antisense oligonucleotide according to the present embodiment is:

(A) a base sequence with a sequence identity of 90% to 100% to a base sequence complementary to a target region present in the base sequence as set forth in SEQ ID NO: 1 composed of consecutive 14 to 22 bases starting from a base located at one of position 123, position 124, position 185, position 186, position 263, position 264, position 324, position 325, position 442, position 443, and position 447 to position 453 counted from a 5' end;
(B) a base sequence complementary to a base sequence of the target region with deletion, substitution, insertion, or addition of one or several bases; or
(C) a base sequence capable of hybridizing under stringent conditions with an oligonucleotide having the target region.

With regard to the present embodiment, each base sequence provided in the sequence listing only shows information of the sequence of the nucleobase moiety. The information of the structure of the oligonucleotide including not only the nucleobase moiety but also the sugar moiety and the phosphate moiety is provided in the form shown in Table 2-1 to Table 2-4, Table 3-1, Table 3-2, and Table 4 provided below.

Herein, "sequence identity" means the following: two base sequences are aligned by a mathematical algorithm known in the technical field (preferably, this algorithm tolerates gap introduction to either one of the sequences or both the sequences for the sake of optimum alignment), and, in the resulting optimum alignment, the proportion (%) of matching bases across the entire base sequences overlapping each other is referred to as "sequence identity". A person skilled in the art can easily check the "sequence identity" between base sequences. For example, NCBI BLAST (National Center for Biotechnology Information Basic Local Alignment Search Tool) can be used.

The base sequence of the single-stranded antisense oligonucleotide according to the present embodiment

preferably has a sequence identity of 95% to 100%, more preferably has a sequence identity of 98% to 100%, further preferably has a sequence identity of 100%, to a base sequence complementary to the above-described particular target region present in the base sequence as set forth in SEQ ID NO: 1.

In the present embodiment, examples of the "base sequence with deletion, substitution, insertion, or addition of one or several bases" include a base sequence after the deletion, substitution, insertion, or addition that has a sequence identity of 80% or more, 85% or more, 90% or more, 95% or more, 97% or more, 98% or more, or 99% or more to a base sequence before the deletion, substitution, insertion, or addition. As for the specific number meant by the "one or several bases", the number of bases for each of the deletion, substitution, insertion, or addition may be one, two, three, four, or five, which may be separated from each other or next to each other.

Herein, "stringent conditions" refers to conditions of incubation carried out at room temperature for 12 hours in a solution containing 6×SSC (the composition of 1×SSC is 0.15-M NaCl, 0.015-M sodium citrate, pH7.0), 0.5% SDS, 5×Denhardt's solution, 100 μg/mL denatured salmon sperm DNA, and 50% (v/v) formamide, followed by rinsing with 0.5×SSC at a temperature of 50°C or more. This expression also includes more stringent conditions, such as, for example, incubation at 45°C or 60°C for 12 hours and rinsing with 0.2×SSC or 0.1×SSC wherein the rinsing is carried out at a temperature of 60°C or 65°C or more.

In an aspect of the present embodiment, preferably, the target region is a base sequence composed of consecutive 14 to 22 bases present in the base sequence as set forth in SEQ ID NO: 1 starting from a base located at one of position 123, position 124, position 185, position 186, position 263, position 264, position 324, position 325, position 442, position 443, and position 447 to position 453 counted from the 5' end.

In another aspect of the present embodiment, preferably, the target region is a base sequence composed of consecutive 14 to 22 bases present in the base sequence as set forth in SEQ ID NO: 1 starting from a base located at one of position 324, position 325, and position 448 to position 453 counted from the 5' end.

In another aspect of the present embodiment, preferably, the target region is a base sequence composed of consecutive 18 to 22 bases present in the base sequence as set forth in SEQ ID NO: 1 starting from a base located at one of position 448 to position 453 counted from the 5' end.

In another aspect of the present embodiment, preferably, the target region is a base sequence composed of consecutive 18 to 20 bases present in the base sequence as set forth in SEQ ID NO: 1 starting from a base located at one of position 450 to position 451 and position 453 counted from the 5' end.

The single-stranded antisense oligonucleotide is capable of binding to the target region in RPS25. Herein, the single-stranded antisense oligonucleotide according to the present invention "binding to the target region in RPS25" encompasses direct binding of the single-stranded antisense oligonucleotide according to the present invention to RPS25 mRNA, as well as direct binding thereof to RPS25 mRNA precursor.

An aspect of the single-stranded antisense oligonucleotide according to the present invention is a single-stranded oligonucleotide that is capable of modulating expression of RPS25 gene and that has one of the base sequences shown in Table 1, where the single-stranded oligonucleotide is complementary to a target region in human RPS25 mRNA shown in Table 1. As long as it includes a base sequence shown in Table 1, the single-stranded oligonucleotide may be longer by one to five bases toward the 3' end and/or the 5' end. It can be understood that the target region is a region of human RPS25 mRNA that is involved with modulation of expression of human RPS25 gene in particular (for example, a region that has an mRNA secondary structure to which the antisense nucleotide can bind easily). For example, in Table 1, the 5' end position being "451" and the 3' end position being "465" mean that a base sequence from position 451 to position 465 of the base sequence as set forth in SEQ ID NO: 1 counted from the 5' end thereof is the target region in human RPS25 mRNA targeted by the corresponding single-stranded antisense oligonucleotide (sequence name: "h451-465").

[Table 1]

| Sequence name | Antisense oligonucleotide sequence (5'-3') | hRPS25 target region (SEQ ID NO: 1) | |
|---|---|---|---|
| | | 5' end position | 3' end position |
| h451-465 | T'T'C'C'A'A'A'T'G'T'A'C'A'G'C' | 451 | 465 |
| h450-465 | T'T'C'C'A'A'A'T'G'T'A'C'A'G'C'T' | 450 | 465 |
| h325-341 | T′ T′ T′ A′ C′ T′ A′ A′ G′ G′ A′ G′ C′ T′ C′ C′ T′ | 325 | 341 |
| h450-466 | T′ T′ T′ C′ C′ A′ A′ A′ T′ G′ T′ A′ C′ A′ G′ C′ T′ | 450 | 466 |
| h451-467 | T′ T′ T′ T′ C′ C′ A′ A′ A′ T′ G′ T′ A′ C′ A′ G′ C′ | 451 | 467 |

(continued)

| Sequence name | Antisense oligonucleotide sequence (5'-3') | hRPS25 target region (SEQ ID NO: 1) | |
|---|---|---|---|
| | | 5' end position | 3' end position |
| h450-467 | T′ T′ T′ T′ C′ C′ A′ A′ A′ T′ G′ T′ A′ C′ A′ G′ C′ T′ | 450 | 467 |
| h451-469 | A′ T′ T′ T′ T′ T′ C′ C′ A′ A′ A′ T′ G′ T′ A′ C′ A′ G′ C′ | 451 | 469 |
| h453-472 | T′ T′ T′ A′ T′ T′ T′ T′ T′ C′ C′ A′ A′ A′ T′ G′ T′ A′ C′ A′ | 453 | 472 |

In Table 1 provided above, each of symbol "A'", symbol "C'", symbol "G'", and symbol "T'" is selected from natural nucleosides (a, A, c, C, g, G, t, and U described below) or modified nucleosides (including sugar-modified nucleosides). As for the sugar-modified nucleosides, symbol "A'" is selected from A (M), A (m), A (L), A (Y), A (Gx), A (5'-CP), A (Mx), and A (S) described below; symbol "C'" is selected from 5 (x), C (M), 5 (m), 5 (L), 5 (Y), 5 (Gx), 5 (5'-CP), C (Mx), and 5 (S) described below; symbol "G'" is selected from G (M), G (m), G (L), G (Y), G (Gx), G (5'-CP), G (Mx), and G (S) described below; and symbol "T'" is selected from U (M), T (m), T (L), T (Y), T (Gx), T (5'-CP), U (Mx), and T (S) described below.

In an aspect of the present embodiment, preferably, the base sequence of the single-stranded antisense oligonucleotide is a base sequence selected from the group consisting of base sequences as set forth in SEQ ID NOs: 5 to 47 and 49 to 56.

In an aspect of the present embodiment, more preferably, the base sequence of the single-stranded antisense oligonucleotide is a base sequence selected from the group consisting of SEQ ID NOs: 6, 9, 12, 15 to 18, 22, 24, 27 to 29, 31 to 36, 38, 40 to 42, 47, and 49 to 54.

In an aspect of the present embodiment, further preferably, the base sequence of the single-stranded antisense oligonucleotide is a base sequence selected from the group consisting of base sequences as set forth in SEQ ID NOs: 6, 9, 12, 15 to 18, 22, 24, 27 to 29, 31 to 32, 34 to 36, 38, 40 to 42, 47, and 49 to 54.

In an aspect of the present embodiment, further more preferably, the base sequence of the single-stranded antisense oligonucleotide is a base sequence selected from the group consisting of base sequences as set forth in SEQ ID NOs: 41, 47, 50, and 53 to 55.

<Pharmacologically Acceptable Salt>

The single-stranded antisense oligonucleotide according to the present embodiment may be in the form of a pharmacologically acceptable salt. Herein, the "pharmacologically acceptable salt" means a salt of the single-stranded antisense oligonucleotide according to the present invention that is a physiologically acceptable salt of the single-stranded antisense oligonucleotide according to the present invention, more specifically, a salt of the single-stranded antisense oligonucleotide that has a desirable biological activity and that does not have any undesirable toxicological effect. The same is true for a double-stranded antisense oligonucleotide and an antisense oligonucleotide complex, which are described below.

<Pharmaceutically Acceptable Salt>

In an aspect of the present embodiment, the single-stranded antisense oligonucleotide may be in the form of a pharmaceutically acceptable salt. Herein, the "pharmaceutically acceptable salt" means the pharmacologically acceptable salt as described above in the form of an acid addition salt or a base addition salt. Examples of the acid addition salt include inorganic acid salts such as hydrochloride, hydrobromide, sulfate, hydriodide, nitrate, and phosphate, as well as organic acid salts such as citrate, oxalate, phthalate, fumarate, maleate, succinate, malate, acetate, formate, propionate, benzoate, trifluoroacetate, methanesulfonate, benzenesulfonate, para-toluenesulfonate, and camphorsulfonate. Examples of the base addition salt include inorganic base salts such as sodium salt, potassium salt, calcium salt, magnesium salt, barium salt, and aluminum salt, as well as organic base salts such as trimethylamine, triethylamine, pyridine, picoline, 2,6-lutidine, ethanolamine, diethanolamine, triethanolamine, tromethamine [tris(hydroxymethyl)methylamine], tert-butylamine, cyclohexylamine, dicyclohexylamine, and N,N-dibenzylethylamine, and the like. Further examples thereof include salts with basic amino acids or acidic amino acids such as arginine, lysine, ornithine, aspartic acid, and glutamic acid (amino acid salts). The same is true for a double-stranded antisense oligonucleotide and an antisense oligonucleotide complex, which are described below.

<Structure of Single-Stranded Antisense Oligonucleotide>

The single-stranded antisense oligonucleotide according to the present embodiment includes a gap region, a 3' wing region bonded to the 3' end of the gap region, and a 5' wing region bonded to the 5' end of the gap region (see Fig. 1, for example). The single-stranded antisense oligonucleotide is preferably in the form of a single strand. In an aspect of the present embodiment, the single-stranded antisense oligonucleotide may be hybridized with a second oligonucleotide (which is described below) to form a double strand (a double-stranded antisense oligonucleotide). Preferably, the base sequence of the second oligonucleotide is a base sequence with a sequence identity of 90% to 100% to a base sequence complementary to the base sequence of the single-stranded antisense oligonucleotide.

The single-stranded antisense oligonucleotide is a so-called gapmer-type single-stranded antisense oligonucleotide. A gapmer-type single-stranded antisense oligonucleotide inhibits the function of the target RNA by the mechanism described below. Firstly, the single-stranded antisense oligonucleotide binds to the target region of the target RNA (in Fig. 2, top and center). Then, RNaseH, which is an RNase, recognizes the complex of the single-stranded antisense oligonucleotide and the target RNA, and binds to it (in Fig. 2, center). Subsequently, due to the RNaseH-driven enzymatic degradation reaction, the target RNA is cleaved and degraded. At this point, the single-stranded antisense oligonucleotide is not affected by the RNaseH-driven enzymatic degradation (in Fig. 2, bottom). As a result, the single-stranded antisense oligonucleotide can bind to another target RNA and cleave and degrade this RNA. Because a gapmer-type single-stranded antisense oligonucleotide functions as a catalyst in the above RNaseH-driven enzymatic degradation reaction, administration of only a small amount is expected to exhibit a certain level of sustained effect.

Moreover, the single-stranded antisense oligonucleotide, in the present embodiment, can be suitably used for modulating expression of RPS25 gene by the above-described mechanism (including the case where it acts via modulation of maturing of RPS25 mRNA precursor. Further, according to the present embodiment, even via intrathecal injection, which is a route of administration usually adopted in clinical settings, the effect of modulating RPS25 gene expression of the single-stranded antisense oligonucleotide can be exhibited. Herein, "modulating RPS25 gene expression" means, at least, inhibition of expression of RPS25 gene, and, as a result, it means, at least, inhibition of function of RPS25 protein (such as RAN translation).

(Gap Region)

The gap region includes at least one 5'-CP nucleic acid. The gap region is preferably a deoxyribose-based nucleic acid composed of 5 to 20 bases that may include a nucleic acid having a modified sugar moiety. In other words, the gap region is a deoxyribose-containing nucleic acid composed of 5 to 20 bases that may have a modified sugar moiety. Another way of understanding it is that the gap region is formed of one of or both a natural nucleotide and a non-natural nucleotide composed of 5 to 20 bases whose sugar moiety is deoxyribose. Because its sugar moiety is deoxyribose or modified deoxyribose, the gap region is capable of forming, together with the target RNA (RPS25 mRNA and/or the like), a complex that can be recognized by RNaseH. Here, examples of the nucleic acid including modified deoxyribose include 5'-CP nucleic acid.

The gap region is preferably composed of 5 to 20 bases, more preferably composed of 6 to 17 bases, further preferably composed of 7 to 13 bases, further more preferably composed of 9 to 13 bases.

Examples of the natural nucleotide whose sugar moiety is deoxyribose include deoxyadenosine monophosphate, deoxyguanosine monophosphate, thymidine monophosphate, deoxycytidine monophosphate, deoxy-5-methylcytidine monophosphate (also called 5-methyldeoxycytidine), and the like. In other words, examples of the natural nucleotide constituting the gap region include those including structural formulae corresponding to each of the symbols a, g, t, and c described below.

Examples of the non-natural nucleotide whose sugar moiety is deoxyribose or modified deoxyribose include 5'-CP nucleic acid, 2-thio-thymidine monophosphate, 2-aminoadenosine monophosphate, 7-deazaguanosine monophosphate, and the like.

As long as the effect of the present invention is exhibited, the gap region may be a natural nucleotide whose sugar moiety is deoxyribose where part of the sugar moiety is modified sugar. In other words, in an aspect of the present embodiment, the gap region may be a nucleic acid in which part of the sugar moiety is deoxyribose and another part of the sugar moiety is modified sugar (such as modified deoxyribose, for example).

In the present embodiment, the 5'-CP nucleic acid is preferably at least located at position 2 counted from the 5' end of the gap region.

In the present embodiment, the 5'-CP nucleic acid preferably comprises two or more 5'-CP nucleic acids, more preferably two to five 5'-CP nucleic acids, in the gap region. Herein, "comprising two or more 5'-CP nucleic acids" encompasses an aspect of comprising two or more 5'-CP nucleic acids of interest consecutively, and an aspect of comprising two or more 5'-CP nucleic acids scattered within the gap region.

In the present embodiment, the 5'-CP nucleic acid preferably comprises consecutive two to four 5'-CP nucleic

acids located at at least one position in the gap region.

In the present embodiment, the 5'-CP nucleic acid is preferably located on the 5' end side in the gap region. It is also preferable that the 5'-CP nucleic acid be located on the 5' end side and the 3' end side in the gap region. Herein, "located on the 5' end side in the gap region" means that the subject is located on the 5' end side of the center of the gap region. "Located on the 3' end side in the gap region" means that the subject is located on the 3' end side of the center of the gap region.

In the present embodiment, the 5'-CP nucleic acid preferably occupies one ninth or more of the gap region, more preferably one fifth or more. The upper limit may be one half, for example.

In the present embodiment, in the gap region, the bond between the 5'-CP nucleic acid and a nucleoside located adjacent to the 3' end of the 5'-CP nucleic acid is preferably a phosphorothioate bond (except for a case where the 5'-CP nucleic acid is located at the 3' end of the gap region). Herein, "the 5'-CP nucleic acid is located at the 3' end of the gap region" can also be understood as "the 5'-CP nucleic acid is bonded to a nucleoside in the 3' wing region".

In an aspect of the present embodiment, in the gap region, the bond between the 5'-CP nucleic acid and a nucleoside located adjacent to the 3' end of the 5'-CP nucleic acid is preferably a phosphorothioate bond (except for a case where the 5'-CP nucleic acid is located at the 3' end of the gap region and a case where the nucleoside located adjacent to the 3' end of the 5'-CP nucleic acid is a 5'-CP nucleic acid).

In the present embodiment, in the gap region, the bond between the 5'-CP nucleic acid and a nucleoside located adjacent to the 5' end of the 5'-CP nucleic acid is preferably a phosphodiester bond.

(3' Wing Region)

The 3' wing region is a modified nucleic acid having a substituent at 2' position. Another way of understanding it is that the 3' wing region is composed of modified nucleotides having a substituent at 2' position. Preferably, the modified nucleic acid having a substituent at 2' position in the 3' wing region includes at least one selected from the group consisting of non-bridged 2'-modified nucleic acids (2'-O-methyl nucleic acid, 2'-MOE nucleic acid, and MCE nucleic acid) and bridged modified nucleic acids (LNA, AmNA, GuNA, and scpBNA). When the 3' wing region described above and the 5' wing region described below are composed of these particular modified nucleotides, high binding affinity for the target RNA is expected to be obtained and thereby the function of the target RNA can be effectively reduced. In an aspect of the present embodiment, the 3' wing region may be a modified nucleic acid whose sugar moiety is modified sugar. Examples of the modified nucleic acid whose sugar moiety is modified sugar include those mentioned above in the section (Sugar Modification, Modified Sugar). In an aspect of the present embodiment, the modified nucleic acid having a substituent at 2' position in the 3' wing region may be solely composed of 2'-MOE nucleic acid. The 3' wing region in one single-stranded antisense oligonucleotide may include a plurality of types of modified nucleic acids.

In the present embodiment, the modified nucleic acid having a substituent at 2' position in the 3' wing region preferably includes at least one selected from the group consisting of 2'-MOE nucleic acid, LNA, AmNA, GuNA, and scpBNA, and more preferably, it is composed of a modified nucleic acid selected from the group consisting of 2'-MOE nucleic acid, LNA, AmNA, GuNA, and scpBNA. In another aspect of the present embodiment, the modified nucleic acid having a substituent at 2' position in the 3' wing region is preferably composed of a modified nucleic acid selected from the group consisting of 2'-MOE nucleic acid, AmNA, GuNA, and scpBNA.

The 3' wing region is preferably composed of 3 to 5 bases, more preferably composed of 3 to 4 bases.

(5' Wing Region)

The 5' wing region is a modified nucleic acid having a substituent at 2' position. Another way of understanding it is that the 5' wing region is composed of modified nucleotides having a substituent at 2' position. Preferably, the modified nucleic acid in the 5' wing region includes at least one selected from the group consisting of non-bridged 2'-modified nucleic acids (2'-O-methyl nucleic acid, 2'-MOE nucleic acid, and MCE nucleic acid) and bridged modified nucleic acids (LNA, AmNA, GuNA, and scpBNA). In an aspect of the present embodiment, the 5' wing region may be a modified nucleic acid whose sugar moiety is modified sugar. Examples of the modified nucleic acid whose sugar moiety is modified sugar include those mentioned above in the section (Sugar Modification, Modified Sugar). In an aspect of the present embodiment, the modified nucleic acid having a substituent at 2' position in the 5' wing region may be solely composed of 2'-MOE nucleic acid. The 5' wing region in one single-stranded antisense oligonucleotide may include a plurality of types of modified nucleic acids. In another aspect of the present embodiment, the modified nucleic acid in the 3' wing region and the 5' wing region may be solely composed of 2'-MOE nucleic acid, or may be solely composed of bridged modified nucleic acid.

In the present embodiment, the modified nucleic acid having a substituent at 2' position in the 5' wing region preferably includes at least one selected from the group consisting of 2'-MOE nucleic acid, LNA, AmNA, GuNA, and scpBNA, and more preferably, it is composed of a modified nucleic acid selected from the group consisting of 2'-MOE

nucleic acid, LNA, AmNA, GuNA, and scpBNA. In another aspect of the present embodiment, the modified nucleic acid having a substituent at 2' position in the 5' wing region is preferably composed of a modified nucleic acid selected from the group consisting of 2'-MOE nucleic acid, AmNA, GuNA, and scpBNA.

In an aspect of the present embodiment, preferably, the modified nucleic acid having a substituent at 2' position in the 3' wing region includes at least one selected from the group consisting of 2'-MOE nucleic acid, LNA, AmNA, GuNA, and scpBNA, and

the modified nucleic acid having a substituent at 2' position in the 5' wing region includes at least one selected from the group consisting of 2'-MOE nucleic acid, LNA, AmNA, GuNA, and scpBNA.

More preferably, the modified nucleic acid having a substituent at 2' position in the 3' wing region is composed of a modified nucleic acid selected from the group consisting of 2'-MOE nucleic acid, LNA, AmNA, GuNA, and scpBNA, and the modified nucleic acid having a substituent at 2' position in the 5' wing region is composed of a modified nucleic acid selected from the group consisting of 2'-MOE nucleic acid, LNA, AmNA, GuNA, and scpBNA.

In another aspect of the present embodiment, more preferably, the modified nucleic acid having a substituent at 2' position in the 3' wing region is composed of a modified nucleic acid selected from the group consisting of 2'-MOE nucleic acid, AmNA, GuNA, and scpBNA, and

the modified nucleic acid having a substituent at 2' position in the 5' wing region is composed of a modified nucleic acid selected from the group consisting of 2'-MOE nucleic acid, AmNA, GuNA, and scpBNA.

The 5' wing region is preferably composed of 3 to 5 bases, more preferably composed of 3 to 4 bases.

In an aspect of the present embodiment, preferably, the gap region is composed of 5 to 20 bases, the 3' wing region is composed of 3 to 5 bases, and the 5' wing region is composed of 3 to 5 bases.

In an aspect of the present embodiment, more preferably, the gap region is composed of 7 to 13 bases, the 3' wing region is composed of 3 to 5 bases, and the 5' wing region is composed of 3 to 5 bases.

In an aspect of the present embodiment, further preferably, the gap region is composed of 9 to 13 bases, the 3' wing region is composed of 3 to 4 bases, and the 5' wing region is composed of 3 to 4 bases.

(Description Style for Gapmer-Type Structure)

The single-stranded antisense oligonucleotide according to the present invention is of gapmer-type. For describing the structure of the gapmer-type, "X-Y-Z" may also be used. In this description style, "X" represents the base count of the 5' wing region, "Y" represents the base count of the gap region, and "Z" represents the base count of the 3' wing region.

Examples of "X-Y-Z" include 2-8-4, 2-8-3, 2-8-5, 2-9-2, 2-9-3, 2-9-4, 2-9-5, 2-10-3, 2-10-4, 2-10-5, 2-11-3, 2-11-4, 2-11-5, 2-12-3, 2-12-4, 2-12-5, 3-8-2, 3-8-3, 3-8-4, 3-8-5, 3-9-3, 3-9-4, 3-9-5, 3-10-3, 3-10-4, 3-10-5, 3-11-3, 3-11-4, 3-11-5, 3-12-3, 3-12-4, 3-12-5, 3-13-3, 3-13-4, 4-8-2, 4-8-3, 4-8-4, 4-8-5, 4-9-3, 4-9-4, 4-9-5, 4-10-3, 4-10-4, 4-10-5, 4-11-2, 4-11-3, 4-11-4, 4-11-5, 4-12-4, 4-13-3, 5-8-2, 5-8-3, 5-8-4, 5-8-5, 5-9-2, 5-9-3, 5-9-4, 5-9-5, 5-10-2, 5-10-3, 5-10-4, 5-10-5, 5-11-2, 5-11-3, 5-11-4, 5-11-5, and the like. For example, "2-8-4" means that the 5' wing region is a 2-mer oligonucleotide, the gap region is an 8-mer oligonucleotide, and the 3' wing region is a 4-mer oligonucleotide.

The single-stranded antisense oligonucleotide according to the present invention is composed of 12 to 30 bases, preferably 15 to 20 bases, more preferably 18 to 20 bases. When the single-stranded antisense oligonucleotide according to the present invention is composed of 15 to 20 bases, or 18 to 20 bases, the binding to RPS25 mRNA is particularly strong, allowing for effective modulation of RPS25 gene expression.

In the present embodiment, nucleosides of the single-stranded antisense oligonucleotide are bonded to each other via a phosphate group and/or a modified phosphate group, preferably via a phosphodiester bond or a phosphorothioate bond.

In an aspect of the present embodiment, at least one nucleoside-nucleoside bond in the single-stranded antisense oligonucleotide is preferably a phosphorothioate bond. In another aspect of the present embodiment, at least one nucleoside-nucleoside bond in the single-stranded antisense oligonucleotide is preferably a phosphodiester bond.

In an aspect of the present embodiment, the proportion of phosphorothioate bonds among all of nucleoside-nucleoside bonds in the single-stranded antisense oligonucleotide is preferably from 50% to 80%, more preferably from 50% to 70%.

An aspect of the single-stranded antisense oligonucleotide according to the present invention is a gapmer-type single-stranded antisense oligonucleotide that has a gap region composed of 5 to 20 bases, a 5' wing region composed of 3 to 5 bases, and a 3' wing region composed of 3 to 5 bases. The gap region is positioned between the 5' wing region and the 3' wing region. The gap region includes at least one 5'-CP nucleic acid. Preferably, each of the 5' wing region and the 3' wing region includes 2'-MOE nucleic acid, LNA, AmNA, GuNA, or scpBNA, in a number of at least one. Each of the 5' wing region and the 3' wing region may include 2'-O-alkylated nucleotide or 2'-F nucleotide. As the 2'-O-alkylated nucleotide, a nucleotide having 2'-O-alkylated (such as 2'-O-methylated, for example) D-ribofuranose may be used. The gapmer-type single-stranded antisense oligonucleotide may be hybridized with a second oligonucleotide to form a double strand.

In an aspect of the present embodiment, the single-stranded antisense oligonucleotide is preferably at least one selected from the group consisting of those of Example 2, Example 5, Example 8, Example 11 to Example 14, Example 18, Example 20, Example 23 to Example 25, Example 27 to Example 32, Example 34, Example 36 to Example 38, Example 43, and Example 45 to Example 50 in Table 2-1 to Table 2-4 provided below. More preferably, the single-stranded antisense oligonucleotide is at least one selected from the group consisting of those of Example 37, Example 43, Example 46, and Examples 49 to 51 in Table 2-1 to Table 2-4 provided below.

<Double-Stranded Antisense Oligonucleotide>

The double-stranded antisense oligonucleotide according to the present embodiment is a double-stranded antisense oligonucleotide or a pharmaceutically acceptable salt thereof comprising:

the single-stranded antisense oligonucleotide; and
a second oligonucleotide hybridized to the single-stranded antisense oligonucleotide. Preferably, the base sequence of the second oligonucleotide is a base sequence with a sequence identity of 90% to 100% to a base sequence complementary to the base sequence of the single-stranded antisense oligonucleotide.

The double-stranded antisense oligonucleotide may dissociate in a solution to separate into the single-stranded antisense oligonucleotide and the second oligonucleotide. After separation, the single-stranded antisense oligonucleotide is capable of binding to the above-described target RNA. The single-stranded antisense oligonucleotide may also be understood as "a first oligonucleotide" from its relationship with the second oligonucleotide. An antisense strand for the above-described target RNA is present only in the first oligonucleotide among the constituent oligonucleotides of the double-stranded antisense oligonucleotide, but, for the sake of convenience, the double-stranded oligonucleotide composed of the first oligonucleotide and the second oligonucleotide is called "a double-stranded antisense oligonucleotide".

<<Method of Producing Single-Stranded Antisense Oligonucleotide>>

The single-stranded antisense oligonucleotide according to the present invention can be produced by solid phase synthesis by means of a phosphoramidite method. Firstly, with the use of a commercially-available automatic nucleic-acid synthesizer, for example, a single-stranded oligonucleotide having a certain base sequence is synthesized on a solid support. Then, with the use of a basic substance and/or the like, the single-stranded oligonucleotide thus synthesized is removed from the solid support, followed by deprotection, and thereby a crude single-stranded oligonucleotide is obtained. Subsequently, the resulting crude single-stranded oligonucleotide is purified by HPLC and/or the like. This production method is not the only method to produce the single-stranded antisense oligonucleotide according to the present invention; the single-stranded antisense oligonucleotide according to the present invention can also be produced by other methods known to a person skilled in the art where the base sequence, the modified moiety, and/or the like of the nucleic acid are changed as appropriate. AmNA, GuNA, and scpBNA can be produced by methods described by International Patent Laying-Open No. WO 2011/052436 (PTL 2), International Patent Laying-Open No. WO 2014/046212 (PTL 3), and International Patent Laying-Open No. WO 2015/125783 (PTL 4), respectively. 2'-MOE nucleic acid can be produced by using an amidite that is available as a reagent. 5'-CP nucleic acid can be produced by a method described by International Patent Laying-Open No. WO 2020/158910 (PTL 5). LNA can be produced by a method described by International Patent Laying-Open No. WO 99/14226 (PTL 6).

<<Method of Producing Double-Stranded Antisense Oligonucleotide>>

The double-stranded antisense oligonucleotide according to the present invention can be produced in the following manner: firstly, by the same method as for producing the single-stranded antisense oligonucleotide, an oligonucleotide (a second oligonucleotide) having a certain sequence identity to a base sequence complementary to the single-stranded antisense oligonucleotide is produced, and, subsequently, the single-stranded antisense oligonucleotide and the second oligonucleotide are hybridized to each other.

<<Antisense Oligonucleotide Complex>>

The antisense oligonucleotide complex according to the present embodiment has:

the above-described single-stranded antisense oligonucleotide or a pharmaceutically acceptable salt thereof, or the above-described double-stranded antisense oligonucleotide or a pharmaceutically acceptable salt thereof; and

an additional substance bonded to the single-stranded antisense oligonucleotide or to the second oligonucleotide. The additional substance is selected from the group consisting of a polyethylene glycol, a peptide, an alkyl chain (such as a saturated aliphatic hydrocarbon, for example), a nucleic acid, a ligand compound, an antibody, a protein, and a sugar chain (such as a hydrocarbon and a polysaccharide, for example).

In an aspect of the present embodiment, the antisense oligonucleotide complex has:

the single-stranded antisense oligonucleotide or a pharmaceutically acceptable salt thereof; and
an additional substance bonded to the single-stranded antisense oligonucleotide, wherein the additional substance is selected from the group consisting of a polyethylene glycol, a peptide, an alkyl chain (such as a saturated aliphatic hydrocarbon, for example), a nucleic acid, a ligand compound, an antibody, a protein, and a sugar chain (such as a hydrocarbon and a polysaccharide, for example).

In another aspect of the present embodiment, the antisense oligonucleotide complex has:

the double-stranded antisense oligonucleotide or a pharmaceutically acceptable salt thereof; and
an additional substance bonded to the single-stranded antisense oligonucleotide or to the second oligonucleotide, wherein the additional substance is selected from the group consisting of a polyethylene glycol, a peptide, an alkyl chain (such as a saturated aliphatic hydrocarbon, for example), a nucleic acid, a ligand compound, an antibody, a protein, and a sugar chain (such as a hydrocarbon and a polysaccharide, for example).

Herein, "additional substance" means a substance bonded to the single-stranded antisense oligonucleotide or to the second oligonucleotide, used for providing certain action. The additional substance may be bonded to the 5' end of the single-stranded antisense oligonucleotide, or may be bonded to the 3' end thereof, or may be bonded to both the 5' end and the 3' end thereof. Also, the additional substance may be bonded to the 5' end of the second oligonucleotide, or may be bonded to the 3' end thereof, or may be bonded to both the 5' end and the 3' end thereof. In an aspect of the present embodiment, preferably, the additional substance is bonded to any one of the 5' end of the single-stranded antisense oligonucleotide, that of the second oligonucleotide, the 3' end of the single-stranded antisense oligonucleotide, and that of the second oligonucleotide. Also, the additional substance may be bonded to the single-stranded antisense oligonucleotide or to the second oligonucleotide directly via a covalent bond. The additional substance may be bonded to the single-stranded antisense oligonucleotide or to the second oligonucleotide via a linker substance. Examples of the linker substance include linkers that are composed of alkyl, polyethylene glycol, peptide, disulfide, or nucleic acid, or of a combination of these. The method for binding the additional substance to the single-stranded antisense oligonucleotide or to the second oligonucleotide may be, for example, a method described in Examples below.

Non-limiting examples of a peptide used as the additional substance include the following: CPPs (Cell Penetrating Peptides), nuclear translocation peptides, TAT (Trans-Activator of Transcription protein), polyarginine, glucagon-like peptide 1 analogs, synthetic cyclic RGD peptides, and brain translocation peptides.

Non-limiting examples of a ligand compound used as the additional substance include the following: N-acetylgalactosamine (GalNAc), sugars (such as glucose and mannose), lipids (such as cholesterol, palmitic acid, and docosahexaenoic acid), vitamins (such as folic acid, vitamin A, and vitamin E (tocopherol)), amino acids, and monoamine receptor ligands (such as indatraline).

Non-limiting examples of an antibody used as the additional substance include the following: anti-insulin-receptor antibody, anti-transferrin-receptor antibody, anti-LDL-receptor-associated-protein antibody, anti-CD22 antibody, anti-CD30 antibody, and anti-HER2 antibody.

Non-limiting examples of a protein used as the additional substance include the following: albumin.

Non-limiting examples of a nucleic acid used as the additional substance include the following: natural nucleotides, aptamers.

It should be noted that the nucleic acid used as the additional substance is not included when counting the base length of the antisense oligonucleotide.

<<Agent for Modulating Expression of RPS25 Gene>>

An agent for modulating expression of RPS25 gene according to the present embodiment comprises the single-stranded antisense oligonucleotide, the double-stranded antisense oligonucleotide, or the antisense oligonucleotide complex according to the present invention, as an active ingredient. In an aspect of the present embodiment, the agent for modulating expression may be an agent for reducing expression of RPS25 gene. In another aspect of the present embodiment, the agent for modulating expression may be an agent for reducing RAN translation. In another aspect of the present embodiment, the agent for modulating expression may be an agent for reducing expression of a dipeptide repeat

through reduction of RAN translation. The single-stranded antisense oligonucleotide according to the present invention binds to RPS25 mRNA or mRNA precursor to reduce expression of RPS25 gene and, in turn, reduce RAN translation which is caused by the translation product of the gene. The administration method and the preparation for the agent for modulating expression of RPS25 gene according to the present invention may be any administration method and preparation that are known in the field.

<<Pharmaceutical Composition Containing Single-Stranded Antisense Oligonucleotide and/or the Like as Active Ingredient>>

A pharmaceutical composition according to the present embodiment comprises the single-stranded antisense oligonucleotide or a pharmaceutically acceptable salt thereof, the double-stranded antisense oligonucleotide or a pharmaceutically acceptable salt thereof, or the antisense oligonucleotide complex or a pharmaceutically acceptable salt thereof according to the present invention, as an active ingredient. The administration method and the preparation for the pharmaceutical composition according to the present embodiment may be any administration method and preparation that are known in the field. Hereinafter, the pharmaceutical composition may also be expressed as "a pharmaceutical composition of the antisense oligonucleotide and/or the like".

The pharmaceutical composition is used for treating or preventing a disease associated with RPS25 gene, more specifically, a disease that can be induced by a dipeptide repeat produced by RAN translation. Another way of understanding it is that the pharmaceutical composition can be used for treating or preventing a disease whose symptom(s) is expected to be alleviated by reduction of expression of RPS25 gene. A disease of this kind may also be expressed as "a repeat disease". Specific examples of repeat diseases include various neuropsychiatric diseases and muscular diseases including C9orf72 ALS, C9orf72 FTLD, Huntington's disease, spinocerebellar ataxia (type 1, 2, 3, 6, 7, 8, 12, 17), dentatorubral-pallidoluysian atrophy, spinal and bulbar muscular atrophy, Friedreich ataxia, fragile X-associated tremor/ataxia syndrome, myotonic dystrophy, and the like.

<<Agent for Treating Repeat Disease and Agent for Preventing Repeat Disease>>

An agent for treating a repeat disease according to the present embodiment comprises the above-described single-stranded antisense oligonucleotide or a pharmaceutically acceptable salt thereof, the above-described double-stranded antisense oligonucleotide or a pharmaceutically acceptable salt thereof, or the above-described antisense oligonucleotide complex or a pharmaceutically acceptable salt thereof, as an active ingredient. An agent for preventing a repeat disease according to the present embodiment comprises the above-described single-stranded antisense oligonucleotide or a pharmaceutically acceptable salt thereof, the above-described double-stranded antisense oligonucleotide or a pharmaceutically acceptable salt thereof, or the above-described antisense oligonucleotide complex or a pharmaceutically acceptable salt thereof, as an active ingredient. Preferably, the repeat disease is at least one selected from the group consisting of C9orf72 ALS, C9orf72 FTLD, Huntington's disease, spinocerebellar ataxia, dentatorubral-pallidoluysian atrophy, spinal and bulbar muscular atrophy, Friedreich ataxia, fragile X-associated tremor/ataxia syndrome, and myotonic dystrophy.

<C9orf72 ALS>

C9orf72 ALS as mentioned above means a type of ALS that has a mutation that involves abnormal repeat expansion of GGGGCC sequence present in the intron region of C9orf72 gene between the exon 1a region and the exon 1b region. C9orf72 gene is the most frequently found ALS-causing gene, and responsible for about 6% of sporadic ALS and about 40% of familial ALS. ALS is a neurodegenerative disease that causes selective death of motor neurons leading to muscle atrophy. Diagnosis of ALS is made based on clinical characteristics or electrophysiological characteristics of a combination of upper and lower motor neuron degeneration. More specifically, when one of four regions (namely, brainstem, cervical cord, thoracic cord, and lumbosacral cord) shows signs of upper and lower motor neuron degeneration and the other regions have electromyographic finding, the diagnosis is "laboratory-supported probable"; when two of the four regions show the signs, the diagnosis is "probable"; and three of the four regions show the signs, the diagnosis is "definite".

<C9orf72 FTLD>

C9orf72 FTLD as mentioned above means an FTLD that has a mutation that involves abnormal repeat expansion of GGGGCC sequence present in the intron region of C9orf72 gene between the exon 1a region and the exon 1b region. FTLD of this type manifests in the form of progressive abnormal behavior and cognitive dysfunction causing daily living to be more challenging, and it also shows at least three symptoms from behavioral disinhibition, indifference and/or inertia,

adherence and/or stereotyped behavior, hyperorality, changes in dietary habits, and the like. FTLD of this type is diagnosed as behavioral variant FTLD when imaging examination shows atrophy and/or impaired metabolism and/or impaired blood flow in the frontal lobe and/or in the anterior temporal lobe and when the disease can be differentiated from certain diseases. FTLD of this type is diagnosed as semantic dementia FTLD when memory loss of names of objects and meaning of words, and symptoms of loss of knowledge of objects or superficial dyslexia and/or agraphia are observed, atrophy is found in the anterior dominant temporal lobe, and the disease can be differentiated from certain diseases.

<Huntington's disease>

Huntington's disease as mentioned above means a hereditary neurodegenerative disease that exhibits autosomal dominant inheritance developed by abnormal repeat expansion of CAG sequence present in the exon 1 region of huntingtin gene. Huntington's disease manifests in the form of movement disorder characterized in involuntary movement, psychiatric symptoms, and cognitive symptoms. Diagnosis of Huntington's disease is made when particular neurologic signs are observed and genetic diagnosis identifies abnormal expansion mutation of CAG sequence, or when the course of the disease is progressive, family history of autosomal dominant inheritance and certain neurologic signs and clinical laboratory signs are observed, and differential diagnosis denies the possibility of similar diseases.

<Spinocerebellar Ataxia (Type 1, 2, 3, 6, 7, 8, 12, 17), and Dentatorubral-Pallidoluysian Atrophy>

Each of spinocerebellar ataxia (type 1, 2, 3, 6, 7, 8, 12, 17) and dentatorubral-pallidoluysian atrophy as mentioned above means a hereditary neurodegenerative disease that exhibits autosomal dominant inheritance developed by abnormal repeat expansion of a particular three-base sequence (CAG or CTG) present on the disease gene in the disease. In spinocerebellar ataxia type 1, 2, 3, 6, 7, 12, and 17 and in dentatorubral-pallidoluysian atrophy, CAG repeats are observed. In spinocerebellar ataxia type 8, CTG repeats are observed. Spinocerebellar ataxia and dentatorubral-pallidoluysian atrophy manifest themselves in the form of cerebellar or posterior column ataxia or spastic paraplegia as their major symptom and are basically indolent, and diagnosis of them is made by a combination of genetic diagnosis, neuropathological diagnosis, and the like.

<Spinal and Bulbar Muscular Atrophy>

Spinal and bulbar muscular atrophy as mentioned above means a hereditary disease that is developed by abnormal repeat expansion of CAG sequence present in the exon region of androgen receptor gene. Diagnosis of spinal and bulbar muscular atrophy is made by a combination of neurologic signs (bulbar syndrome, lower motor neuron signs, finger tremor, tendon hyporeflexia of extremities), clinical signs, laboratory signs, genetic diagnosis, and the like.

<Friedreich Ataxia>

Friedreich ataxia as mentioned above means a hereditary neurodegenerative disease that exhibits autosomal recessive inheritance developed by mutation of frataxin gene. Most cases of Friedreich ataxia are caused by abnormal repeat expansion of GAA sequence present in the first intron.

<Fragile X-Associated Tremor/Ataxia Syndrome>

Fragile X-associated tremor/ataxia syndrome as mentioned above means a hereditary neurodegenerative disease developed by abnormal repeat expansion of CGG sequence present in 5'UTR of FMP1 gene. Diagnosis of fragile X-associated tremor/ataxia syndrome is made by a combination of clinical symptoms (cerebellar ataxia, action tremor, parkinsonism, dementia, mental retardation), signs of middle cerebellar peduncle by MRI examination, genetic diagnosis, and the like.

<Myotonic Dystrophy>

Myotonic dystrophy as mentioned above means a hereditary muscular disease that exhibits autosomal dominant inheritance developed by abnormal repeat expansion of CUG sequence present in 3'UTR of DMPK gene.

<Individuals>

The individual mentioned above means a mammal. Preferably, the individual refers to a human, a monkey and ape, a marmoset, a dog, a pig, a rabbit, a guinea pig, a rat, and a mouse. More preferably, the individual is a human.

In an aspect of the present embodiment, the single-stranded antisense oligonucleotide or a pharmaceutical composition thereof may be administered via a route of administration suitable for a subject (individual) susceptible to delayed central toxicity. Herein, "delayed central toxicity" means a type of central toxicity that manifests after a lapse of time period during which acute-phase central toxicity can appear and after the subject is recovered. Examples of symptoms that can appear due to delayed central toxicity include a decrease in locomotor activity, abnormal gait and abnormal hindlimb function, tremor, weakness in hindlimbs and tail, loss of hindlimb reflex, body weight loss, and the like. The toxicity is assessed by scoring the performance status signs noticed on the observation day according to the criteria given below, and the scores are summed over the course of the observation period to calculate the Clinical sign score. A high score is given to a sign of strong toxicity, and hence, a single-stranded antisense oligonucleotide with a low Clinical sign score can be judged as safe. In addition, the presence or absence of abnormal signs in cerebral pathological examination is also scored to calculate the Pathological score. The score is the average value of all the groups. Here, "a subject (individual) susceptible to delayed central toxicity" refers to a subject who can be identified by the use of biomarkers and/or the like.

Clinical sign score;

0 points: No abnormalities observed
1 point: Abnormal hindlimb function, tremor, decrease in locomotor activity
2 points: Dragging of hindlimbs, weakness in tail and hindlimbs
3 points: Complete loss of hindlimb function, hindlimb paralysis, lying on side, lying on belly
4 points: Euthanized

Pathological score;

0 points: No abnormalities observed
1 point: Abnormalities observed (such as single cell necrosis and vacuolization)

It is known that nucleic acid drugs can exhibit toxicity by hybridizing with an RNA (off-target candidate gene or off-target candidate RNA) whose base sequence is the same as or similar to the target RNA and affecting the off-target candidate gene (this phenomenon can be referred to as "off-target toxicity in a narrow sense"). For avoiding unexpected off-target toxicity in a narrow sense, it is important to derive (design) the target RNA from a region that will not hybridize with a non-target RNA. In an aspect of the present embodiment, for the purpose of assessing the effect of the single-stranded antisense oligonucleotide or a pharmaceutical composition thereof on off-target candidate genes, it is possible to search for similar base sequences with the use of "Ultrafast Sequence Search GGGenome (https//gggenome.dbcls.jp/ja/)" and/or the like to estimate narrow-sense off-target candidate genes. When there are not many off-target candidate genes, the risk of narrow-sense off-target toxicity can be considered low.

For the administration of the single-stranded antisense oligonucleotide or a pharmaceutical composition thereof according to the present invention (including an agent for treating C9orf72 ALS and an agent for preventing C9orf72 ALS), the method and form of administration are not particularly limited. In other words, any administration method and any preparation known in the field can be used as the administration method and preparation for the antisense oligonucleotide according to the present invention and the like. Examples of the administration method include oral administration, parenteral administration, and the like. Examples of the parenteral administration include ophthalmic administration, vaginal administration, intrarectal administration, intranasal administration, transdermal administration, intravenous injection, infusion, subcutaneous, intraperitoneal, or intramuscular injection, pulmonary administration via suction or inhalation, intrathecal injection, intraventricular administration, and the like.

To the preparation of the antisense oligonucleotide according to the present invention and the like, various pharmaceutical additives such as excipient, binder, wetting agent, disintegrating agent, lubricant, diluent, taste masking agent, fragrant agent, dissolution promoter, suspending agent, emulsifier, stabilizer, preservative, isotonic agent, and the like can be mixed, as needed.

In the case of topical administration of a pharmaceutical composition of the antisense oligonucleotide according to the present invention and the like, preparations such as transdermal patch, ointment, lotion, cream, gel, drops, suppository, spray, liquid preparation, powder, and the like can be used, for example.

In the case of oral administration of a pharmaceutical composition of the antisense oligonucleotide according to the present invention and the like, preparations such as powder, granule, suspension in or solution dissolved in water or non-aqueous medium, capsule, powder agent, pill, and the like can be used, for example.

In the case of parenteral, intrathecal, or intraventricular administration of a pharmaceutical composition of the antisense oligonucleotide according to the present invention and the like, preparations such as sterile aqueous solution can be used, for example.

The effective dose of the single-stranded antisense oligonucleotide according to the present invention can be determined as appropriate depending on the sex, age, body weight, symptoms, and the like of the individual who is the administration target. Further, it can also be determined as appropriate depending on the method, route, frequency, and the like of administration. For example, the dose may be from 0.01 to 100 mg/kg and the like. It is preferably from 0.1 to 50 mg/kg, further preferably from 0.1 to 10 mg/kg.

<<Method of Modulating Expression of RPS25 Gene>>

A method of modulating expression of RPS25 gene according to the present embodiment comprises administering the above-described single-stranded antisense oligonucleotide or a pharmaceutically acceptable salt thereof or the above-described antisense oligonucleotide complex or a pharmaceutically acceptable salt thereof, as an active ingredient, to a cell, a tissue, or an individual expressing the RPS25 gene.

In the present embodiment, the method for administering the single-stranded antisense oligonucleotide and the like to a cell, a tissue, or an individual may be performed in vitro or in vivo. In the case of in vivo administration, the route of administration is the above-described route of administration.

In the present embodiment, examples of "a cell expressing RPS25 gene" include neurons constituting the central nervous system, neurons constituting the peripheral nervous system, cells constituting other dermal tissue, and the like.

A method of treating or preventing a repeat disease according to the present embodiment comprises administering the above-described single-stranded antisense oligonucleotide or a pharmaceutically acceptable salt thereof or the above-described antisense oligonucleotide complex or a pharmaceutically acceptable salt thereof, as an active ingredient, to an individual suffering from the repeat disease. Preferably, the individual is a subject (individual) susceptible to delayed central toxicity.

Examples of the repeat disease include neuropsychiatric diseases, muscular diseases, and the like described above. The form, administration route, and dose for administration to an individual can be selected as appropriate from those described above.

In the above, the antisense oligonucleotide according to the present embodiment is described. The single-stranded antisense oligonucleotide having the above-described configuration can modulate RPS25 gene expression. Here, the activity to reduce RPS25 gene expression (knockdown activity) can be measured by a known method. Examples of the method for measuring the knockdown activity include methods described in Nature (2015) 518 (7539):409-12 (NPL 12) and International Patent Laying-Open No. WO 2022/097727 (PTL 7). It can also be measured by transfection of the antisense oligonucleotide to HEK293T cells, as described below. By the way, because the single-stranded antisense oligonucleotide includes at least one 5'-CP nucleic acid in the gap region, delayed central toxicity is reduced.

<<Method for Evaluating RPS25 Gene Expression-Reducing Activity>>

<Introduction of Antisense Oligonucleotide into Cells>

"Cells expressing RPS25 gene" are treated with the antisense oligonucleotide for 6 hours to 3 days by a method such as lipofection, electroporation, or direct addition introduction. The cells to be used may be any cells as long as they express RPS25 gene, and examples thereof include HEK293T cells, more preferably neurons, further preferably human-derived neurons. The cells thus treated with the antisense oligonucleotide may be collected immediately after the treatment, or may be continued to be cultured after removal of the antisense oligonucleotide.

<Evaluation of Amount of RPS25 mRNA>

Total RNA is extracted from the collected cells and subjected to reverse transcription reaction, and the resulting complementary DNA is subjected to real-time PCR and/or the like with the use of a probe specific to RPS25 gene, to measure the amount of RPS25 mRNA. Examples of the probe for use in real-time PCR include Taqman probe. Examples of the method for the reaction include a method that involves repeating, a number of times that is not particularly limited, a three-step procedure of "(cDNA denaturing)-(annealing)-(elongation reaction)" or a two-step procedure of "(cDNA denaturing)-(annealing and elongation reaction)". For example, the two- or three-step procedure is repeated 25 to 45 times, preferably 35 to 40 times. For example, the temperature for (cDNA denaturing) is from 90°C to 98°C, preferably from 92°C to 95°C. For example, the temperature for (annealing) is from 40°C to 70°C, preferably from 50°C to 60°C. For example, the temperature for (elongation reaction) is from 65°C to 75°C, preferably it is the optimum temperature for the polymerase used in the reaction. For example, the temperature for (annealing and elongation reaction) is from 55°C to

70°C.

<Evaluation of Amount of RPS25 Protein>

The cells thus collected are lysed to obtain an extract. By an immunochemical technique such as Western blotting and ELISA (Enzyme-Linked Immuno Sorbent Assay), the amount of RPS25 protein contained in the extract is assessed. In the case of Western blotting, the apparatus to be used in each of the steps of electrophoresis, transfer, and detection is not particularly limited. The reaction time and reaction temperature for reaction of the membrane with the primary antibody or the secondary antibody can be selected as needed, and examples thereof include overnight at 4°C, and 1 to 3 hours at room temperature.

It should be noted that the present invention is not limited to the above-described embodiment. For example, the single-stranded antisense oligonucleotide encompasses the aspects described below.

An antisense oligonucleotide according to a first aspect of the present invention is a single-stranded antisense oligonucleotide, or a pharmaceutically acceptable salt thereof, capable of modulating expression and/or function of RPS25 gene, wherein

nucleosides of the single-stranded antisense oligonucleotide are bonded to each other via a phosphate group and/or a modified phosphate group,
the single-stranded antisense oligonucleotide includes a gap region, a 3' wing region bonded to a 3' end of the gap region, and a 5' wing region bonded to a 5' end of the gap region,
the gap region is a deoxyribose-based nucleic acid, which may contain a nucleic acid having a modified sugar moiety,
the gap region includes at least one 5'-CP nucleic acid,
each of the 3' wing region and the 5' wing region is a modified nucleic acid having a substituent at 2' position,
the single-stranded antisense oligonucleotide is composed of 12 to 30 bases, and
a base sequence of the single-stranded antisense oligonucleotide is:

a base sequence with a sequence identity of 90% to 100% to a base sequence complementary to at least one target region having the same base length as the single-stranded antisense oligonucleotide present in a base sequence as set forth in SEQ ID NO: 1;
a base sequence complementary to a base sequence of the target region with deletion, substitution, insertion, or addition of one or several bases; or
a base sequence capable of hybridizing under stringent conditions with an oligonucleotide having the target region.

An antisense oligonucleotide according to a second aspect of the present invention is a single-stranded antisense oligonucleotide, or a pharmaceutically acceptable salt thereof, capable of modulating expression and/or function of RPS25 gene, wherein

nucleosides of the single-stranded antisense oligonucleotide are bonded to each other via a phosphate group and/or a modified phosphate group,
the single-stranded antisense oligonucleotide includes a gap region, a 3' wing region bonded to a 3' end of the gap region, and a 5' wing region bonded to a 5' end of the gap region,
the gap region is a deoxyribose-based nucleic acid , which may contain a nucleic acid having a modified sugar moiety,
the gap region includes at least one 5'-CP nucleic acid,
each of the 3' wing region and the 5' wing region is a modified nucleic acid having a substituent at 2' position,
the single-stranded antisense oligonucleotide is composed of 12 to 30 bases, and
a base sequence of the single-stranded antisense oligonucleotide is:

a base sequence with a sequence identity of 90% to 100% to a base sequence complementary to at least one target region having the same base length as the single-stranded antisense oligonucleotide present in a base sequence as set forth in SEQ ID NO: 1;
a base sequence complementary to a base sequence of the target region with deletion, substitution, insertion, or addition of one or several bases; or
a base sequence capable of hybridizing under stringent conditions with an oligonucleotide having the target region

(except for single-stranded antisense oligonucleotides of Reference Examples 1 to 31).

An antisense oligonucleotide according to a third aspect of the present invention is a single-stranded antisense oligonucleotide, or a pharmaceutically acceptable salt thereof, capable of modulating expression and/or function of RPS25 gene, wherein

nucleosides of the single-stranded antisense oligonucleotide are bonded to each other via a phosphate group and/or a modified phosphate group,
the single-stranded antisense oligonucleotide includes a gap region, a 3' wing region bonded to a 3' end of the gap region, and a 5' wing region bonded to a 5' end of the gap region,
the gap region is a deoxyribose-based nucleic acid , which may contain a nucleic acid having a modified sugar moiety,
the gap region includes at least one 5'-CP nucleic acid,
each of the 3' wing region and the 5' wing region is a modified nucleic acid having a substituent at 2' position,
the single-stranded antisense oligonucleotide is composed of 12 to 30 bases, and
a base sequence of the single-stranded antisense oligonucleotide is:

a base sequence with a sequence identity of 90% to 100% to a base sequence complementary to at least one target region having the same base length as the single-stranded antisense oligonucleotide present in a base sequence as set forth in SEQ ID NO: 1;
a base sequence complementary to a base sequence of the target region with deletion, substitution, insertion, or addition of one or several bases; or
a base sequence capable of hybridizing under stringent conditions with an oligonucleotide having the target region

(except for single-stranded antisense oligonucleotides of Reference Examples 1 to 31 and Design Examples 1 to 18).

In an aspect of the present invention, preferably, the base sequence of the single-stranded antisense oligonucleotide is:
a base sequence with a sequence identity of 95% to 100% to a base sequence complementary to at least one target region having the same base length as the single-stranded antisense oligonucleotide present in the base sequence as set forth in SEQ ID NO: 1.
In an aspect of the present invention, preferably, the base sequence of the single-stranded antisense oligonucleotide is:
a base sequence complementary to at least one target region having the same base length as the single-stranded antisense oligonucleotide present in the base sequence as set forth in SEQ ID NO: 1.
In an aspect of the present invention, preferably,

the gap region is composed of 5 to 20 bases,
the 3' wing region is a modified nucleic acid composed of 3 to 5 bases having a substituent at 2' position, and
the 5' wing region is a modified nucleic acid composed of 3 to 5 bases having a substituent at 2' position.

In an aspect of the present invention, preferably, the 5'-CP nucleic acid is at least located at position 2 counted from the 5' end of the gap region.
In an aspect of the present invention, preferably, the 5'-CP nucleic acid comprises two or more 5'-CP nucleic acids in the gap region.
In an aspect of the present invention, preferably, the 5'-CP nucleic acid comprises two to five 5'-CP nucleic acids in the gap region.
In an aspect of the present invention, preferably, the 5'-CP nucleic acid occupies one ninth or more of the gap region.
In an aspect of the present invention, preferably, the 5'-CP nucleic acid occupies one fifth or more of the gap region.
In an aspect of the present invention, preferably, the 5'-CP nucleic acid comprises consecutive two to four 5'-CP nucleic acids located at at least one position in the gap region.
In an aspect of the present invention, preferably, the 5'-CP nucleic acid is located on the 5' end side in the gap region.
In an aspect of the present invention, preferably, the 5'-CP nucleic acid is located on the 5' end side and the 3' end side in the gap region.
In an aspect of the present invention, preferably, the single-stranded antisense oligonucleotide is composed of 15 to 20 bases.
In an aspect of the present invention, preferably, the single-stranded antisense oligonucleotide is composed of 18

to 20 bases.

In an aspect of the present invention, preferably,

the modified nucleic acid having a substituent at 2' position in the 3' wing region includes at least one selected from the group consisting of 2'-MOE nucleic acid, LNA, AmNA, GuNA, and scpBNA, and
the modified nucleic acid having a substituent at 2' position in the 5' wing region includes at least one selected from the group consisting of 2'-MOE nucleic acid, LNA, AmNA, GuNA, and scpBNA.

In an aspect of the present invention, preferably,

the modified nucleic acid having a substituent at 2' position in the 3' wing region is composed of a modified nucleic acid selected from the group consisting of 2'-MOE nucleic acid, LNA, AmNA, GuNA, and scpBNA, and
the modified nucleic acid having a substituent at 2' position in the 5' wing region is composed of a modified nucleic acid selected from the group consisting of 2'-MOE nucleic acid, LNA, AmNA, GuNA, and scpBNA.

In an aspect of the present invention, preferably,

the modified nucleic acid having a substituent at 2' position in the 3' wing region is composed of a modified nucleic acid selected from the group consisting of 2'-MOE nucleic acid, AmNA, GuNA, and scpBNA, and
the modified nucleic acid having a substituent at 2' position in the 5' wing region is composed of a modified nucleic acid selected from the group consisting of 2'-MOE nucleic acid, AmNA, GuNA, and scpBNA.

In an aspect of the present invention, preferably, at least one nucleoside-nucleoside bond in the single-stranded antisense oligonucleotide is a phosphorothioate bond.

In an aspect of the present invention, preferably, at least one nucleoside-nucleoside bond in the single-stranded antisense oligonucleotide is a phosphodiester bond.

In an aspect of the present invention, preferably, a proportion of phosphorothioate bonds among all of nucleoside-nucleoside bonds in the single-stranded antisense oligonucleotide is from 50% to 80%.

In an aspect of the present invention, preferably, a proportion of phosphorothioate bonds among all of nucleoside-nucleoside bonds in the single-stranded antisense oligonucleotide is from 50% to 70%.

In an aspect of the present invention, preferably, in the gap region, a bond between the 5'-CP nucleic acid and a nucleoside located adjacent to a 3' end of the 5'-CP nucleic acid is a phosphorothioate bond (except for a case where the 5'-CP nucleic acid is located at the 3' end of the gap region).

In an aspect of the present invention, preferably, in the gap region, a bond between the 5'-CP nucleic acid and a nucleoside located adjacent to a 3' end of the 5'-CP nucleic acid is a phosphorothioate bond (except for a case where the 5'-CP nucleic acid is located at the 3' end of the gap region and a case where the nucleoside located adjacent to the 3' end of the 5'-CP nucleic acid is a 5'-CP nucleic acid).

In an aspect of the present invention, preferably, in the gap region, a bond between the 5'-CP nucleic acid and a nucleoside located adjacent to a 5' end of the 5'-CP nucleic acid is a phosphodiester bond.

In an aspect of the present invention, preferably, the base sequence of the single-stranded antisense oligonucleotide is:

a base sequence with a sequence identity of 90% to 100% to a base sequence complementary to a target region present in the base sequence as set forth in SEQ ID NO: 1 composed of consecutive 14 to 22 bases starting from a base located at one of position 123, position 124, position 185, position 186, position 263, position 264, position 324, position 325, position 442, position 443, and position 447 to position 453 counted from a 5' end;
a base sequence complementary to a base sequence of the target region with deletion, substitution, insertion, or addition of one or several bases; or
a base sequence capable of hybridizing under stringent conditions with an oligonucleotide having the target region.

In an aspect of the present invention, preferably, the base sequence of the single-stranded antisense oligonucleotide is:

a base sequence with a sequence identity of 90% to 100% to a base sequence complementary to a target region present in the base sequence as set forth in SEQ ID NO: 1 composed of consecutive 14 to 22 bases starting from a base located at one of position 324, position 325, and position 448 to position 453 counted from a 5' end;
a base sequence complementary to a base sequence of the target region with deletion, substitution, insertion, or addition of one or several bases; or

a base sequence capable of hybridizing under stringent conditions with an oligonucleotide having the target region.

In an aspect of the present invention, preferably,

the base sequence of the single-stranded antisense oligonucleotide is a base sequence with a sequence identity of 90% to 100% to a base sequence complementary to a target region present in the base sequence as set forth in SEQ ID NO: 1 composed of consecutive 18 to 22 bases starting from a base located at one of position 448 to position 453 counted from a 5' end,
the 3' wing region is composed of 3 to 5 bases, and
the 5' wing region is composed of 3 to 5 bases.

In an aspect of the present invention, preferably, the base sequence of the single-stranded antisense oligonucleotide is a base sequence with a sequence identity of 90% to 100% to a base sequence complementary to a target region present in the base sequence as set forth in SEQ ID NO: 1 composed of consecutive 18 to 20 bases starting from a base located at one of position 450 to position 451 and position 453 counted from a 5' end.
In an aspect of the present invention, preferably, the base sequence of the single-stranded antisense oligonucleotide is a base sequence selected from the group consisting of base sequences as set forth in SEQ ID NOs: 5 to 47 and 49 to 56.
In an aspect of the present invention, preferably, the base sequence of the single-stranded antisense oligonucleotide is a base sequence selected from the group consisting of base sequences as set forth in SEQ ID NOs: 6, 9, 12, 15 to 18, 22, 24, 27 to 29, 31 to 36, 38, 40 to 42, 47, and 49 to 54.
In an aspect of the present invention, preferably, the base sequence of the single-stranded antisense oligonucleotide is a base sequence selected from the group consisting of base sequences as set forth in SEQ ID NOs: 41, 47, 50, and 53 to 55.
An antisense oligonucleotide complex according to an aspect of the present invention is an antisense oligonucleotide complex, or a pharmaceutically acceptable salt thereof, comprising:

the above-descried single-stranded antisense oligonucleotide or a pharmaceutically acceptable salt thereof; and
an additional substance bonded to the single-stranded antisense oligonucleotide, wherein
the additional substance is selected from the group consisting of a polyethylene glycol, a peptide, an alkyl chain, a nucleic acid, a ligand compound, an antibody, a protein, and a sugar chain.

A pharmaceutical according to an aspect of the present invention comprises the above-descried single-stranded antisense oligonucleotide or a pharmaceutically acceptable salt thereof or the above-described antisense oligonucleotide complex or a pharmaceutically acceptable salt thereof, as an active ingredient.
In an aspect of the pharmaceutical, preferably, the above-descried single-stranded antisense oligonucleotide or a pharmaceutically acceptable salt thereof or the above-described antisense oligonucleotide complex or a pharmaceutically acceptable salt thereof is administered in such a manner that the central nervous system is exposed thereto.
In an aspect of the pharmaceutical, preferably, the above-descried single-stranded antisense oligonucleotide or a pharmaceutically acceptable salt thereof or the above-described antisense oligonucleotide complex or a pharmaceutically acceptable salt thereof is administered to a subject susceptible to delayed central toxicity.
An agent for modulating expression and/or function of RPS25 gene according to an aspect of the present invention comprises the above-descried single-stranded antisense oligonucleotide or a pharmaceutically acceptable salt thereof or the above-described antisense oligonucleotide complex or a pharmaceutically acceptable salt thereof, as an active ingredient.
An agent for inhibiting dipeptide repeat production attributable to RAN translation according to an aspect of the present invention comprises the above-descried single-stranded antisense oligonucleotide or a pharmaceutically acceptable salt thereof or the above-described antisense oligonucleotide complex or a pharmaceutically acceptable salt thereof, as an active ingredient.
An agent for treating a repeat disease according to an aspect of the present invention comprises the above-descried single-stranded antisense oligonucleotide or a pharmaceutically acceptable salt thereof or the above-described antisense oligonucleotide complex or a pharmaceutically acceptable salt thereof, as an active ingredient.
An agent for preventing a repeat disease according to an aspect of the present invention comprises the above-descried single-stranded antisense oligonucleotide or a pharmaceutically acceptable salt thereof or the above-described antisense oligonucleotide complex or a pharmaceutically acceptable salt thereof, as an active ingredient.
In an aspect of the treating agent and the preventing agent, preferably, the repeat disease is at least one selected from the group consisting of C9orf72 ALS, C9orf72 FTLD, Huntington's disease, spinocerebellar ataxia, dentatorubral-pallidoluysian atrophy, spinal and bulbar muscular atrophy, Friedreich ataxia, fragile X-associated tremor/ataxia syndrome, and myotonic dystrophy.

A method of treating or preventing a repeat disease according to an aspect of the present invention comprises administering the above-descried single-stranded antisense oligonucleotide or a pharmaceutically acceptable salt thereof or the above-described antisense oligonucleotide complex or a pharmaceutically acceptable salt thereof, to an individual.

In an aspect of the method of treating or preventing a repeat disease, preferably, the repeat disease is at least one selected from the group consisting of C9orf72 ALS, C9orf72 FTLD, Huntington's disease, spinocerebellar ataxia, dentatorubral-pallidoluysian atrophy, spinal and bulbar muscular atrophy, Friedreich ataxia, fragile X-associated tremor/-ataxia syndrome, and myotonic dystrophy.

An aspect of the present invention provides the above-descried single-stranded antisense oligonucleotide or a pharmaceutically acceptable salt thereof or the above-described antisense oligonucleotide complex or a pharmaceutically acceptable salt thereof, for use for treating or preventing C9orf72 ALS.

An aspect of the present invention provides the above-descried single-stranded antisense oligonucleotide or a pharmaceutically acceptable salt thereof or the above-described antisense oligonucleotide complex or a pharmaceutically acceptable salt thereof, for use for producing an agent for treating or preventing C9orf72 ALS.

[Examples]

In the following, Examples of the present invention will be described, but the scope of the present invention is not limited to these Examples.

<<Preparation of Single-Stranded Antisense Oligonucleotide for RPS25 Gene>>

Firstly, the single-stranded antisense oligonucleotides specified in Table 2-1 to Table 2-4, Table 3-1, Table 3-2, and Table 4 were designed. For some of the designed ones, a single-stranded antisense oligonucleotide for RPS25 gene was prepared in the manner described below.

A single-stranded antisense oligonucleotide that included, as a modified nucleic acid, 2'-O-methyl nucleic acid, 2'-MOE nucleic acid, AmNA, scpBNA, 5'-CP nucleic acid, and/or GuNA, and/or a nucleic acid whose nucleobase moiety was 5-methylcytosine was synthesized, with the use of an automatic nucleic-acid synthesizer (model nS-8, manufactured by GeneDesign), in a scale of 0.2 μmol. The chain length expansion was carried out by following a standard phosphoramidite protocol. It should be noted that when adding a monomer having a levulinyl protecting group at 5' position, the solid support was treated in 0.5-M hydrazine hydrate in pyridine/acetic acid (1:1 v/v) for 1 hour at room temperature outside the synthesis chamber, and after deprotection, the solid support was attached to the synthesis apparatus to resume synthesis. As the solid support, CPG resin was used. For sulfidation for forming a phosphorothioated (PS) backbone, DDTT (((Dimethylamino-methylidene)amino)-3H-1,2,4-dithiazaoline-3-thione) and/or the like was used. The single-stranded antisense oligonucleotide including 2'-MOE nucleic acid, AmNA, and/or scpBNA was obtained in the form where the terminal 5' hydroxy group was not protected by DMTr (4,4'-dimethoxytrityl) group and the 3' position was supported on the solid phase. Then, the single-stranded antisense oligonucleotide was removed from the solid support by alkali treatment, and then collected in the form of solution. Subsequently, solvent was distilled off from the solution thus collected, and thereby a crude product was obtained. The resulting crude product was purified by reversed-phase HPLC, and thereby a purified single-stranded antisense oligonucleotide was obtained. The purity and the structure of the resulting single-stranded antisense oligonucleotide were checked by LC-MS (manufactured by Waters).

In Table 2-1 to Table 2-4, Table 3-1, and Table 3-2 below, single-stranded antisense oligonucleotides produced by the above-described method are listed. The single-stranded antisense oligonucleotides listed in Table 2-1 to Table 2-4, Table 3-1, and Table 3-2 are single-stranded antisense oligonucleotides for human RPS25 mRNA (SEQ ID NO: 1).

[Table 2-1]

| Ex. | Sequence name | Antisense oligonucleotide sequence (5'-3') | X-Y-Z | Mw (actual) | SEQ ID NO: |
|---|---|---|---|---|---|
| 1 | h451-465-AQ | T(Y)^T(Y)-5(Y)^5(x)-A(5'-CP)^a-A(5'-CP)^t^g^t^a^5(x)^A(Y)-G(Y)^5(S) | 3-9-3 | 5134.2 | 5 |
| 2 | h451-465-AR | T(Y)^T(Y)^5(Y)-5(5'-CP)-A(5'-CP)-a^a^t^g^t^a^5(x)^A(Y)-G(Y)^5(S) | 3-9-3 | 5133.2 | 6 |
| 3 | h451-465-AS | T(GtB)^T(GtB)-5(GtB)^5(x)-A(5'-CP)^a-A(5'-CP)^t^g^t^a^5(x)^A(Y)^G(Y)^5(S) | 3-9-3 | 5360.3 | 7 |

(continued)

| Ex. | Sequence name | Antisense oligonucleotide sequence (5'-3') | X-Y-Z | Mw (actual) | SEQ ID NO: |
|---|---|---|---|---|---|
| 4 | h451-465-AT | T(S)-T(Y)^5(Y)-5(x)-A(5'-CP)-A(5'-CP)-A(5'-CP)^t^g^t^a^5(x)^A(Y)^G(Y)^5(S) | 3-9-3 | 5142.9 | 8 |
| 5 | h451-465-AU | T(Y)^T(Y)^5(Y)-5(x)-A(5'-CP)-A(5'-CP)-A(5'-CP)^t^g^t^a^5(x)^A(Y)^G(Y)^5(S) | 3-9-3 | 5159.7 | 9 |
| 6 | h451-465-AW | T(Y)^T(Y)-5(Y)^5(x)-A(5'-CP)^A(5'-CP)-A(5'-CP)^t^g^t^a^5(x)^A(Y)-G(Y)^5(S) | 3-9-3 | 5158.6 | 10 |
| 7 | h451-465-AX | T(Y)^T(Y)^5(Y)-5(x)-A(5'-CP)-A(5'-CP)^A(5'-CP)^t^g^t^a^5(x)^A(Y)-G(Y)^5(S) | 3-9-3 | 5158.0 | 11 |
| 8 | h451-465-AY | T(Y)^T(Y)-5(Y)-5(x)-A(5'-CP)^A(5'-CP)^A(5'-CP)^t^g^t^a^5(x)^A(Y)-G(Y)^5(S) | 3-9-3 | 5159.9 | 12 |
| 9 | h451-465-AZ | T(Y)^T(GtB)^5(GtB)-5(x)-A(5'-CP)-A(5'-CP)-A(5'-CP)^t^g^t^a^5(x)^A(Y)^G(Y)^5(S) | 3-9-3 | 5300.1 | 13 |
| 10 | h451-465-BA | T(GtB)^T(GtB)^5(GtB)-5(x)-A(5'-CP)-A(5'-CP)-A(5'-CP)^t^g^t^a^5(x)^A(Y)^G(Y)^5(S) | 3-9-3 | 5370.3 | 14 |
| 11 | h451-465-BB | T(S)-T(S)-5(S)-5(5'-CP)-A(5'-CP)-A(5'-CP)-A(5'-CP)^t^g^t^a^5(x)-A(S)^G(S)^5(S) | 3-9-3 | 5133.0 | 15 |
| 12 | h451-465-BC | T(S)-T(Y)^5(Y)-5(5'-CP)-A(5'-CP)-A(5'-CP)-A(5'-CP)^t^g^t^a^5(x)^A(Y)^G(Y)^5(S) | 3-9-3 | 5169.0 | 16 |
| 13 | h451-465-BD | T(Y)-T(Y)-5(Y)^5(5'-CP)^A(5'-CP)^a^a^t^g^t^A(5'-CP)^5(5'-CP)^A(Y)-G(Y)-5(S) | 3-9-3 | 5185.2 | 17 |

[Table 2-2]

| Ex. | Sequence name | Antisense oligonucleotide sequence (5'-3') | X-Y-Z | Mw (actual) | SEQ ID NO: |
|---|---|---|---|---|---|
| 14 | h451-465-BE | T(Y)^T(Y)^5(Y)-5(5'-CP)-A(5'-CP)-A(5'-CP)-A(5'-CP)^t^g^t^a^5(x)^A(Y)^G(Y)^5(S) | 3-9-3 | 5185.1 | 18 |
| 15 | h451-465-BF | T(Y)^T(Y)-5(Y)^5(5'-CP)-A(5'-CP)^A(5'-CP)-A(5'-CP)^t^g^t^a^5(x)^A(Y)-G(Y)^5(S) | 3-9-3 | 5185.7 | 19 |
| 16 | h451-465-BG | T(Y)^T(Y)^5(Y)-5(5'-CP)-A(5'-CP)-A(5'-CP)^A(5'-CP)^t^g^t^a^5(x)^A(Y)-G(Y)^5(S) | 3-9-3 | 5184.4 | 20 |
| 17 | h451-465-BH | T(Y)^T(Y)-5(Y)-5(5'-CP)-A(5'-CP)^A(5'-CP)^A(5'-CP)^t^g^t^a^5(x)^A(Y)-G(Y)^5(S) | 3-9-3 | 5185.0 | 21 |
| 18 | h451-465-BI | T(S)^T(S)^5(S)-5(5'-CP)-A(5'-CP)-A(5'-CP)-A(5'-CP)^t^g^t^a^5(x)^A(S)^G(S)^5(S) | 3-9-3 | 5181.0 | 22 |
| 19 | h451-465-BJ | T(Y)^T(GtB)^5(GtB)-5(5'-CP)-A(5'-CP)-A(5'-CP)-A(5'-CP)^t^g^t^a^5(x)^A(Y)^G(Y)^5(S) | 3-9-3 | 5326.2 | 23 |
| 20 | h451-465-BK | T(Y)^T(GtB)-5(GtB)^5(5'-CP)-A(5'-CP)^A(5'-CP)-A(5'-CP)^t^g^t^a^5(x)^A(Y)-G(Y)^5(S) | 3-9-3 | 5326.2 | 24 |
| 21 | h451-465-BL | T(Y)^T(GtB)^5(GtB)-5(5'-CP)-A(5'-CP)-A(5'-CP)^A(5'-CP)^t^g^t^a^5(x)^A(Y)-G(Y)^5(S) | 3-9-3 | 5326.3 | 25 |
| 22 | h451-465-BM | T(Y)^T(GtB)-5(GtB)-5(5'-CP)-A(5'-CP)^A(5'-CP)^A(5'-CP)^t^g^t^a^5(x)^A(Y)-G(Y)^5(S) | 3-9-3 | 5326.3 | 26 |

(continued)

| Ex. | Sequence name | Antisense oligonucleotide sequence (5'-3') | X-Y-Z | Mw (actual) | SEQ ID NO: |
|---|---|---|---|---|---|
| 23 | h451-465-BN | T(GtB)^T(GtB)^5(GtB)-5(5'-CP)-A(5'-CP)-A(5'-CP)-A(5'-CP)^t^g^t^a^5(x)^A(Y)^G(Y)^5(S) | 3-9-3 | 5396.4 | 27 |
| 24 | h451-465-BO | T(S)-T(Y)^5(Y)-5(5'-CP)-A(5'-CP)-A(5'-CP)-A(5'-CP)^t^g^t^a^5(5'-CP)^A(Y)^G(Y)^5(S) | 3-9-3 | 5195.0 | 28 |
| 25 | h451-465-BP | T(Y)^T(Y)^5(Y)-5(5'-CP)-A(5'-CP)-A(5'-CP)-A(5'-CP)^t^g^t^a^5(5'-CP)^A(Y)^G(Y)^5(S) | 3-9-3 | 5210.4 | 29 |
| 26 | h451-465-BQ | T(Y)^T(Y)-5(Y)^5(5'-CP)-A(5'-CP)^A(5'-CP)-A(5'-CP)^t^g^t^a^5(5'-CP)^A(Y)-G(Y)^5(S) | 3-9-3 | 5211.1 | 30 |

[Table 2-3]

| Ex. | Sequence name | Antisense oligonucleotide sequence (5'-3') | X-Y-Z | Mw (actual) | SEQ ID NO: |
|---|---|---|---|---|---|
| 27 | h451-465-BR | T(Y)^T(Y)^5(Y)-5(5'-CP)-A(5'-CP)-A(5'-CP)^A(5'-CP)^t^g^t^a^5(5'-CP)^A(Y)-G(Y)^5(S) | 3-9-3 | 5210.5 | 31 |
| 28 | h451-465-BS | T(Y)^T(Y)-5(Y)-5(5'-CP)-A(5'-CP)^A(5'-CP)^A(5'-CP)^t^g^t^a^5(5'-CP)^A(Y)-G(Y)^5(S) | 3-9-3 | 5211.4 | 32 |
| 29 | h451-465-BT | T(Y)^T(GtB)^5(GtB)-5(5'-CP)-A(5'-CP)-A(5'-CP)-A(5'-CP)^t^g^t^a^5(5'-CP)^A(Y)^G(Y)^5(S) | 3-9-3 | 5352.3 | 33 |
| 30 | h451-465-BU | T(GtB)^T(GtB)^5(GtB)-5(5'-CP)-A(5'-CP)-A(5'-CP)-A(5'-CP)^t^g^t^a^5(5'-CP)^A(Y)^G(Y)^5(S) | 3-9-3 | 5422.3 | 34 |
| 31 | h450-465-E | T(Y)^T(Y)-5(S)-5(5'-CP)-A(5'-CP)^a^a^t^g^t^a^5(5'-CP)-A(Y)-G(Y)-5(Y)^T(S) | 3-9-4 | 5499.7 | 35 |
| 32 | h450-465-F | T(Y)^T(Y)-5(S)-5(5'-CP)-A(5'-CP)-A(5'-CP)^a^t^g^t^a^5(5'-CP)-A(Y)-G(Y)-5(Y)^T(S) | 3-9-4 | 5510.3 | 36 |
| 33 | h450-465-G | T(Y)^T(Y)-5(S)-5(5'-CP)-A(5'-CP)-A(5'-CP)-A(5'-CP)^t^g^t^a^5(x)^A(Y)-G(Y)-5(Y)^T(S) | 3-9-4 | 5512.1 | 37 |
| 34 | h450-466-C | T(Y)^T(Y)-T(Y)-5(5'-CP)-5(5'-CP)^a^a^a^t^g^t^a^5(x)^A(5'-CP)-G(Y)-5(Y)^T(S) | 3-11-3 | 5783.4 | 38 |
| 35 | h451-467-G | T(Y)^T(m)^T(m)-T(Y)-5(5'-CP)-5(5'-CP)^a^a^a^t^g^t^a^5(5'-CP)-A(Y)-G(Y)^5(S) | 4-10-3 | 5875.0 | 39 |
| 36 | h450-466-D | T(Y)^T(Y)-T(Y)-5(5'-CP)-5(5'-CP)-A(5'-CP)^a^a^t^g^t^a^5(x)^A(5'-CP)-G(Y)-5(Y)^T(S) | 3-11-3 | 5794.3 | 40 |
| 37 | h450-466-E | T(Y)^T(Y)-T(Y)-5(5'-CP)-5(5'-CP)-A(5'-CP)-A(5'-CP)^a^t^g^t^a^5(x)^a^G(Y)-5(Y)^T(S) | 3-11-3 | 5793.3 | 41 |
| 38 | h451-467-H | T(Y)^T(m)^T(m)-T(Y)-5(5'-CP)-5(5'-CP)-A(5'-CP)^a^a^t^g^t^a^5(5'-CP)-A(Y)-G(Y)^5(S) | 4-10-3 | 5887.0 | 42 |
| 39 | h451-467-I | T(Y)^T(m)^T(m)-T(Y)-5(5'-CP)-5(5'-CP)-A(5'-CP)-A(5'-CP)^a^t^g^t^a^5(x)^A(Y)-G(Y)^5(S) | 4-10-3 | 5888.4 | 43 |

[Table 2-4]

| Ex. | Sequence name | Antisense oligonucleotide sequence (5'-3') | X-Y-Z | Mw (actual) | SEQ ID NO: |
|---|---|---|---|---|---|
| 40 | h450-467-D | T(Y)^T(Y)-T(Y)-T(5'-CP)-5(5'-CP)^5(x)^a^a^t^g^t ^a^5(x)^A(5'-CP)-G(Y)-5(Y)^T(S) | 3-12-3 | 6103.0 | 44 |
| 41 | h450-467-E | T(Y)^T(Y)-T(Y)-T(5'-CP)-5(5'-CP)-5(5'-CP)^a^a^t^ g^t^a^5(x)^A(5'-CP)-G(Y)-5(Y)^T(S) | 3-12-3 | 6113.6 | 45 |
| 42 | h450-467-F | T(Y)^T(Y)-T(Y)-T(5'-CP)-5(5'-CP)-5(5'-CP)-A(5'-CP)^a ^a^t^g^t^a^5(x)^a^G(Y)-5(Y)^T(S) | 3-12-3 | 6113.8 | 46 |
| 43 | h451-469-E | A(m)^T(m)-T(m)-T(m)-t-T(5'-CP)-5(5'-CP)^5(x)^a^a ^a^t^g^t^a^5(GtB)-A(GtB)^G(GtB)^5(m) | 4-11-4 | 6808.6 | 47 |
| 44 | h451-469-F | A(m)^T(m)-T(m)-T(m)-T(m)^T(5'-CP)-5(5'-CP)^5(x)^ a^a^a^t^g^T(5'-CP)^A(m)-5(m)-A(m)^G(m)^5(m) | 5-9-5 | 6829.6 | 48 |
| 45 | h451-469-G | A(Y)^T(Y)-T(Y)-T(5'-CP)-T(5'-CP)-T(5'-CP)^5(x)^5(x) ^a^a^a^t^g^t-A(5'-CP)^5(S)-A(Y)^G(Y)^5(S) | 3-12-4 | 6498.1 | 49 |
| 46 | h451-469-H | A(Y)^T(Y)-T(Y)-T(5'-CP)-T(5'-CP)-T(5'-CP)-5(5'-CP)^5 (x)^a^a^a^t^g^t^a^5(S)-A(Y)^G(Y)^5(S) | 3-12-4 | 6498.1 | 50 |
| 47 | h451-469-I | A(Y)^T(Y)-T(Y)-T(5'-CP)-T(5'-CP)-T(5'-CP)-5(5'-CP)^5 (x)^a^a^a^t^g^t-A(5'-CP)^5(S)-A(Y)^G(Y)^5(S) | 3-12-4 | 6508.1 | 51 |
| 48 | h451-469-J | A(Y)^T(Y)-T(Y)-T(5'-CP)-T(5'-CP)-T(5'-CP)^5(x)^5(x) ^a^a^a^t^g^T(5'-CP)-A(5'-CP)-5(S)-A(Y)-G(Y)^5(S) | 3-12-4 | 6492.1 | 52 |
| 49 | h451-469-K | A(Y)^T(Y)-T(Y)-T(5'-CP)-T(5'-CP)-T(5'-CP)^5(x)^5(x) ^a^a^a^t^g^T(5'-CP)-A(5'-CP)-5(GtB)-A(GtB)-G(GtB) ^5(S) | 3-12-4 | 6703.5 | 53 |
| 50 | h453-472-B | T(Y)^T(Y)-T(Y)-A(5'-CP)-T(5'-CP)-T(5'-CP)-T(5'-CP)^t ^t^5(x)^5(x)^a^a^a^t-G(5'-CP)^T(GtB)-A(Y)^5(Y)^ A(GtB) | 3-13-4 | 6946.6 | 54 |
| 51 | h453-472-D | T(GtB)^T(m)-T(m)-A(m)-t-T(5'-CP)^t^t^t^5(x)^5(x) ^a^a^a^t^g^T(m)-A(m)-5(GtB)^A(GtB) | 4-12-4 | 7078.8 | 55 |
| 52 | h453-472-E | T(m)^T(m)^T(m)-A(GtB)-t-T(5'-CP)^t-T(5'-CP)^t^5(x )^5(x)^a^a^a^t^g^T(GtB)-A(m)-5(m)^A(GtB) | 4-12-4 | 7104.9 | 56 |

[Table 3-1]

| Ref. Ex. | Sequence name | Antisense oligonucleotide sequence (5'-3') | X-Y-Z | Mw (actual) | SEQ ID NO: |
|---|---|---|---|---|---|
| 1 | h451-465-C | T(Y)-T(Y)-5(Y)^c^a^a^a^t^g^t^a^c^A(Y)-G(Y)-5(S) | 3-9-3 | 5053.7 | 57 |
| 2 | h451-465-N | T(Y)^T(Y)-5(Y)-5(5'-CP)-A(5'-CP)^a^a^t^g^t^a^c^A(Y)-G( Y)^5(S) | 3-9-3 | 5119.2 | 58 |
| 3 | h451-465-O | T(Y)^T(Y)^5(Y)-5(5'-CP)-A(5'-CP)-A(5'-CP)-A(5'-CP)^t^g^t ^a^c^A(Y)^G(Y)^5(S) | 3-9-3 | 5171.2 | 59 |
| 4 | h451-465-Q | T(Y)-T(Y)^5(5'-CP)^c^a^a^a^t^g^t^a^c^A(5'-CP)^G(Y)-5 (S) | 3-9-3 | 5028.4 | 60 |
| 5 | h451-465-R | T(Y)^T(5'-CP)^5(5'-CP)^c^a^a^a^t^g^t^a^c^A(Y)-G(Y)-5 (S) | 3-9-3 | 5027.2 | 61 |

(continued)

| Ref. Ex. | Sequence name | Antisense oligonucleotide sequence (5'-3') | X-Y-Z | Mw (actual) | SEQ ID NO: |
|---|---|---|---|---|---|
| 6 | h451-465-A B | T(Y)-T(Y)-5(Y)^5(x)^a^a^a^t^g^t^a^5(x)^A(Y)-G(Y)-5(S) | 3-9-3 | 5080.2 | 62 |
| 7 | h451-465-AP | T(Y)^T(GtB)^5(GtB)^5(x)^a^a^a^t^g^t^a^5(x)^A(Y)-G(Y)-5(S) | 3-9-3 | 5254.1 | 63 |
| 8 | h451-465-AL | T(Y)^T(Y)^5(Y)-5(5′-CP)-A(5′-CP)^a^a^t^g^t^A(5′-CP)-5(5′-CP)-A(Y)^G(Y)^5(S) | 3-9-3 | 5184.3 | 64 |
| 9 | h451-465-A M | T(Y)^T(Y)-5(Y)^5(5′-CP)-A(5′-CP)^a^a^t^g^t^A(5′-CP)-5(5′-CP)^A(Y)-G(Y)^5(S) | 3-9-3 | 5185.0 | 65 |
| 10 | h450-465-C | T(Y)-T(Y)-5(S)^c^a^a^a^t^g^t^a^c^A(Y)-G(Y)-5(Y)-T(S) | 3-9-4 | 5411.6 | 66 |
| 11 | h325-341-B | T(Y)-T(Y)-T(Y)^a^c^t^a^a^g^g^a^g^c^t^5(Y)-5(Y)-T(S) | 3-11-3 | 5710.2 | 67 |
| 12 | h325-341-L | T(Y)-T(Y)-T(Y)-A(5′-CP)^c^t^a^a^g^g^a^g^c^t^5(Y)-5(Y)-T(S) | 3-11-3 | 5719.1 | 68 |
| 13 | h325-341-M | T(Y)-T(Y)-T(Y)^a-5(5′-CP)^t^a^a^g^g^a^g^c^t^5(Y)-5(Y)-T(S) | 3-11-3 | 5732.7 | 69 |
| 14 | h325-341-N | T(Y)-T(Y)-T(Y)^a^c-T(5′-CP)^a^a^g^g^a^g^c^t^5(Y)-5(Y)-T(S) | 3-11-3 | 5719.2 | 70 |
| 15 | h325-341-O | T(Y)-T(Y)-T(Y)^a^c^t-A(5′-CP)^a^g^g^a^g^c^t^5(Y)-5(Y)-T(S) | 3-11-3 | 5719.2 | 71 |
| 16 | h325-341-P | T(Y)-T(Y)-T(Y)^a^c^t^a-A(5′-CP)^g^g^a^g^c^t^5(Y)-5(Y)-T(S) | 3-11-3 | 5718.3 | 72 |

[Table 3-2]

| Ref. Ex. | Sequence name | Antisense oligonucleotide sequence (5'-3') | X-Y-Z | Mw (actual) | SEQ ID NO: |
|---|---|---|---|---|---|
| 17 | h325-341-Q | T(Y)-T(Y)-T(Y)^a^c^t^a^a-G(5′-CP)^g^a^g^c^t^5(Y)-5(Y)-T(S) | 3-11-3 | 5719.2 | 73 |
| 18 | h325-341-R | T(Y)-T(Y)-T(Y)^a^c^t^a^a^g-G(5′-CP)^a^g^c^t^5(Y)-5(Y)-T(S) | 3-11-3 | 5719.3 | 74 |
| 19 | h325-341-S | T(Y)-T(Y)-T(Y)^a^c^t^a^a^g^g-A(5′-CP)^g^c^t^5(Y)-5(Y)-T(S) | 3-11-3 | 5718.9 | 75 |
| 20 | h325-341-T | T(Y)-T(Y)-T(Y)^a^c^t^a^a^g^g^a-G(5′-CP)^c^t^5(Y)-5(Y)-T(S) | 3-11-3 | 5718.9 | 76 |
| 21 | h325-341-U | T(Y)-T(Y)-T(Y)^a^c^t^a^a^g^g^a^g-5(5′-CP)^t^5(Y)-5(Y)-T(S) | 3-11-3 | 5734.0 | 77 |
| 22 | h325-341-V | T(Y)-T(Y)-T(Y)^a^c^t^a^a^g^g^a^g^c-T(5′-CP)^5(Y)-5(Y)-T(S) | 3-11-3 | 5719.0 | 78 |
| 23 | h325-341-I | T(Y)^T(Y)-T(Y)-A(5′-CP)-5(5′-CP)^t^a^a^g^g^a^g^c^t^5(Y)-5(Y)^T(S) | 3-11-3 | 5775.4 | 79 |
| 24 | h325-341-J | T(Y)^T(Y)^T(Y)-A(5′-CP)-5(5′-CP)^t^a^a^g^g^a^g^5(5′-CP)-T(5′-CP)-5(Y)^5(Y)^T(S) | 3-11-3 | 5841.9 | 80 |

(continued)

| Ref. Ex. | Sequence name | Antisense oligonucleotide sequence (5'-3') | X-Y-Z | Mw (actual) | SEQ ID NO: |
|---|---|---|---|---|---|
| 25 | h325-341-K | T(Y)^T(Y)-T(Y)^A(5′-CP)-5(5′-CP)^t^a^a^g^g^a^g^5(5′-CP)-T(5′-CP)^5(Y)-5(Y)^T(S) | 3-11-3 | 5841.0 | 81 |
| 26 | h450-466-B | T(Y)-T(Y)-T(Y)^c^c^a^a^a^t^g^t^a^c^a^G(Y)-5(Y)-T(S) | 3-11-3 | 5679.7 | 82 |
| 27 | h450-467-C | T(Y)-T(Y)-T(Y)^t^5(x)^5(x)^a^a^a^t^g^t^a^5(x)^a^G(Y)-5(Y)-T(S) | 3-12-3 | 6103.0 | 83 |
| 28 | h451-469-A | A(m)^T(m)-T(m)-T(m)-T(m)^t^5(x)^5(x)^a^a^a^t^g^t^A(m)-5(m)-A(m)-G(m)^5(m) | 5-9-5 | 6751.5 | 84 |
| 29 | h451-469-D | A(Y)-T(Y)-T(Y)^t^t^t^5(x)^5(x)^a^a^a^t^g^t^a^5(S)-A(Y)-G(Y)-5(S) | 3-12-4 | 6409.9 | 85 |
| 30 | h453-472-A | T(Y)-T(Y)-T(Y)^a^t^t^t^t^t^5(x)^5(x)^a^a^a^t^g^T(GtB)-A(Y)-5(Y)-A(GtB) | 3-13-4 | 6848.4 | 86 |
| 31 | h453-472-C | T(GtB)^T(m)-T(m)-A(m)-t^t^t^t^t^5(x)^5(x)^a^a^a^t^g^T(m)-A(m)-5(GtB)^A(GtB) | 4-12-4 | 7068.8 | 87 |

[Table 4]

| Design Ex. | Sequence name | Antisense oligonucleotide sequence (5'-3') | X-Y-Z | SEQ ID NO: |
|---|---|---|---|---|
| 1 | h451-465-S | T(Y)-T(Y)-5(Y)-5(5'-CP)^a^a^a^t^g^t^a^c^A(Y)-G(Y)-5(S) | 3-9-3 | 88 |
| 2 | h451-465-T | T(Y)-T(Y)-5(Y)^c-A(5'-CP)^a^a^t^g^t^a^c^A(Y)-G(Y)-5(S) | 3-9-3 | 89 |
| 3 | h451-465-U | T(Y)-T(Y)-5(Y)^c^a-A(5'-CP)^a^t^g^t^a^c^A(Y)-G(Y)-5(S) | 3-9-3 | 90 |
| 4 | h451-465-V | T(Y)-T(Y)-5(Y)^c^a^a-A(5'-CP)^t^v^t^a^c^A(Y)-G(Y)-5(S) | 3-9-3 | 91 |
| 5 | h451-465-W | T(Y)-T(Y)-5(Y)^c^a^a^a-T(5'-CP)^g^t^a^c^A(Y)-G(Y)-5(S) | 3-9-3 | 92 |
| 6 | h451-465-X | T(Y)-T(Y)-5(Y)^c^a^a^a^t-G(5'-CP)^t^a^c^A(Y)-G(Y)-5(S) | 3-9-3 | 93 |
| 7 | h451-465-Y | T(Y)-T(Y)-5(Y)^c^a^a^a^t^g-T(5'-CP)^a^c^A(Y)-G(Y)-5(S) | 3-9-3 | 94 |
| 8 | h451-465-Z | T(Y)-T(Y)-5(Y)^c^a^a^a^t^g^t-A(5'-CP)^c^A(Y)-G(Y)-5(S) | 3-9-3 | 95 |
| 9 | h451-465-AA | T(Y)-T(Y)-5(Y)^c^a^a^a^t^g^t^a-5(5'-CP)^A(Y)-G(Y)-5(S) | 3-9-3 | 96 |
| 10 | h451-465-AC | T(Y)-T(Y)-5(Y)-5(5′-CP)^a^a^a^t^g^t^a^5(x)^A(Y)-G(Y)-5(S) | 3-9-3 | 97 |
| 11 | h451-465-AD | T(Y)-T(Y)-5(Y)^5(x)-A(5′-CP)^a^a^t^g^t^a^5(x)^A(Y)-G(Y)-5(S) | 3-9-3 | 98 |
| 12 | h451-465-AE | T(Y)-T(Y)-5(Y)^5(x)^a-A(5′-CP)^a^t^g^t^a^5(x)^A(Y)-G(Y)-5(S) | 3-9-3 | 99 |
| 13 | h451-465-AF | T(Y)-T(Y)-5(Y)^5(x)^a^a-A(5′-CP)^t^g^t^a^5(x)^A(Y)-G(Y)-5(S) | 3-9-3 | 100 |
| 14 | h451-465-AG | T(Y)-T(Y)-5(Y)^5(x)^a^a^a-T(5′-CP)^g^t^a^5(x)^A(Y)-G(Y)-5(S) | 3-9-3 | 101 |
| 15 | h451-465-AH | T(Y)-T(Y)-5(Y)^5(x)^a^a^a^t-G(5′-CP)^t^a^5(x)^A(Y)-G(Y)-5(S) | 3-9-3 | 102 |
| 16 | h451-465-AI | T(Y)-T(Y)-5(Y)^5(x)^a^a^a^t^g-T(5′-CP)^a^5(x)^A(Y)-G(Y)-5(S) | 3-9-3 | 103 |

(continued)

| Design Ex. | Sequence name | Antisense oligonucleotide sequence (5'-3') | X-Y-Z | SEQ ID NO: |
|---|---|---|---|---|
| 17 | h451-465-AJ | T(Y)-T(Y)-5(Y)^5(x)^a^a^a^t^g^t-A(5'-CP)^5(x)^A(Y)-G(Y)-5(S) | 3-9-3 | 104 |
| 18 | h451-465-AK | T(Y)-T(Y)-5(Y)^5(x)^a^a^a^t^g^t^a-5(5'-CP)^A(Y)-G(Y)-5(S) | 3-9-3 | 105 |

Herein, symbols or expressions described below may be used to represent corresponding structures.

[Chem. 2]

—

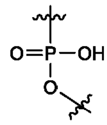

^

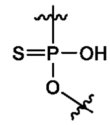

=

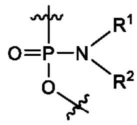

x

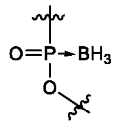

a

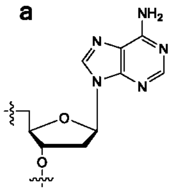

A

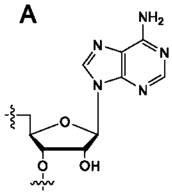

A(M)

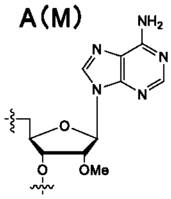

A(m)

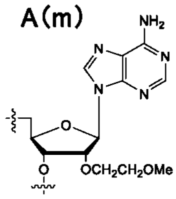

A(L)

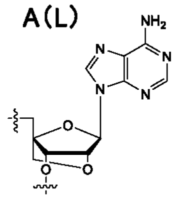

A(Y)

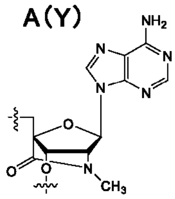

A(Gx)

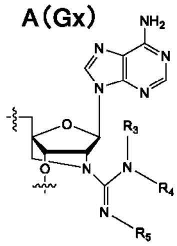

A(S)

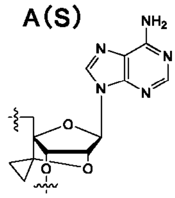

A(5'—CP)

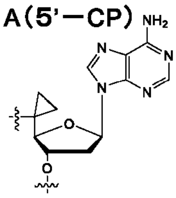

A(Mx)

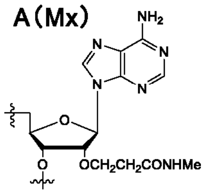

c

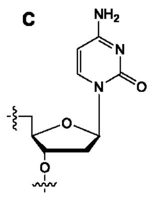

5(x)

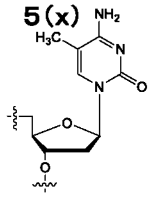

C

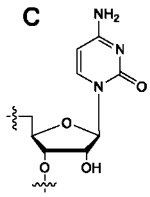

C(M)

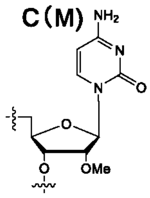

5(m)

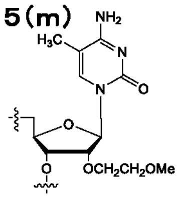

5(L)

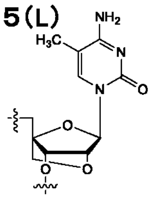

5(Y)

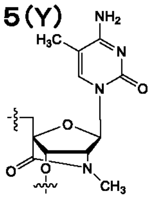

5(Gx)

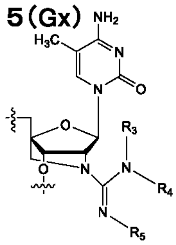

5(S)

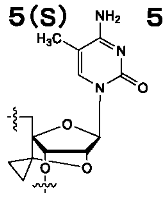

5(5'—CP)

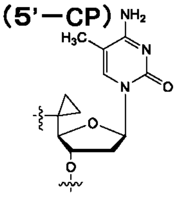

C(Mx)

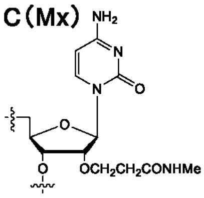

[Chem. 3]

g
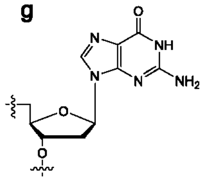
G
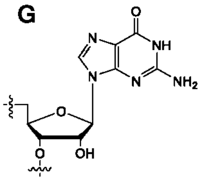
G(M)
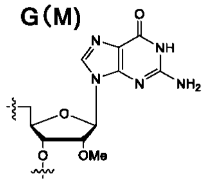
G(m)
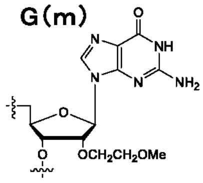
G(L)
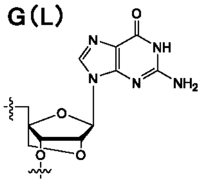

G(Y)
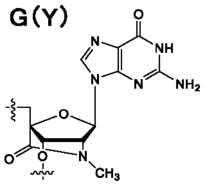
G(Gx)
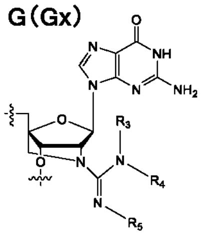
G(S)
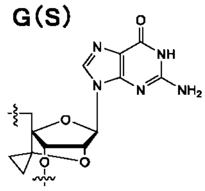
G(5'−CP)
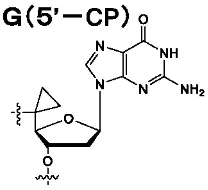
G(Mx)
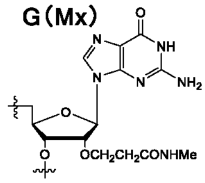

t
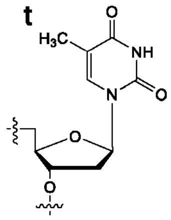
U
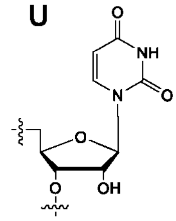
U(M)
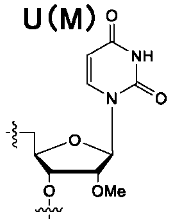
T(m)
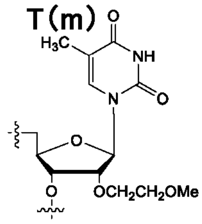
T(L)
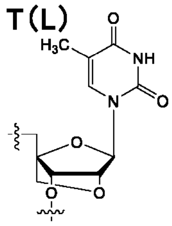

T(Y)
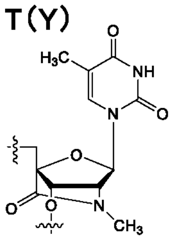
T(Gx)
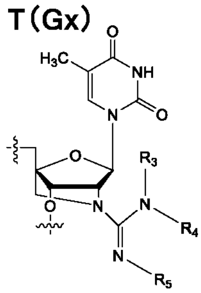
T(S)
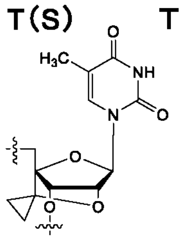
T(5'−CP)
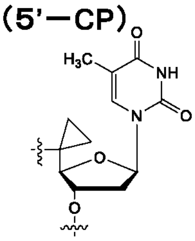
U(Mx)
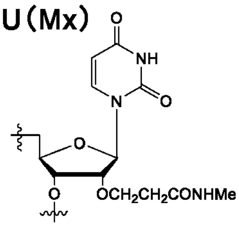

In the above-described structural formulae, $R^1$ and $R^2$ independently represents a hydrogen atom, or a linear or branched alkyl group having 1 to 3 carbon atoms. Each of $R^1$ and $R^2$ involving in a bond represented by "=" above represents a methyl group. $R^3$, $R^4$, and $R^5$ independently represents a hydrogen atom, a linear or branched alkyl group having 1 to 7 carbon atoms, or a cycloalkyl group having 3 to 7 carbon atoms. GuNA represented by "Gx" above is represented by "Gm" when $R^3$ and $R^5$ are both hydrogen atoms and $R^4$ is a methyl group; it is represented by "Gdm" when $R^3$ is a hydrogen atom and $R^4$ and $R^5$ are both methyl groups; and it is represented by "GtB" when $R^3$ and $R^5$ are both hydrogen atoms and $R^4$ is a tert-butyl group.

<<Evaluation of RPS25 Gene Expression>>

Evaluation of RPS25 gene expression was carried out for respective single-stranded antisense oligonucleotides thus produced, by expression evaluation with the use of human fetal kidney cells. Evaluation of RPS25 gene expression can also be carried out with the use of neurons derived from human iPS cells. Evaluation of gene expression in the present examples means evaluation of the amount of mRNA by measuring the amount of complementary DNA (cDNA) obtained by reverse transcription reaction. In the following, the specific procedure of each type of expression evaluations will be described.

<Expression Evaluation with Use of Human Fetal Kidney Cells>

Human fetal kidney cells HEK293T (ATCC (registered trademark) CRL-3216 (trademark)) were cultured in a culture medium under the conditions of 37°C and 5% $CO_2$. The culture medium used for HEK293T cells had the composition as described below.

Composition of Culture Medium for HEK293T Cells

Dulbecco modified Eagle medium (DMEM): manufactured by SIGMA, Cat# D6429
10% fetal bovine serum (FBS): manufactured by biowest, Cat# S1820
100-Fold diluted penicillin-streptomycin mixed solution: manufactured by Nacalai Tesque, Cat# 09367-34 (penicillin 10000 units/ml, streptomycin 10000 μg/ml, a stabilizer contained)

Firstly, the day before measuring the amount of expression of human RPS25 gene, HEK293T cells were plated in a 96-well plate (12000 cells/well) and cultured overnight under the conditions of 37°C and 5% $CO_2$. Subsequently, the cells were transfected by lipofection with the single-stranded antisense oligonucleotide diluted with phosphate-buffered physiological saline (PBS) (in a final concentration of 0.5 nM, 5 nM, 15 nM, or 50 nM). As the negative control group, cells transfected with PBS that was free of single-stranded antisense oligonucleotide were used. The transfected cells were cultured in a growth medium under the conditions of 37°C and 5% $CO_2$ for 48 hours. Subsequently, the growth medium was removed, and with the use of Taqman Fast Cells-to-CT Kit (manufactured by Thermo Fisher Scientific, Cat# 4399003), reverse transcription reaction was carried out for the extracted total RNA. With the use of the resulting complementary DNA (cDNA) obtained from the reverse transcription reaction, Taqman gene expression assays (manufactured by Applied Biosystems), and a gene-specific probe designed in advance (see below), real-time PCR was carried out (40 cycles of 95°C for 3 seconds and 60°C for 30 seconds).

List of Gene-Specific Probes Used in Evaluation of Human RPS25 Gene Expression

Human RPS25: Hs01568661_g1
Human GAPDH: 4326317E (internal control)

The expression rate of human RPS25 mRNA for respective single-stranded antisense oligonucleotides for human RPS25 mRNA, as determined by the above-described method, is shown in Table 5-1 to Table 5-3 and Table 6-1 to Table 6-2 (in the section "hRPS25 expression rate"). At this time, the expression rate of human RPS25 mRNA determined for the negative control group was defined as 1.00. A single-stranded antisense oligonucleotide with an expression rate of 0.80 or less was judged as capable of reducing expression of human RPS25 mRNA. "-" in the tables means that no measurement was performed. Generally, it is expected that when mRNA expression is reduced, subsequent protein translation and the like are also reduced. Hence, it can be judged that a single-stranded antisense oligonucleotide with an expression rate of 0.80 or less is capable of modulating the function of human RPS25 gene.

[Table 5-1]

| Ex. | SEQ ID NO: | Sequence name | hRPS25 expression rate | | |
|---|---|---|---|---|---|
| | | | @0.5 nM | @5 nM | @50 nM |
| 1 | 5 | h451-465-AQ | - | - | - |
| 2 | 6 | h451-465-AR | 0.62 | 0.14 | 0.05 |
| 3 | 7 | h451-465-AS | - | - | - |
| 4 | 8 | h451-465-AT | - | - | - |
| 5 | 9 | h451-465-AU | 0.80 | 0.32 | 0.10 |
| 6 | 10 | h451-465-AW | - | - | - |
| 7 | 11 | h451-465-AX | - | - | - |
| 8 | 12 | h451-465-AY | - | - | 0.14 |
| 9 | 13 | h451-465-AZ | - | - | - |
| 10 | 14 | h451-465-BA | - | - | - |
| 11 | 15 | h451-465-BB | - | - | 0.44 |
| 12 | 16 | h451-465-BC | - | - | 0.14 |

(continued)

| Ex. | SEQ ID NO: | Sequence name | hRPS25 expression rate | | |
|---|---|---|---|---|---|
| | | | @0.5 nM | @5 nM | @50 nM |
| 13 | 17 | h451-465-BD | - | - | 0.27 |
| 14 | 18 | h451-465-BE | 0.88 | 0.49 | 0.10 |
| 15 | 19 | h451-465-BF | - | - | - |
| 16 | 20 | h451-465-BG | - | - | - |
| 17 | 21 | h451-465-BH | - | - | - |
| 18 | 22 | h451-465-BI | - | - | 0.32 |
| 19 | 23 | h451-465-BJ | - | - | - |
| 20 | 24 | h451-465-BK | - | - | 0.32 |

[Table 5-2]

| Ex. | SEQ ID NO: | Sequence name | hRPS25 expression rate | | |
|---|---|---|---|---|---|
| | | | @0.5 nM | @5 nM | @50 nM |
| 21 | 25 | h451-465-BL | - | - | - |
| 22 | 26 | h451-465-BM | - | - | - |
| 23 | 27 | h451-465-BN | - | - | 0.24 |
| 24 | 28 | h451-465-BO | - | - | 0.23 |
| 25 | 29 | h451-465-BP | - | - | 0.28 |
| 26 | 30 | h451-465-BQ | - | - | - |
| 27 | 31 | h451-465-BR | - | - | 0.47 |
| 28 | 32 | h451-465-BS | - | - | 0.31 |
| 29 | 33 | h451-465-BT | - | - | 0.66 |
| 30 | 34 | h451-465-BU | - | - | 0.29 |
| 31 | 35 | h450-465-E | - | - | 0.12 |
| 32 | 36 | h450-465-F | - | - | 0.13 |
| 33 | 37 | h450-465-G | - | - | - |
| 34 | 38 | h450-466-C | 0.59 | 0.18 | 0.05 |
| 35 | 39 | h451-467-G | - | - | - |
| 36 | 40 | h450-466-D | 0.44 | 0.10 | 0.04 |
| 37 | 41 | h450-466-E | 0.31 | 0.07 | 0.04 |
| 38 | 42 | h451-467-H | - | - | 0.08 |
| 39 | 43 | h451-467-I | - | - | - |
| 40 | 44 | h450-467-D | - | - | - |

[Table 5-3]

| Ex. | SEQ ID NO: | Sequence name | hRPS25 expression rate | | |
|---|---|---|---|---|---|
| | | | @0.5 nM | @5 nM | @50 nM |
| 41 | 45 | h450-467-E | - | - | - |
| 42 | 46 | h450-467-F | - | - | - |
| 43 | 47 | h451-469-E | 0.31 | 0.16 | 0.11 |

(continued)

| Ex. | SEQ ID NO: | Sequence name | hRPS25 expression rate | | |
|---|---|---|---|---|---|
| | | | @0.5 nM | @5 nM | @50 nM |
| 44 | 48 | h451-469-F | 1.14 | 1.15 | 0.90 |
| 45 | 49 | h451-469-G | 0.90 | 0.27 | 0.05 |
| 46 | 50 | h451-469-H | 0.79 | 0.15 | 0.09 |
| 47 | 51 | h451-469-I | 1.35 | 0.27 | 0.09 |
| 48 | 52 | h451-469-J | 0.89 | 0.32 | 0.10 |
| 49 | 53 | h451-469-K | 0.86 | 0.09 | 0.05 |
| 50 | 54 | h453-472-B | 0.31 | 0.12 | 0.04 |
| 51 | 55 | h453-472-D | 1.02 | 0.47 | 0.05 |
| 52 | 56 | h453-472-E | 1.06 | 0.77 | 0.07 |

[Table 6-1]

| Reference Ex. | SEQ ID NO: | Sequence name | hRPS25 expression rate | | |
|---|---|---|---|---|---|
| | | | @0.5 nM | @5 nM | @50 nM |
| 1 | 57 | h451-465-C | 0.25 | 0.12 | 0.04 |
| 2 | 58 | h451-465-N | - | - | 0.08 |
| 3 | 59 | h451-465-O | 0.92 | 0.52 | 0.06 |
| 4 | 60 | h451-465-Q | - | - | 0.86 |
| 5 | 61 | h451-465-R | - | - | 0.62 |
| 6 | 62 | h451-465-AB | 0.29 | 0.13 | 0.04 |
| 7 | 63 | h451-465-AP | - | - | - |
| 8 | 64 | h451-465-AL | 0.87 | 0.27 | 0.07 |
| 9 | 65 | h451-465-AM | - | - | - |
| 10 | 66 | h450-465-C | - | - | 0.03 |
| 11 | 67 | h325-341-B | - | - | 0.08 |
| 12 | 68 | h325-341-L | - | - | 0.31 |
| 13 | 69 | h325-341-M | - | - | 0.16 |
| 14 | 70 | h325-341-N | - | - | 0.26 |
| 15 | 71 | h325-341-O | - | - | 0.13 |

[Table 6-2]

| Reference Ex. | SEQ ID NO: | Sequence name | hRPS25 expression rate | | |
|---|---|---|---|---|---|
| | | | @0.5 nM | @5 nM | @50 nM |
| 16 | 72 | h325-341-P | - | - | 0.30 |
| 17 | 73 | h325-341-Q | - | - | 0.19 |
| 18 | 74 | h325-341-R | - | - | 0.29 |
| 19 | 75 | h325-341-S | - | - | 0.25 |
| 20 | 76 | h325-341-T | - | - | 0.23 |
| 21 | 77 | h325-341-U | - | - | 0.20 |
| 22 | 78 | h325-341-V | - | - | 0.22 |

(continued)

| Reference Ex. | SEQ ID NO: | Sequence name | hRPS25 expression rate | | |
|---|---|---|---|---|---|
| | | | @0.5 nM | @5 nM | @50 nM |
| 23 | 79 | h325-341-I | - | - | 0.27 |
| 24 | 80 | h325-341-J | - | - | 0.46 |
| 25 | 81 | h325-341-K | - | - | 0.25 |
| 26 | 82 | h450-466-B | 0.40 | 0.13 | 0.06 |
| 27 | 83 | h450-467-C | - | - | - |
| 28 | 84 | h451-469-A | 1.04 | 0.75 | 0.16 |
| 29 | 85 | h451-469-D | 0.60 | 0.11 | 0.05 |
| 30 | 86 | h453-472-A | 0.25 | 0.11 | 0.02 |
| 31 | 87 | h453-472-C | 0.87 | 0.52 | 0.06 |

<Expression Evaluation with Use of Mouse Primary Culture Neurons>

Mouse primary culture neurons are cultured in a culture medium under the conditions of 37°C and 5% $CO_2$. The culture medium used for mouse primary culture neurons has the composition as described below.

Composition of Culture Medium for Mouse Primary Culture Neurons

B-27 Electrophysiology Kit: manufactured by gibco, Cat# A1413701
100-Fold diluted 200-mM L-glutamine solution: manufactured by Nacalai Tesque, Cat# 16948-04
100-Fold diluted penicillin-streptomycin mixed solution: manufactured by Nacalai Tesque, Cat# 09367-34 (penicillin 10000 units/ml, streptomycin 10000 μg/ml, a stabilizer contained)

Firstly, mouse primary culture neurons (derived from mouse fetal cerebrum) are plated in a 96-well plate at 40000 cells/well, followed by culturing under the conditions of 37°C and 5% $CO_2$ for 5 days. Subsequently, the single-stranded antisense oligonucleotide diluted with phosphate-buffered physiological saline (PBS) (with a final concentration of 0.01 μM, 0.1 μM, or 1 μM) is added to the culture medium. For the negative control group, PBS that is free of single-stranded antisense oligonucleotide is added to the culture medium. The cells are cultured in the culture medium under the conditions of 37°C and 5% $CO_2$ for 48 hours. Subsequently, the culture medium is removed, and with the use of Taqman Fast Cells-to-CT Kit (manufactured by Thermo Fisher Scientific, Cat# 4399003), extracted total RNA is subjected to reverse transcription reaction. With the use of the resulting complementary DNA (cDNA) obtained from the reverse transcription reaction, Taqman gene expression assays (manufactured by Applied Biosystems), and a gene-specific probe designed in advance (see below), real-time PCR is carried out (40 cycles of 95°C for 3 seconds and 60°C for 30 seconds).

List of Gene-Specific Probes Used in Evaluation of Mouse RPS25 Gene Expression

Mouse Rps25: Mm02342783_g1
Mouse GAPDH: 4352339E (internal control)

<<Evaluation with Use of Motor Neurons Derived from Human iPS Cells>>

Human iPS cells are induced to differentiate into motor neurons, which are used for evaluation. Maintaining the cells and inducing differentiation are carried out in the media described below, under the conditions of 37°C and 5% $CO_2$.

List of Medium Compositions

(SNL cell medium)

DMEM (manufactured by Sigma-Aldrich, Cat# D6429),
100-Fold diluted penicillin-streptomycin mixed solution (manufactured by ThermoFisher Scientific, Cat# 15140-122)
10-Fold diluted fetal bovine serum (manufactured by ThermoFisher Scientific, Cat# 10437-028)

(iPS cell medium)

Medium for primate ES/iPS cells (manufactured by REPROCELL, Cat# RCHEMD001B)
100-Fold diluted penicillin-streptomycin mixed solution (manufactured by ThermoFisher Scientific, cat# 15140-122)

(Mixed medium A)

DMEM/Ham's F12 GlutaMAX (manufactured by ThermoFisher Scientific, Cat# 10565-018)
2 mM L-glutamine (manufactured by ThermoFisher Scientific, Cat# 25030-081)
Non-Essential Amino Acid (NEAA) (manufactured by ThermoFisher Scientific, Cat# 11140-050)
100-Fold diluted penicillin-streptomycin mixed solution (manufactured by ThermoFisher Scientific, cat# 15140-122)
2 $\mu$g/mL Heparin (manufactured by Sigma-Aldrich, H-4784)
N2 supplement (manufactured by ThermoFisher Scientific, Cat# 17502-048)

(Mixed medium B)

Neurobasal medium (manufactured by ThermoFisher Scientific, cat# 21103-049)
2 mM L-glutamine (manufactured by ThermoFisher Scientific, Cat# 25030-081)
Non-Essential Amino Acid (NEAA) (manufactured by ThermoFisher Scientific, Cat# 11140-050)
Antibiotic-Antimycotic (manufactured by ThermoFisher Scientific, cat# 15240-062)
2 $\mu$g/mL Heparin (manufactured by Sigma-Aldrich, H-4784)
N2 supplement (manufactured by ThermoFisher Scientific, Cat# 17502-048)
10 ng/mL IGF-1 (manufactured by PeproTech, cat# 100-11)
10 ng/mL Human CNTF (manufactured by PeproTech, cat# 450-13)
10 ng/mL Human GDNF (manufactured by R&D Systems, cat# 212-GD-050)
B27 supplement (manufactured by ThermoFisher Scientific, cat# 12587010)
200 $\mu$M Ascorbic acid (manufactured by Sigma-Aldrich, Cat# A5960)
10 ng/mL Human BDNF (manufactured by Peprotech, Cat# 450-02)

(Neuron medium)

Neurobasal medium Electro (manufactured by ThermoFisher Scientific, cat# A14098-01)
2 mM L-glutamine (manufactured by ThermoFisher Scientific, Cat# 25030-081)
Non-Essential Amino Acid (NEAA) (manufactured by ThermoFisher Scientific, Cat# 11140-050)
Antibiotic-Antimycotic (manufactured by ThermoFisher Scientific, cat# 15240-062)
2 $\mu$g/mL Heparin (manufactured by Sigma-Aldrich, H-4784)
N2 supplement (manufactured by ThermoFisher Scientific, Cat# 17502-048)
10 ng/mL IGF-1 (manufactured by PeproTech, cat# 100-11)
10 ng/mL Human CNTF (manufactured by PeproTech, cat# 450-13)
10 ng/mL Human GDNF (manufactured by R&D Systems, cat# 212-GD-050)
B27 supplement, Electro (manufactured by ThermoFisher Scientific, cat# A14097-01)
200 $\mu$M Ascorbic acid (manufactured by Sigma-Aldrich, Cat# A5960)

10 ng/mL Human BDNF (manufactured by PeproTech, Cat# 450-02)
25 μM 2-mercaptoethanol (manufactured by ThermoFisher Scientific, cat# 21985-0123)
0.1% bovine serum albumin (manufactured by Sigma-Aldrich, cat# A9576)

<Mitomycin Treatment of Feeder Cells>

As feeder cells for plating human iPS cells, SNL cells (manufactured by Cell Biolabs, Cat# CBA-316) treated with mitomycin are prepared. The mitomycin treatment of SNL cells is carried out in the manner described below. Firstly, 0.1% gelatin (manufactured by FUJIFILM Wako Pure Chemical, Cat# 190-15805) is added to a 10-cm petri dish (manufactured by IWAKI, Cat# 3020-100), which is then left to stand for at least 1 hour in an incubator under the conditions of 37°C and 5% $CO_2$ (hereinafter, this procedure may also be called "gelatin treatment"). Subsequently, excess gelatin is sucked out of the petri dish, and, with the use of the SNL cell medium, SNL cells which were thawed are plated in the petri dish at $1\times10^6$ to $2\times10^6$ cells per petri dish. Every 3 to 4 days, the cells are diluted 8 to 16 folds and passaged, until the cells proliferate into the necessary cell count. Then, in a 15-cm petri dish (manufactured by IWAKI, Cat# 3030-150) with 0.1-% gelatin treatment, the SNL cells are plated at $2\times10^6$ to $4\times10^6$ cells per petri dish. After the cells are cultured to 80 to 90% confluency, mitomycin C (manufactured by Kyowa Kirin, YJ code 4231400D1031) diluted to 0.4 mg/mL with the SNL cell medium is added to the petri dish in a final concentration of 6.2 μg/mL. The petri dish is left to stand in an incubator under the conditions of 37°C and 5% $CO_2$ for 2 hours and 15 minutes. Subsequently, the medium is removed from the petri dish, and the SNL cells are rinsed once with PBS. 2.5% trypsin/EDTA (manufactured by ThermoFisher Scientific, Cat# 15090-046) is diluted with PBS (final concentration, 0.25%), and then added to the SNL cells, which are then left to stand for 1 minute at room temperature. Subsequently, the SNL cells are collected into a tube, centrifuged, suspended in CELLBANKER (R) (manufactured by ZENOAQ Resource, Cat# CB011), and then cryopreserved.

<Maintaining Human iPS Cells>

0.1% gelatin is added to a 10-cm petri dish, which is then left to stand in an incubator under the conditions of 37°C and 5% $CO_2$ for at least 1 hour. The mitomycin-treated SNL cells are suspended with the use of the SNL cell medium. Subsequently, the SNL cells ($1.5\times10^6$ cells) are plated in the 10-cm petri dish, followed by culturing for 2 to 3 days. Then, the SNL cell medium is removed from the petri dish, and the SNL cells are rinsed with PBS. Subsequently, into the petri dish, human iPS cells (strain 201B7, obtained from iPS Academia Japan, AJ-H1-01) suspended in the iPS cell medium containing Y-27632 (manufactured by Tocris, Cat# 1254) in an amount of 1/1000 are plated. The medium is changed every day from two days after the plating, until the start of induced differentiation.

<Induced Differentiation of Human iPS Cells to Motor Neurons>

Y-27632 (final concentration, 10 μM) is added to the cell culture liquid of the human iPS cells, and, in this way, the iPS cells are exposed to the Y-27632 for at least 1 hour. Culture supernatant is removed and the cells are rinsed with PBS, and then Cell dissociation solution (CTK solution) (manufactured by REPROCELL, Cat# RCHETP002) is added thereto for reaction at room temperature for 1 minute. The CTK solution is removed, and the cells are rinsed twice with PBS, followed by addition of 1 mL of the iPS cell medium. The cells are scraped off with the use of a cell scraper, and passed through a cell strainer (manufactured by Becton, Dickinson, Cat# 352350) to disperse the cell clusters to obtain cell suspension. The resulting suspension is transferred to a 6-well plate (manufactured by Corning, Cat# 3471). The medium is changed to the mixed medium A supplemented with LDN193189 (manufactured by Stemgent, Cat# 04-0074) (final concentration, 0.3 μM), SB431542 (manufactured by Tocris, Cat# 1614) (final concentration, 2 μM), CHIR-99021 (manufactured by Stemgent, Cat# 04-0004-10) (final concentration, 3 μM), and Y-27632 (final concentration, 10 μM), and the cells are cultured in an incubator under the conditions of 37°C and 5% $CO_2$ (Culture Day 0).

On Culture Day 2 and 4, the culture liquid is removed with a pipette, and the medium is changed to a fresh medium, which is the mixed medium A supplemented with LDN193189 (final concentration, 0.3 μM), SB431542 (final concentration, 2 μM), and CHIR-99021 (final concentration, 3 μM).

On Culture Day 7, 9, and 11, the culture liquid is removed with a pipette, and the medium is changed to a fresh medium, which is the mixed medium A supplemented with LDN193189 (final concentration, 0.3 μM), SB431542 (final concentration, 2 μM), CHIR-99021 (final concentration, 3 μM), Purmorphamine (manufactured by FUJIFILM Wako Pure Chemical, Cat# 166-23991) (final concentration, 0.5 μM), and Retinoic Acid (manufactured by Sigma-Aldrich, Cat# R2625) (final concentration, 0.1 μM).

On Culture Day 14 and 16, the culture liquid is removed with a pipette, and the medium is changed to a fresh medium, which is the mixed medium A supplemented with Purmorphamine (final concentration, 0.5 μM), Retinoic Acid (final concentration, 0.1 μM), Human BDNF (final concentration, 10 ng/mL), and Ascorbic Acid (manufactured by Sigma-Aldrich, Cat# A5960) (final concentration, 200 μM).

On Culture Day 18, the medium is changed to a fresh medium, which is the mixed medium B supplemented with Purmorphamine (final concentration, 0.5 μM), Retinoic Acid (final concentration, 0.1 μM), and Compound E (manufac-

tured by Calbiochem, Cat# 565790) (final concentration, 0.1 μM).

On Culture Day 21, cell clusters are rinsed with PBS, followed by centrifugation to remove the supernatant. To the cell clusters, Accutase (manufactured by Innovative Cell Technologies, Cat# AT104) and Y27632 (final concentration, 10 μM) are added, followed by incubation at 37°C for 10 minutes. The cell clusters are cooled with ice, and then the cell clusters are dispersed by pipetting. After centrifugation (300×g, 5 minutes, 4°C), a process of collecting the precipitated cells and suspending them in the mixed medium B is repeated twice. Thus, motor neurons derived from iPS cells were obtained. The resulting motor neurons are suspended in CELLBANKER (R), split into portions, and frozen for storage.

<Culturing of Motor Neurons Derived from Human iPS Cells, and Evaluation of Single-Stranded Antisense Nucleotide>

To a 96 Well Optical Btm Plt Polybase Black w/ Lid Cell Culture Sterile PS (manufactured by ThermoFisher Scientific, Cat# 165305), Poly-L-Ornitine Solution (PLO) solution (manufactured by Sigma-Aldrich, Cat# P4957) diluted 6.66 folds with PBS is added, followed by leaving to stand at room temperature for at least 2 hours. After rinsing with PBS three times, iMatrix diluted with PBS is added to the plate in a concentration of 0.5 μg/cm$^2$, followed by leaving to stand overnight at 4°C. Then, the motor neurons derived from human iPS cells previously cryopreserved are thawed, and suspended in the neuron medium. Subsequently, the supernatant is removed by centrifugation, and the cells are resuspended in the neuron medium containing Culture One Supplement (manufactured by ThermoFisher Scientific, A3320201) in an amount of 1/100 and Compound E (final concentration, 0.1 μM). These cells are plated in a coated 96-well plate at 30000 cells/well, and cultured in an incubator under the conditions of 37°C and 5% $CO_2$ for 28 days. Every 2 to 3 days, half the amount of the neuron medium is changed. Until Day 7 after the start of the culturing, as the neuron medium, a medium containing Culture One Supplement and Compound E is used.

After plating, on Culture Day 1, 11, 18, 26 (each of them is expressed as D1, D11, D18, D26), the single-stranded antisense oligonucleotide (in a final concentration of 0.01 μM, 0.1 μM, 1 μM) diluted with PBS is added to the culture medium. For cells of the negative control group, PBS that is free of single-stranded antisense oligonucleotide is added to the culture medium. The cells are cultured in the culture medium under the conditions of 37°C and 5% $CO_2$ for 48 hours or 72 hours, and then the medium containing the single-stranded antisense nucleotide is removed, followed by continued culture in the neuron medium (every 2 to 3 days, half the amount of the medium is changed). Subsequently, the culture medium is removed, and with the use of Taqman Fast Cells-to-CT Kit (manufactured by Thermo Fisher Scientific, Cat# 4399003), extracted total RNA is subjected to reverse transcription reaction. With the use of the resulting complementary DNA (cDNA) obtained from the reverse transcription reaction, Taqman gene expression assays (manufactured by Applied Biosystems), and a gene-specific probe designed in advance (see below), real-time PCR is carried out (40 cycles of 95°C for 3 seconds and 60°C for 30 seconds). "Expression-reducing rate" is calculated by Equation (1) below.

$$\text{(Expression-reducing rate)} = 1-\text{(Expression rate)} \qquad \text{(Equation (1))}$$

It is judged that the greater the value of the expression-reducing rate is, the more likely the single-stranded antisense oligonucleotide is capable of reducing expression of human RPS25 mRNA.

List of Gene-Specific Probes Used in Evaluation of Human RPS25 Gene Expression

Human RPS25: Hs01568661_g1
Human GAPDH: 4326317E (internal control)

<<Evaluation of RPS25 Protein Expression>>

Evaluation of RPS25 protein expression is carried out for respective single-stranded antisense oligonucleotides thus produced, with the use of human fetal kidney cells. The evaluation of the amount of protein expression according to the present example means evaluation of the amount of protein translated from mRNA. In the following, the specific procedure of the expression evaluation will be described.

<Expression Evaluation with Use of Human Fetal Kidney Cells>

Human fetal kidney cells HEK293T (ATCC (registered trademark) CRL-3216 (trademark)) are cultured in a culture medium under the conditions of 37°C and 5% $CO_2$. The culture medium used for HEK293T cells has the

composition as described below.

Composition of Culture Medium for HEK293T Cells

Dulbecco modified Eagle medium (DMEM): manufactured by SIGMA, Cat# D6429
10% fetal bovine serum (FBS): manufactured by biowest, Cat# S1820
100-Fold diluted penicillin-streptomycin mixed solution: manufactured by Nacalai Tesque, Cat# 09367-34 (penicillin 10000 units/ml, streptomycin 10000 μg/ml, a stabilizer contained)

<Quantitative Protein Analysis by Western Blotting>

Firstly, HEK293T cells are plated in a 6-well plate (500000 cells/well), and cultured overnight under the conditions of 37°C and 5% $CO_2$. Subsequently, the cells are transfected by lipofection with the single-stranded antisense oligonucleotide diluted with phosphate-buffered physiological saline (PBS) (final concentration, 50 nM). As the negative control group, cells transfected with PBS that is free of single-stranded antisense oligonucleotide are used. The transfected cells are cultured in a growth medium under the conditions of 37°C and 5% $CO_2$ for 48 hours. Subsequently, the growth medium is removed, followed by rinsing with PBS, and then the cells are collected with the use of a cell scraper. The liquid thus collected is centrifuged under the conditions of 2700×g, 5 minutes, and 4°C to precipitate the cells. After the supernatant is removed, 1 mL of RIPA Lysis and Extraction buffer (manufactured by ThemoFisher Scientific, Cat# 89900) containing Protease Inhibitor (manufactured by ThermoFisher Scientific, Cat# 1860932) in an amount of 1/100 is added, followed by disrupting the cells with the use of an ultrasonic homogenizer. Subsequently, centrifugation is carried out under the conditions of 15000×g, 10 minutes, and 4°C, and the supernatant is to be used as a sample. The sample thus collected is subjected to protein quantification with the use of Pierce (trademark) BCA Protein Assay kit (manufactured by Thermo-Scientific, Cat# 23225). All samples are adjusted to the same concentration, and then Pierce (trademark) Lane Marker Reducing Sample Buffer (manufactured by ThermoFisher Scientifier, Cat# 39000) is added, followed by heat treatment at 95°C for 5 minutes. The sample thus prepared is loaded in an amount of protein of 10 μg to 20 μg/lane, followed by electrophoresis. The electrophoresis is carried out with the use of Criterion (trademark) TDX (trademark) Precast Gel 4-15% (manufactured by BIO-RAD, Cat# 5671085J10) under the conditions of constant voltage of 200 V for 30 minutes. As the electrophoresis buffer, Running Buffer Solution (10×) for SDS-PAGE (manufactured by Nacalai Tesque, Cat# 30329-61) diluted to 1× concentration is used.

After electrophoresis, transfer is carried out in the semi-dry mode. Trans-Blot Turbo Transfer Pack (manufactured by BIO-RAD, Cat# 1704157) is used as the membrane, and BIO-RAD Trans-Blot Turbo Transfer System in the Standard protocol (30 minutes) is used as the transfer apparatus. After the transfer, the membrane is rinsed with TBST. The composition of TBST is as follows: tris buffer physiological saline (pH7.4) (manufactured by Nacalai Tesque, Cat# 35438-81) diluted to 1× concentration containing 0.06% polyoxyethylene sorbitan monolaurate (Tween-20) (manufactured by Nacalai Tesque, Cat# 28353-85). After rinsing, blocking is carried out by shaking in Blocking One (manufactured by Nacalai Tesque, Cat# 03953-95) or PVDF Blocking Reagent (manufactured by Toyobo, Cat# NYPBR01) under the conditions of 1 hour at room temperature. After blocking, rinsing with TBST is carried out, followed by adding a diluted primary antibody and then shaking overnight under the conditions of 4°C. The primary antibody and the solvent for dilution are as follows.

·RPS25

Antibody: Anti-RPS25 antibody (manufactured by Abcam, Cat# ab102940)
Solvent: Canget signal Solution 1 (manufactured by Toyobo, Cat# NKB-101)

·β-actin

Antibody: β-Actin (13E5) Rabbit mAb (HRP conjugate) (manufactured by Cell Signaling, Cat# 5125)
Solvent: Blocking One

After shaking with the primary antibody, rinsing with TBST is carried out, followed by adding a diluted secondary

antibody and then shaking at room temperature for 1 hour. The secondary antibody and the solvent for dilution are as follows.

·RPS25

Antibody: Rabbit IgG (H+L) Cross-Adsorbed Secondary Antibody (manufactured by Invitrogen, Cat# A24537)
Solvent: Canget Signal Solution 2 (manufactured by Toyobo, Cat# NKB-101)

After shaking with the secondary antibody, rinsing with TBST is carried out, followed by detection with the use of ECL prime (manufactured by Amersham, Cat# RPN2232). For the detection and analysis, Amersham Imager 680 is used.
The expression rate of human RPS25 protein for respective single-stranded antisense oligonucleotides is calculated by the above-described method. At this time, the expression rate of human RPS25 protein determined for the negative control group is defined as 1.00. When the expression rate of protein is lower than 0.80, it can be judged that the single-stranded antisense oligonucleotide is capable of modulating the function of RPS25 gene.

<<Evaluation of Cytotoxicity of Single-Stranded Antisense Oligonucleotide>>

Human cervical cancer cells, HeLa-S3 cells, were cultured in a growth medium under the conditions of 37°C and 5% $CO_2$. The growth medium had the composition as described below.

Composition of Growth Medium Used for Cytotoxicity Evaluation

10% fetal bovine serum (FBS): manufactured by GIBCO, CAT# 10437028
1% non-essential amino acid (NEAA): manufactured by GIBCO, Cat# 11140050
Dulbecco's Modified Eagle Medium Low Glucose (containing L-glutamine, phenol red) (manufactured by FUJIFILM Wako Pure Chemical, Cat# 041-29775)

The day before the experiment, the cells were plated in a 96-well plate ($1.0\times10^4$ cells/well). The plated cells were cultured overnight under the conditions of 37°C and 5% $CO_2$, and then, to Opti-Minimum Essential Medium (manufactured by Thermo Fisher Scientific, Cat# 31985070), the single-stranded antisense oligonucleotide (final concentration, 1 to 200 nM) in the form of a complex with Lipofectamine 3000 (manufactured by Thermo Fisher Scientific, Cat# L3000-015) was added, followed by culturing the cells under the conditions of 37°C and 5% $CO_2$ for 24 hours. Subsequently, to the growth medium, Caspase-Glo 3/7 Assay System (manufactured by Promega, Cat# G8093) or CellTiter-Blue Cell Viability Assay (manufactured by Promega, Cat# G8081) was added to evaluate caspase activity and cell viability. Evaluation results of cytotoxicity (cell viability) of the single-stranded antisense oligonucleotide determined by the above-described method are shown in Table 7-1 to Table 7-3.

[Table 7-1]

| Ex. | SEQ ID NO: | Sequence name | Cell viability | | | |
|---|---|---|---|---|---|---|
| | | | 24 hours later | | 72 hours later | |
| | | | @30 nM | @100 nM | @30 nM | @100 nM |
| 1 | 5 | h451-465-AQ | 0.88 | 0.90 | 0.66 | 0.59 |
| 4 | 8 | h451-465-AT | 1.00 | 0.96 | 1.10 | 1.03 |
| 5 | 9 | h451-465-AU | 0.99 | 0.99 | 0.74 | 0.85 |
| 6 | 10 | h451-465-AW | 1.03 | 1.04 | 1.04 | 0.98 |
| 7 | 11 | h451-465-AX | 1.04 | 1.03 | 1.07 | 0.97 |
| 8 | 12 | h451-465-AY | 0.97 | 0.91 | 1.10 | 1.01 |
| 9 | 13 | h451-465-AZ | 0.95 | 0.88 | 1.30 | 1.02 |
| 10 | 14 | h451-465-BA | 0.93 | 0.90 | 1.08 | 0.97 |

(continued)

| Ex. | SEQ ID NO: | Sequence name | Cell viability | | | |
|---|---|---|---|---|---|---|
| | | | 24 hours later | | 72 hours later | |
| | | | @30 nM | @100 nM | @30 nM | @100 nM |
| 11 | 15 | h451-465-BB | 1.00 | 0.95 | 1.06 | 1.11 |
| 12 | 16 | h451-465-BC | 0.91 | 0.83 | 1.10 | 1.02 |
| 13 | 17 | h451-465-BD | 0.98 | 0.99 | 1.00 | 0.98 |
| 14 | 18 | h451-465-BE | 0.91 | 0.90 | 0.95 | 0.84 |
| 15 | 19 | h451-465-BF | 0.84 | 0.81 | 1.02 | 0.91 |
| 16 | 20 | h451-465-BG | 0.90 | 0.87 | 0.82 | 0.77 |
| 17 | 21 | h451-465-BH | 0.89 | 0.89 | 0.76 | 0.76 |
| 18 | 22 | h451-465-BI | 0.98 | 0.96 | 1.10 | 1.06 |
| 19 | 23 | h451-465-BJ | 0.93 | 0.89 | 1.31 | 0.99 |
| 20 | 24 | h451-465-BK | 1.00 | 0.98 | 1.21 | 1.12 |
| 21 | 25 | h451-465-BL | 1.02 | 1.00 | 1.29 | 1.08 |
| 22 | 26 | h451-465-BM | 0.97 | 0.96 | 1.30 | 1.12 |

[Table 7-2]

| Ex. | SEQ ID NO: | Sequence name | Cell viability | | | |
|---|---|---|---|---|---|---|
| | | | 24 hours later | | 72 hours later | |
| | | | @30 nM | @100 nM | @30 nM | @100 nM |
| 23 | 27 | h451-465-BN | 0.96 | 0.89 | 1.15 | 1.16 |
| 24 | 28 | h451-465-BO | 0.84 | 0.84 | 1.05 | 0.96 |
| 25 | 29 | h451-465-BP | 0.74 | 0.67 | 1.06 | 0.98 |
| 26 | 30 | h451-465-BQ | 0.91 | 0.87 | 0.99 | 0.81 |
| 27 | 31 | h451-465-BR | 1.02 | 0.98 | 1.16 | 1.15 |
| 28 | 32 | h451-465-BS | 0.98 | 0.89 | 1.20 | 1.12 |
| 29 | 33 | h451-465-BT | 0.99 | 0.99 | 1.16 | 1.14 |
| 30 | 34 | h451-465-BU | 0.95 | 0.92 | 1.13 | 1.10 |
| 31 | 35 | h450-465-E | 1.04 | 0.97 | 1.13 | 1.04 |
| 32 | 36 | h450-465-F | 0.98 | 0.98 | 1.09 | 1.10 |
| 33 | 37 | h450-465-G | 0.91 | 0.94 | 1.12 | 0.57 |
| 34 | 38 | h450-466-C | 0.96 | 0.86 | 0.74 | 0.27 |
| 35 | 39 | h451-467-G | 0.86 | 0.97 | 1.06 | 0.33 |
| 36 | 40 | h450-466-D | 0.99 | 0.88 | 1.11 | 0.47 |
| 37 | 41 | h450-466-E | 0.97 | 0.94 | 1.14 | 0.52 |
| 38 | 42 | h451-467-H | 0.96 | 0.84 | 1.09 | 0.62 |
| 39 | 43 | h451-467-I | 1.02 | 1.01 | 1.20 | 0.84 |
| 40 | 44 | h450-467-D | 0.96 | 0.91 | 1.19 | 0.22 |
| 41 | 45 | h450-467-E | 0.91 | 0.91 | 1.07 | 0.27 |
| 42 | 46 | h450-467-F | 0.93 | 0.87 | 1.12 | 0.29 |

[Table 7-3]

| Ex. | SEQ ID NO: | Sequence name | Cell viability | | | |
|---|---|---|---|---|---|---|
| | | | 24 hours later | | 72 hours later | |
| | | | @30 nM | @100 nM | @30 nM | @100 nM |
| 43 | 47 | h451-469-E | 0.96 | 0.91 | 0.93 | 0.95 |
| 44 | 48 | h451-469-F | 0.98 | 1.03 | 1.07 | 1.06 |
| 45 | 49 | h451-469-G | 0.99 | 1.00 | 0.47 | 0.33 |
| 46 | 50 | h451-469-H | 0.99 | 0.90 | 0.35 | 0.20 |
| 47 | 51 | h451-469-I | 0.99 | 0.97 | 0.54 | 0.37 |
| 48 | 52 | h451-469-J | 1.00 | 1.00 | 0.91 | 0.75 |
| 49 | 53 | h451-469-K | 0.95 | 0.94 | 0.43 | 0.31 |
| 50 | 54 | h453-472-B | 0.72 | 0.69 | 0.11 | 0.10 |
| 51 | 55 | h453-472-D | 0.89 | 0.83 | 0.25 | 0.14 |
| 52 | 56 | h453-472-E | 0.98 | 0.91 | 0.56 | 0.53 |

<<Evaluation of Serum Stability of Single-Stranded Antisense Oligonucleotide>>

To Tris-EDTA buffer (pH=8.0) solution (4 μL) containing 400 pmol of the single-stranded antisense oligonucleotide, mouse serum (20 μL) or human serum (20 μL) was mixed, followed by addition of mineral oil (15 μL). The resulting solution was incubated at 37°C, and, then, an 8-mol/L urea solution (10 μL) was mixed thereto for inactivating nuclease in the serum. After ultrapure water (10 μL) was added, centrifugation was carried out to separate the resultant into an aqueous layer containing the single-stranded antisense oligonucleotide and a mineral oil layer, and the aqueous layer was subjected to analysis by LC-MS (manufactured by Waters), where, from the integrated intensity of the UV-chromatogram for the obtained single-stranded antisense oligonucleotide, the residual single-stranded antisense oligonucleotide was calculated. "Residual oligonucleotide (%)" refers to the rate, relative to the non-degraded single-stranded antisense oligonucleotide at the time of analysis which was carried out immediately after mixing with the serum, of the residual non-degraded single-stranded antisense oligonucleotide remaining 72 hours later.

A single-stranded antisense oligonucleotide with a residual rate of 50% or more after 72 hours is judged to be stable.

<<Evaluation of In Vivo RPS25 Gene Expression>>

Evaluation of RPS25 gene expression was carried out by performing intraventricular administration for mice and measuring the amount of mRNA in different parts of the prefrontal cortex. Evaluation of gene expression according to the present example means measuring the amount of complementary DNA (cDNA) obtained from reverse transcription reaction to assess the amount of mRNA. In the following, the specific procedure of the expression evaluation will be described.

FVB mice (CLEA Japan) were anesthetized with isoflurane (manufactured by Pfizer, Cat# 114133403). Then, the FVB mice under anesthesia were administered with the antisense oligonucleotide dissolved in artificial cerebrospinal fluid (manufactured by Tocris Bioscience, Cat# 3525 /25 mL) (10 μL/individual) with the use of a double needle (manufactured by TOP, medical instrument approval number 15800BZZ01460000) inserted in a 50-μL Hamilton syringe (manufactured by Hamilton, Cat# 705LT). To the mice in the negative control group, artificial cerebrospinal fluid alone was administered (10 μL/individual).

After a certain period of time following the administration, the FVB mice were euthanized and three parts, namely the brain, the cervical cord, and the lumbar cord, were sampled. The sampled tissue was immersed in RNA later (manufactured by Applied Biosystems, Cat# AM7024) and left to stand overnight, followed by stored at - 80°C. From the tissue sample thus stored, RNA was extracted with the use of RNeasy Mini Kit (manufactured by QIAGEN, Cat# 74106). The mRNA thus extracted was subjected to reverse transcription reaction with the use of High-Capacity cDNA Reverse Transcription Kit (manufactured by Applied Biosystem, Cat# 4368814). For the reverse transcription reaction, 1 μg of mRNA diluted to 20 μL was used. With the use of the complementary DNA (cDNA) obtained from the reverse transcription reaction, Taqman expression assays (manufactured by Applied Biosystems), and a gene-specific probe designed in advance (see below), real-time PCR was carried out (40 cycles of 95°C for 3 seconds and 60°C for 30 seconds).

List of Gene-Specific Probes Used in Evaluation of Mouse RPS25 Gene Expression

Mouse Rps25: Mm02342783_g1
Mouse GAPDH: 4352339E (internal control)

The expression rate of mouse RPS25 mRNA for respective single-stranded antisense oligonucleotides determined by the above-described method is shown in Table 8-1 to Table 8-2. At this time, the expression rate of mouse RPS25 RNA determined for the negative control group was defined as 1.00. Generally, it is expected that when mRNA expression is reduced, subsequent protein translation and the like are also reduced; hence, it can be judged that a single-stranded antisense oligonucleotide with an expression rate of 0.80 or less is capable of modulating the function of mouse RPS25 gene. It should be noted that the target region to which each of the single-stranded antisense oligonucleotides listed in Table 8-1 to Table 8-2 binds is a region whose sequence is conserved between human RPS25 gene and mouse RPS25 gene.

[Table 8-1]

| Ex. | SEQ ID NO: | Sequence name | In vivo evaluation (prefrontal cortex) | | |
|---|---|---|---|---|---|
| | | | Dose | Post-administration days | mRPS25 expression rate |
| 2 | 6 | h451-465-AR | 20 nmol | 28 | 0.93 |
| 5 | 9 | h451-465-AU | 20 nmol | 28 | 0.64 |
| 14 | 18 | h451-465-BE | 20 nmol | 28 | 0.56 |
| 34 | 38 | h450-466-C | 20 nmol | 28 | 0.38 |
| 37 | 41 | h450-466-E | 20 nmol | 28 | 0.39 |
| 43 | 47 | h451-469-E | 20 nmol | 28 | 0.46 |
| 44 | 48 | h451-469-F | 60 nmol | 28 | 0.78 |
| 45 | 49 | h451-469-G | 20 nmol | 28 | 0.33 |
| 46 | 50 | h451-469-H | 20 nmol | 28 | 0.15 |
| 47 | 51 | h451-469-I | 20 nmol | 28 | 0.45 |
| 48 | 52 | h451-469-J | 20 nmol | 28 | 0.55 |
| 49 | 53 | h451-469-K | 20 nmol | 28 | 0.39 |
| 50 | 54 | h453-472-B | 20 nmol | 28 | 0.49 |
| 51 | 55 | h453-472-D | 60 nmol | 28 | 0.44 |
| 52 | 56 | h453-472-E | 60 nmol | 28 | 0.77 |

[Table 8-2]

| Ref. Ex. | SEQ ID NO: | Sequence name | In vivo evaluation (prefrontal cortex) | | |
|---|---|---|---|---|---|
| | | | Dose | Post-administration days | mRPS25 expression rate |
| 1 | 57 | h451-465-C | 20 nmol | 27 | 0.47 |
| 2 | 58 | h451-465-N | 20 nmol | 27 | 0.50 |
| 3 | 59 | h451-465-O | 20 nmol | 27 | 0.40 |
| 6 | 62 | h451-465-AB | 20 nmol | 28 | 0.79 |
| 8 | 64 | h451-465-AL | 20 nmol | 28 | 0.63 |
| 28 | 84 | h451-469-A | 60 nmol | 27 | 0.43 |
| 29 | 85 | h451-469-D | 20 nmol | 28 | 0.13 |
| 30 | 86 | h453-472-A | 20 nmol | 28 | 0.28 |

(continued)

| Ref. Ex. | SEQ ID NO: | Sequence name | In vivo evaluation (prefrontal cortex) | | |
|---|---|---|---|---|---|
| | | | Dose | Post-administration days | mRPS25 expression rate |
| 31 | 87 | h453-472-C | 60 nmol | 28 | 0.51 |

<<In Vivo Evaluation of Central Toxicity of Single-Stranded Antisense Oligonucleotide>>

Evaluation of central toxicity of the single-stranded antisense oligonucleotide was carried out by performing intraventricular administration and then rearing the mice for a time period ranging from 1 hour to 28 days after administration, followed by checking the performance status and the body weight and performing pathological examination (hematoxylin-eosin staining) at the time of tissue sampling.

Results are given in Table 9-1 to Table 9-2. In Table 9-1 to Table 9-2, the values in the section "score" under "in vivo acute neuroTox" were calculated based on the evaluation criteria below. For each individual, among all the observed signs, the highest point was recorded for each of Items 1 to 5 and all the recorded points were summed. For example, when ptosis, lying on side, decreased respiration, and convulsion were observed in one individual, the total point is 1+4+4=9. The higher the point is, the more serious the signs are, and therefore it can be judged that the higher the total point is, the more serious the acute central toxicity sign is. The score is the average value of all the groups. "-" in the tables means that no measurement was performed.

1. Hyperactivity (1 point), stereotyped behavior (grooming, circling) (1 point), rearing behavior (1 point), vocalization (1 point), hypersensitivity/Straub tail (1 point)
2. Ptosis, eyelid closure, decrease (loss) of locomotor activity (1 point)
3. Staggering (1 point), ataxic gait (2 points)
4. Abnormal position/irregular respiration (1 point), lying on side/lying on belly (2 points), decreased/abnormal respiration (4 points)
5. Jumping, tremor, twitch (2 points), convulsion (4 points)

The values in the section "score" under "in vivo delayed neuroTox" were calculated based on the evaluation criteria below. The score is the average value of all the groups. "ICR" means Crl:CD1 mice, and "FVB" means FVB/NJcl mice.

Clinical sign score;

0 points: No abnormalities observed
1 point: Abnormal hindlimb function, tremor, decrease in locomotor activity
2 points: Dragging of hindlimbs, weakness in tail and hindlimbs
3 points: Complete loss of hindlimb function, hindlimb paralysis, lying on side, lying on belly
4 points: Euthanized

Pathological score;

0 points: No abnormalities observed
1 point: Abnormalities observed (such as single cell necrosis and vacuolization)

As for Example 51 in Table 9-1 and Reference Example 31 in Table 9-2, central toxicity evaluation was carried out by the method described below.

<<In Vivo Evaluation of Central Toxicity of Single-Stranded Antisense Oligonucleotide (2)>>

Evaluation of acute central toxicity (in vivo acute neuroTox) of the single-stranded antisense oligonucleotides of Example 51 in Table 9-1 and Reference Example 31 in Table 9-2 was carried out by performing intraventricular administration to ICR mice, followed by assessing 1 day after administration. Evaluation was performed based on

behavior rating, and the behavior rating of the mice was performed by Functional observational battery (FOB). Evaluation items were 1) position, 2) abnormality in appearance, 3) stereotyped behavior, 4) response to stimulation, 5) grip, 6) respiration, and 7) shivering/convulsion, and for each item, normal was given a score of 0, slightly abnormal was given a score of 1, and extremely abnormal was given a score of 2. For each item, the scores were summed and entered in the section "score" under "in vivo acute neuroTox" in Table 9-1 and Table 9-2.

<<In Vivo Evaluation of Central Toxicity of Single-Stranded Antisense Oligonucleotide (3)>>

Evaluation of delayed central toxicity (Clinical Sign Score of in vivo delayed neuroTox) of the single-stranded antisense oligonucleotides of Example 51 in Table 9-1 and Reference Example 31 in Table 9-2 was carried out by performing intraventricular administration to ICR mice, followed by assessing for 28 days after administration. Evaluation was performed based on behavior rating, and behavior rating of the mice was performed by Functional observational battery (FOB). Evaluation items were 1) position, 2) abnormality in appearance, 3) stereotyped behavior, 4) response to stimulation, 5) grip, 6) respiration, and 7) shivering/convulsion, and for each item, normal was given a score of 0, slightly abnormal was given a score of 1, and extremely abnormal was given a score of 2. For each item, the scores were summed and entered in the section "Clinical Sign Score" under "in vivo delayed neuroTox" in Table 9-1 and Table 9-2.

[Table 9-1]

| Ex. | SEQ ID NO: | Sequence name | in vivo acute neuroTox | | in vivo delayed neuroTox | | | | |
|---|---|---|---|---|---|---|---|---|---|
| | | | Dose | score | Dose | Clinical Sign score | Observation date | Pathological score | Sampling date |
| 2 | 6 | h451-465-AR | 20 nmol | 1.00 | 20 nmol | 0.00 | d28 | - | - |
| 5 | 9 | h451-465-AU | 20 nmol | 1.67 | 20 nmol | 0.00 | d28 | - | - |
| 14 | 18 | h451-465-BE | 20 nmol | 3.00 | 20 nmol | 0.00 | d28 | - | - |
| 34 | 38 | h450-466-C | 20 nmol | 4.33 | 20 nmol | 0.00 | d28 | 0.33 | d28 |
| 37 | 41 | h450-466-E | 20 nmol | 3.00 | 20 nmol | 0.00 | d28 | 0.00 | d28 |
| 43 | 47 | h451-469-E | 20 nmol | 0.00 | 20 nmol | 0.00/0.00 | d14/d28 | 0.00 | d28 |
| 44 | 48 | h451-469-F | 60 nmol | 2.33 | 60 nmol | 0.00/0.00 | d14/d28 | - | - |
| 45 | 49 | h451-469-G | 20 nmol | 2.00 | 20 nmol | 0.00/0.00 | d14/d28 | - | - |
| 46 | 50 | h451-469-H | 20 nmol | 4.00 | 20 nmol | 0.00/0.00 | d14/d28 | 0.00 | d28 |
| 47 | 51 | h451-469-1 | 20 nmol | 0.00 | 20 nmol | 0.00/0.00 | d14/d28 | - | - |
| 48 | 52 | h451-469-J | 20 nmol | 0.00 | 20 nmol | 0.00/0.00 | d14/d28 | - | - |
| 49 | 53 | h451-469-K | 20 nmol | 0.00 | 20 nmol | 0.00/0.00 | d14/d28 | - | - |
| 50 | 54 | h453-472-B | 20 nmol | 1.67 | 20 nmol | 0.00/0.00 | d14/d28 | 0.00 | d28 |
| 51 | 55 | h453-472-D | 60 nmol | 0.50 | 60 nmol | 0.00 | d28 | 0.00 | d28 |

[Table 9-2]

| Ref. Ex. | SEQ ID NO: | Sequence name | in vivo acute neuroTox | | in vivo delayed neuroTox | | | | |
|---|---|---|---|---|---|---|---|---|---|
| | | | Dose | score | Dose | Clinical Sign score | Observation date | Pathological score | Sampling date |
| 1 | 57 | h451-465-C | 20 nmol | 3.25 | 100 µg (20 nmol) | 1.60 | d28 | 1.00 | d43 |
| 2 | 58 | h451-465-N | 20 nmol | 3.00 | 20 nmol | 0.00 | d27 | 0.67 | d27 |
| 3 | 59 | h451-465-O | 20 nmol | 4.00 | 20 nmol | 0.00 | d27 | 0.33 | d27 |
| 6 | 62 | h451-465-AB | 20 nmol | 2.17 | 20 nmol | 0.00 | d27 | 0.67 | d27 |
| 8 | 64 | h451-465-AL | 20 nmol | 2.00 | 20 nmol | 0.00 | d28 | 0.33 | d28 |
| 11 | 67 | h325-341-B | 20 nmol | 2.17 | 150 µg (26 nmol) | 0.00 | d27 | 0.33 | d27 |
| 26 | 82 | h450-466-B | 150 µg (26 nmol) | 6.00 | 150 µg (26 nmol) | 3.00 | d27 | 0.33 | d21, d30, d42 (one for each date) |
| 28 | 84 | h451-469-A | 60 nmol | 3.00 | 60 nmol | 0.00 | d29 | 0.00 | d29 |
| 29 | 85 | h451-469-D | 20 nmol | 3.33 | 20 nmol | 0.00/0.00 | d14/d28 | 1.00 | d28 |
| 30 | 86 | h453-472-A | 20 nmol | 3.33 | 20 nmol | 0.33/2.67 | d14/d28 | 0.00 | d28 |
| 31 | 87 | h453-472-C | 60 nmol | 0.30 | 60 nmol | 0.00 | d28 | - | - |

<<In Silico Evaluation of Narrow-Sense Off-Target Toxicity>>

Evaluation of narrow-sense off-target toxicity of the single-stranded antisense oligonucleotide was carried out by using the above-mentioned Ultrafast Sequence Search system (GGGenome). Sequence information of each single-stranded antisense oligonucleotide was entered into GGGenome, and based on the number of differences in terms of hybridization with human spliced RNAs and human pre-spliced RNAs registered in the database, the number of narrow-sense off-target RNAs was counted. When there were mismatch, deletion, and insertion in terms of hybridization, the number of those bases was summed, which was regarded as the number of differences. For example, in the case where the base sequence of the target RNA expected from the base sequence of the single-stranded antisense oligonucleotide is "AGCTGTAC", an RNA having a base sequence "ATCTGTAC" has "1" mismatch (the underlined base) and therefore the number of differences is calculated as "1". In the case where the base sequence of the target RNA is "AGCTGTAC", an RNA having a base sequence "ATCTG<G>TAC" has "1" mismatch (the underlined base) and "1" deletion (the base surrounded by angle brackets), and therefore the number of differences is calculated as "2". In the case where the base sequence of the target RNA is "AGCTGTAC", an RNA having a base sequence "ATCTG*AC" has "1" mismatch (the underlined base) and "1" insertion (the position expressed as *), and therefore the number of differences is calculated as "2". In this way, the number of differences in terms of hybridization with non-target RNA (off-target RNA) is counted, and for each single-stranded antisense oligonucleotide, the number of full match RNAs, the number of RNAs with 1 or less differences, and the number of RNAs with 2 or less differences are calculated. The smaller the number of RNAs with 0 differences, 1 or less differences, and 2 or less differences , the lower the risk of narrow-sense off-target toxicity; on the

contrary, the greater the number of RNAs with 0 differences, 1 or less differences, and 2 or less differences , the higher the risk of narrow-sense off-target toxicity. Results are given in Table 10-1 to Table 10-4. For those with a base length of 18 or more, the number of RNAs with 1 or less differences in terms of hybridization is 10 or less and therefore the narrow-sense off-target risk is considered low; hence, they can be regarded as more preferable single-stranded antisense oligonucleotides.

[Table 10-1]

| Ex. | SEQ ID NO: | Sequence name | Base length of antisense oligonucleotide | Off-target gene count | | |
|---|---|---|---|---|---|---|
| | | | | full match | ≤1 | ≤2 |
| 1 | 5 | h451-465-AQ | 15 | 0 | 237 | 4433 |
| 2 | 6 | h451-465-AR | 15 | 0 | 237 | 4433 |
| 3 | 7 | h451-465-AS | 15 | 0 | 237 | 4433 |
| 4 | 8 | h451-465-AT | 15 | 0 | 237 | 4433 |
| 5 | 9 | h451-465-AU | 15 | 0 | 237 | 4433 |
| 6 | 10 | h451-465-AW | 15 | 0 | 237 | 4433 |
| 7 | 11 | h451-465-AX | 15 | 0 | 237 | 4433 |
| 8 | 12 | h451-465-AY | 15 | 0 | 237 | 4433 |
| 9 | 13 | h451-465-AZ | 15 | 0 | 237 | 4433 |
| 10 | 14 | h451-465-BA | 15 | 0 | 237 | 4433 |
| 11 | 15 | h451-465-BB | 15 | 0 | 237 | 4433 |
| 12 | 16 | h451-465-BC | 15 | 0 | 237 | 4433 |
| 13 | 17 | h451-465-BD | 15 | 0 | 237 | 4433 |
| 14 | 18 | h451-465-BE | 15 | 0 | 237 | 4433 |
| 15 | 19 | h451-465-BF | 15 | 0 | 237 | 4433 |
| 16 | 20 | h451-465-BG | 15 | 0 | 237 | 4433 |
| 17 | 21 | h451-465-BH | 15 | 0 | 237 | 4433 |
| 18 | 22 | h451-465-BI | 15 | 0 | 237 | 4433 |
| 19 | 23 | h451-465-BJ | 15 | 0 | 237 | 4433 |
| 20 | 24 | h451-465-BK | 15 | 0 | 237 | 4433 |
| 21 | 25 | h451-465-BL | 15 | 0 | 237 | 4433 |
| 22 | 26 | h451-465-BM | 15 | 0 | 237 | 4433 |
| 23 | 27 | h451-465-BN | 15 | 0 | 237 | 4433 |
| 24 | 28 | h451-465-BO | 15 | 0 | 237 | 4433 |
| 25 | 29 | h451-465-BP | 15 | 0 | 237 | 4433 |
| 26 | 30 | h451-465-BQ | 15 | 0 | 237 | 4433 |

[Table 10-2]

| Ex. | SEQ ID NO: | Sequence name | Base length of antisense oligonucleotide | Off-target gene count | | |
|---|---|---|---|---|---|---|
| | | | | full match | ≤1 | ≤2 |
| 27 | 31 | h451-465-BR | 15 | 0 | 237 | 4433 |
| 28 | 32 | h451-465-BS | 15 | 0 | 237 | 4433 |
| 29 | 33 | h451-465-BT | 15 | 0 | 237 | 4433 |
| 30 | 34 | h451-465-BU | 15 | 0 | 237 | 4433 |

(continued)

| Ex. | SEQ ID NO: | Sequence name | Base length of antisense oligonucleotide | Off-target gene count | | |
|---|---|---|---|---|---|---|
| | | | | full match | ≤1 | ≤2 |
| 31 | 35 | h450-465-E | 16 | 0 | 88 | 2151 |
| 32 | 36 | h450-465-F | 16 | 0 | 88 | 2151 |
| 33 | 37 | h450-465-G | 16 | 0 | 88 | 2151 |
| 34 | 38 | h450-466-C | 17 | 0 | 25 | 966 |
| 35 | 39 | h451-467-G | 17 | 0 | 24 | 934 |
| 36 | 40 | h450-466-D | 17 | 0 | 25 | 966 |
| 37 | 41 | h450-466-E | 17 | 0 | 25 | 966 |
| 38 | 42 | h451-467-H | 17 | 0 | 24 | 934 |
| 39 | 43 | h451-467-I | 17 | 0 | 24 | 934 |
| 40 | 44 | h450-467-D | 18 | 0 | 9 | 384 |
| 41 | 45 | h450-467-E | 18 | 0 | 9 | 384 |
| 42 | 46 | h450-467-F | 18 | 0 | 9 | 384 |
| 43 | 47 | h451-469-E | 19 | 0 | 3 | 106 |
| 44 | 48 | h451-469-F | 19 | 0 | 5 | 114 |
| 45 | 49 | h451-469-G | 19 | 0 | 5 | 114 |
| 46 | 50 | h451-469-H | 19 | 0 | 5 | 114 |
| 47 | 51 | h451-469-I | 19 | 0 | 5 | 114 |
| 48 | 52 | h451-469-1 | 19 | 0 | 5 | 114 |
| 49 | 53 | h451-469-K | 19 | 0 | 3 | 106 |
| 50 | 54 | h453-472-B | 20 | 1 | 2 | 161 |
| 51 | 55 | h453-472-D | 20 | 1 | 2 | 161 |
| 52 | 56 | h453-472-E | 20 | 1 | 2 | 161 |

[Table 10-3]

| Ref. Ex. | SEQ ID NO: | Sequence name | Base length of antisense oligonucleotide | Off-target gene count | | |
|---|---|---|---|---|---|---|
| | | | | full match | ≤1 | ≤2 |
| 1 | 57 | h451-465-C | 15 | 0 | 237 | 4433 |
| 2 | 58 | h451-465-N | 15 | 0 | 237 | 4433 |
| 3 | 59 | h451-465-O | 15 | 0 | 237 | 4433 |
| 4 | 60 | h451-465-Q | 15 | 0 | 237 | 4433 |
| 5 | 61 | h451-465-R | 15 | 0 | 237 | 4433 |
| 6 | 62 | h451-465-AB | 15 | 0 | 237 | 4433 |
| 7 | 63 | h451-465-AP | 15 | 0 | 237 | 4433 |
| 8 | 64 | h451-465-AL | 15 | 0 | 237 | 4433 |
| 9 | 65 | h451-465-AM | 15 | 0 | 237 | 4433 |
| 10 | 66 | h450-465-C | 16 | 0 | 88 | 2151 |
| 11 | 67 | h325-341-B | 17 | 1 | 8 | 433 |
| 12 | 68 | h325-341-L | 17 | 1 | 8 | 433 |

(continued)

| Ref. Ex. | SEQ ID NO: | Sequence name | Base length of antisense oligonucleotide | Off-target gene count | | |
|---|---|---|---|---|---|---|
| | | | | full match | ≤1 | ≤2 |
| 13 | 69 | h325-341-M | 17 | 1 | 8 | 433 |
| 14 | 70 | h325-341-N | 17 | 1 | 8 | 433 |
| 15 | 71 | h325-341-O | 17 | 1 | 8 | 433 |
| 16 | 72 | h325-341-P | 17 | 1 | 8 | 433 |
| 17 | 73 | h325-341-Q | 17 | 1 | 8 | 433 |
| 18 | 74 | h325-341-R | 17 | 1 | 8 | 433 |
| 19 | 75 | h325-341-S | 17 | 1 | 8 | 433 |
| 20 | 76 | h325-341-T | 17 | 1 | 8 | 433 |

[Table 10-4]

| Ref. Ex. | SEQ ID NO: | Sequence name | Base length of antisense oligonucleotide | Off-target gene count | | |
|---|---|---|---|---|---|---|
| | | | | full match | ≤1 | ≤2 |
| 21 | 77 | h325-341-U | 17 | 1 | 8 | 433 |
| 22 | 78 | h325-341-V | 17 | 1 | 8 | 433 |
| 23 | 79 | h325-341-I | 17 | 1 | 8 | 433 |
| 24 | 80 | h325-341-J | 17 | 1 | 8 | 433 |
| 25 | 81 | h325-341-K | 17 | 1 | 8 | 433 |
| 26 | 82 | h450-466-B | 17 | 0 | 25 | 966 |
| 27 | 83 | h450-467-C | 18 | 0 | 9 | 384 |
| 28 | 84 | h451-469-A | 19 | 0 | 5 | 114 |
| 29 | 85 | h451-469-D | 19 | 0 | 5 | 114 |
| 30 | 86 | h453-472-A | 20 | 1 | 2 | 161 |
| 31 | 87 | h453-472-C | 20 | 1 | 2 | 161 |

The embodiment and Examples of the present invention are described above, and the configurations of the above embodiment and Examples may be combined as appropriate.

The embodiment and Examples disclosed herein are illustrative and non-restrictive in any respect. The scope of the present invention is defined by the terms of the claims, not by the embodiment and Examples, and intended to encompass all modifications and variations equivalent in meaning and scope to the claims.

## Claims

**1.** A single-stranded antisense oligonucleotide, or a pharmaceutically acceptable salt thereof, capable of modulating expression and/or function of RPS25 gene, wherein

nucleosides of the single-stranded antisense oligonucleotide are bonded to each other via a phosphate group and/or a modified phosphate group,
the single-stranded antisense oligonucleotide includes a gap region, a 3' wing region bonded to a 3' end of the gap region, and a 5' wing region bonded to a 5' end of the gap region,
the gap region is a deoxyribose-based nucleic acid , which may contain a nucleic acid having a modified sugar moiety,
the gap region includes at least one 5'-CP nucleic acid,
each of the 3' wing region and the 5' wing region is a modified nucleic acid having a substituent at 2' position,

the single-stranded antisense oligonucleotide is composed of 12 to 30 bases, and
a base sequence of the single-stranded antisense oligonucleotide is:

a base sequence with a sequence identity of 90% to 100% to a base sequence complementary to at least one target region having the same base length as the single-stranded antisense oligonucleotide present in a base sequence as set forth in SEQ ID NO: 1;
a base sequence complementary to a base sequence of the target region with deletion, substitution, insertion, or addition of one or several bases; or
a base sequence capable of hybridizing under stringent conditions with an oligonucleotide having the target region.

**2.** A single-stranded antisense oligonucleotide, or a pharmaceutically acceptable salt thereof, capable of modulating expression and/or function of RPS25 gene, wherein

nucleosides of the single-stranded antisense oligonucleotide are bonded to each other via a phosphate group and/or a modified phosphate group,
the single-stranded antisense oligonucleotide includes a gap region, a 3' wing region bonded to a 3' end of the gap region, and a 5' wing region bonded to a 5' end of the gap region,
the gap region is a deoxyribose-based nucleic acid , which may contain a nucleic acid having a modified sugar moiety,
the gap region includes at least one 5'-CP nucleic acid,
each of the 3' wing region and the 5' wing region is a modified nucleic acid having a substituent at position 2',
the single-stranded antisense oligonucleotide is composed of 12 to 30 bases, and
a base sequence of the single-stranded antisense oligonucleotide is:

a base sequence with a sequence identity of 90% to 100% to a base sequence complementary to at least one target region having the same base length as the single-stranded antisense oligonucleotide present in a base sequence as set forth in SEQ ID NO: 1;
a base sequence complementary to a base sequence of the target region with deletion, substitution, insertion, or addition of one or several bases; or
a base sequence capable of hybridizing under stringent conditions with an oligonucleotide having the target region

(except for single-stranded antisense oligonucleotides of Reference Examples 1 to 31).

**3.** A single-stranded antisense oligonucleotide, or a pharmaceutically acceptable salt thereof, capable of modulating expression and/or function of RPS25 gene, wherein

nucleosides of the single-stranded antisense oligonucleotide are bonded to each other via a phosphate group and/or a modified phosphate group,
the single-stranded antisense oligonucleotide includes a gap region, a 3' wing region bonded to a 3' end of the gap region, and a 5' wing region bonded to a 5' end of the gap region,
the gap region is a deoxyribose-based nucleic acid , which may contain a nucleic acid having a modified sugar moiety,
the gap region includes at least one 5'-CP nucleic acid,
each of the 3' wing region and the 5' wing region is a modified nucleic acid having a substituent at 2' position,
the single-stranded antisense oligonucleotide is composed of 12 to 30 bases, and
a base sequence of the single-stranded antisense oligonucleotide is:

a base sequence with a sequence identity of 90% to 100% to a base sequence complementary to at least one target region having the same base length as the single-stranded antisense oligonucleotide present in a base sequence as set forth in SEQ ID NO: 1;
a base sequence complementary to a base sequence of the target region with deletion, substitution, insertion, or addition of one or several bases; or
a base sequence capable of hybridizing under stringent conditions with an oligonucleotide having the target region

(except for single-stranded antisense oligonucleotides of Reference Examples 1 to 31 and Design Examples 1 to

18).

**4.** The single-stranded antisense oligonucleotide or a pharmaceutically acceptable salt thereof according to any one of claims 1 to 3, wherein the base sequence of the single-stranded antisense oligonucleotide is:
a base sequence with a sequence identity of 95% to 100% to a base sequence complementary to at least one target region having the same base length as the single-stranded antisense oligonucleotide present in the base sequence as set forth in SEQ ID NO: 1.

**5.** The single-stranded antisense oligonucleotide or a pharmaceutically acceptable salt thereof according to any one of claims 1 to 4, wherein the base sequence of the single-stranded antisense oligonucleotide is:
a base sequence complementary to at least one target region having the same base length as the single-stranded antisense oligonucleotide present in the base sequence as set forth in SEQ ID NO: 1.

**6.** The single-stranded antisense oligonucleotide or a pharmaceutically acceptable salt thereof according to any one of claims 1 to 5, wherein

the gap region is composed of 5 to 20 bases,
the 3' wing region is a modified nucleic acid composed of 3 to 5 bases having a substituent at 2' position, and
the 5' wing region is a modified nucleic acid composed of 3 to 5 bases having a substituent at 2' position.

**7.** The single-stranded antisense oligonucleotide or a pharmaceutically acceptable salt thereof according to any one of claims 1 to 6, wherein the 5'-CP nucleic acid is at least located at position 2 counted from the 5' end of the gap region.

**8.** The single-stranded antisense oligonucleotide or a pharmaceutically acceptable salt thereof according to any one of claims 1 to 7, wherein the 5'-CP nucleic acid comprises two or more 5'-CP nucleic acids in the gap region.

**9.** The single-stranded antisense oligonucleotide or a pharmaceutically acceptable salt thereof according to any one of claims 1 to 8, wherein the 5'-CP nucleic acid comprises two to five 5'-CP nucleic acids in the gap region.

**10.** The single-stranded antisense oligonucleotide or a pharmaceutically acceptable salt thereof according to any one of claims 1 to 7, wherein the 5'-CP nucleic acid occupies one ninth or more of the gap region.

**11.** The single-stranded antisense oligonucleotide or a pharmaceutically acceptable salt thereof according to claim 10, wherein the 5'-CP nucleic acid occupies one fifth or more of the gap region.

**12.** The single-stranded antisense oligonucleotide or a pharmaceutically acceptable salt thereof according to any one of claims 1 to 11, wherein the 5'-CP nucleic acid comprises consecutive two to four 5'-CP nucleic acids located at at least one position in the gap region.

**13.** The single-stranded antisense oligonucleotide or a pharmaceutically acceptable salt thereof according to any one of claims 1 to 12, wherein the 5'-CP nucleic acid is located on the 5' end side in the gap region.

**14.** The single-stranded antisense oligonucleotide or a pharmaceutically acceptable salt thereof according to any one of claims 1 to 13, wherein the 5'-CP nucleic acid is located on the 5' end side and the 3' end side in the gap region.

**15.** The single-stranded antisense oligonucleotide or a pharmaceutically acceptable salt thereof according to any one of claims 1 to 14, wherein the single-stranded antisense oligonucleotide is composed of 15 to 20 bases.

**16.** The single-stranded antisense oligonucleotide or a pharmaceutically acceptable salt thereof according to any one of claims 1 to 15, wherein the single-stranded antisense oligonucleotide is composed of 18 to 20 bases.

**17.** The single-stranded antisense oligonucleotide or a pharmaceutically acceptable salt thereof according to any one of claims 1 to 16, wherein

the modified nucleic acid having a substituent at 2' position in the 3' wing region includes at least one selected from the group consisting of 2'-MOE nucleic acid, LNA, AmNA, GuNA, and scpBNA, and
the modified nucleic acid having a substituent at 2' position in the 5' wing region includes at least one selected from the group consisting of 2'-MOE nucleic acid, LNA, AmNA, GuNA, and scpBNA.

**18.** The single-stranded antisense oligonucleotide or a pharmaceutically acceptable salt thereof according to any one of claims 1 to 17, wherein

the modified nucleic acid having a substituent at 2' position in the 3' wing region is composed of a modified nucleic acid selected from the group consisting of 2'-MOE nucleic acid, LNA, AmNA, GuNA, and scpBNA, and
the modified nucleic acid having a substituent at 2' position in the 5' wing region is composed of a modified nucleic acid selected from the group consisting of 2'-MOE nucleic acid, LNA, AmNA, GuNA, and scpBNA.

**19.** The single-stranded antisense oligonucleotide or a pharmaceutically acceptable salt thereof according to any one of claims 1 to 18, wherein

the modified nucleic acid having a substituent at 2' position in the 3' wing region is composed of a modified nucleic acid selected from the group consisting of 2'-MOE nucleic acid, AmNA, GuNA, and scpBNA, and
the modified nucleic acid having a substituent at 2' position in the 5' wing region is composed of a modified nucleic acid selected from the group consisting of 2'-MOE nucleic acid, AmNA, GuNA, and scpBNA.

**20.** The single-stranded antisense oligonucleotide or a pharmaceutically acceptable salt thereof according to any one of claims 1 to 19, wherein at least one nucleoside-nucleoside bond in the single-stranded antisense oligonucleotide is a phosphorothioate bond.

**21.** The single-stranded antisense oligonucleotide or a pharmaceutically acceptable salt thereof according to any one of claims 1 to 20, wherein at least one nucleoside-nucleoside bond in the single-stranded antisense oligonucleotide is a phosphodiester bond.

**22.** The single-stranded antisense oligonucleotide or a pharmaceutically acceptable salt thereof according to any one of claims 1 to 21, wherein a proportion of phosphorothioate bonds among all of nucleoside-nucleoside bonds in the single-stranded antisense oligonucleotide is from 50% to 80%.

**23.** The single-stranded antisense oligonucleotide or a pharmaceutically acceptable salt thereof according to claim 22, wherein a proportion of phosphorothioate bonds among all of nucleoside-nucleoside bonds in the single-stranded antisense oligonucleotide is from 50% to 70%.

**24.** The single-stranded antisense oligonucleotide or a pharmaceutically acceptable salt thereof according to any one of claims 1 to 23, wherein in the gap region, a bond between the 5'-CP nucleic acid and a nucleoside located adjacent to a 3' end of the 5'-CP nucleic acid is a phosphorothioate bond (except for a case where the 5'-CP nucleic acid is located at the 3' end of the gap region).

**25.** The single-stranded antisense oligonucleotide or a pharmaceutically acceptable salt thereof according to any one of claims 1 to 24, wherein in the gap region, a bond between the 5'-CP nucleic acid and a nucleoside located adjacent to a 3' end of the 5'-CP nucleic acid is a phosphorothioate bond (except for a case where the 5'-CP nucleic acid is located at the 3' end of the gap region and a case where the nucleoside located adjacent to the 3' end of the 5'-CP nucleic acid is a 5'-CP nucleic acid).

**26.** The single-stranded antisense oligonucleotide or a pharmaceutically acceptable salt thereof according to any one of claims 1 to 25, wherein in the gap region, a bond between the 5'-CP nucleic acid and a nucleoside located adjacent to a 5' end of the 5'-CP nucleic acid is a phosphodiester bond.

**27.** The single-stranded antisense oligonucleotide or a pharmaceutically acceptable salt thereof according to any one of claims 1 to 26, wherein the base sequence of the single-stranded antisense oligonucleotide is:

a base sequence with a sequence identity of 90% to 100% to a base sequence complementary to a target region present in the base sequence as set forth in SEQ ID NO: 1 composed of consecutive 14 to 22 bases starting from a base located at one of position 123, position 124, position 185, position 186, position 263, position 264, position 324, position 325, position 442, position 443, and position 447 to position 453 counted from a 5' end;
a base sequence complementary to a base sequence of the target region with deletion, substitution, insertion, or addition of one or several bases; or
a base sequence capable of hybridizing under stringent conditions with an oligonucleotide having the target region.

**28.** The single-stranded antisense oligonucleotide or a pharmaceutically acceptable salt thereof according to any one of claims 1 to 27, wherein the base sequence of the single-stranded antisense oligonucleotide is:

a base sequence with a sequence identity of 90% to 100% to a base sequence complementary to a target region present in the base sequence as set forth in SEQ ID NO: 1 composed of consecutive 14 to 22 bases starting from a base located at one of position 324, position 325, and position 448 to position 453 counted from a 5' end;
a base sequence complementary to a base sequence of the target region with deletion, substitution, insertion, or addition of one or several bases; or
a base sequence capable of hybridizing under stringent conditions with an oligonucleotide having the target region.

**29.** The single-stranded antisense oligonucleotide or a pharmaceutically acceptable salt thereof according to any one of claims 1 to 28, wherein

the base sequence of the single-stranded antisense oligonucleotide is a base sequence with a sequence identity of 90% to 100% to a base sequence complementary to a target region present in the base sequence as set forth in SEQ ID NO: 1 composed of consecutive 18 to 22 bases starting from a base located at one of position 448 to position 453 counted from a 5' end,
the 3' wing region is composed of 3 to 5 bases, and
the 5' wing region is composed of 3 to 5 bases.

**30.** The single-stranded antisense oligonucleotide or a pharmaceutically acceptable salt thereof according to any one of claims 1 to 29, wherein the base sequence of the single-stranded antisense oligonucleotide is a base sequence with a sequence identity of 90% to 100% to a base sequence complementary to a target region present in the base sequence as set forth in SEQ ID NO: 1 composed of consecutive 18 to 20 bases starting from a base located at one of position 450 to position 451 and position 453 counted from a 5' end.

**31.** The single-stranded antisense oligonucleotide or a pharmaceutically acceptable salt thereof according to any one of claims 1 to 30, wherein the base sequence of the single-stranded antisense oligonucleotide is a base sequence selected from the group consisting of base sequences as set forth in SEQ ID NOs: 5 to 47 and 49 to 56.

**32.** The single-stranded antisense oligonucleotide or a pharmaceutically acceptable salt thereof according to any one of claims 1 to 31, wherein the base sequence of the single-stranded antisense oligonucleotide is a base sequence selected from the group consisting of base sequences as set forth in SEQ ID NOs: 6, 9, 12, 15 to 18, 22, 24, 27 to 29, 31 to 36, 38, 40 to 42, 47, and 49 to 54.

**33.** The single-stranded antisense oligonucleotide or a pharmaceutically acceptable salt thereof according to any one of claims 1 to 32, wherein the base sequence of the single-stranded antisense oligonucleotide is a base sequence selected from the group consisting of base sequences as set forth in SEQ ID NOs: 41, 47, 50, and 53 to 55.

**34.** An antisense oligonucleotide complex, or a pharmaceutically acceptable salt thereof, comprising:

the single-stranded antisense oligonucleotide or a pharmaceutically acceptable salt thereof according to any one of claims 1 to 33; and
an additional substance bonded to the single-stranded antisense oligonucleotide, wherein
the additional substance is selected from the group consisting of a polyethylene glycol, a peptide, an alkyl chain, a nucleic acid, a ligand compound, an antibody, a protein, and a sugar chain.

**35.** A pharmaceutical comprising the single-stranded antisense oligonucleotide or a pharmaceutically acceptable salt thereof according to any one of claims 1 to 33 or the antisense oligonucleotide complex or a pharmaceutically acceptable salt thereof according to claim 34, as an active ingredient.

**36.** The pharmaceutical according to claim 35, wherein the single-stranded antisense oligonucleotide or a pharmaceutically acceptable salt thereof or the antisense oligonucleotide complex or a pharmaceutically acceptable salt thereof is administered in such a manner that the central nervous system is exposed thereto.

**37.** The pharmaceutical according to claim 35 or 36, wherein the single-stranded antisense oligonucleotide or a pharmaceutically acceptable salt thereof or the antisense oligonucleotide complex or a pharmaceutically acceptable

salt thereof is administered to a subject susceptible to delayed central toxicity.

**38.** An agent for modulating expression and/or function of RPS25 gene, comprising the single-stranded antisense oligonucleotide or a pharmaceutically acceptable salt thereof according to any one of claims 1 to 33 or the antisense oligonucleotide complex or a pharmaceutically acceptable salt thereof according to claim 34, as an active ingredient.

**39.** An agent for inhibiting dipeptide repeat production attributable to RAN translation, comprising the single-stranded antisense oligonucleotide or a pharmaceutically acceptable salt thereof according to any one of claims 1 to 33 or the antisense oligonucleotide complex or a pharmaceutically acceptable salt thereof according to claim 34, as an active ingredient.

**40.** An agent for treating a repeat disease, comprising the single-stranded antisense oligonucleotide or a pharmaceutically acceptable salt thereof according to any one of claims 1 to 33 or the antisense oligonucleotide complex or a pharmaceutically acceptable salt thereof according to claim 34, as an active ingredient.

**41.** An agent for preventing a repeat disease, comprising the single-stranded antisense oligonucleotide or a pharmaceutically acceptable salt thereof according to any one of claims 1 to 33 or the antisense oligonucleotide complex or a pharmaceutically acceptable salt thereof according to claim 34, as an active ingredient.

**42.** The agent for treating a repeat disease according to claim 40, or the agent for preventing a repeat disease according to claim 41, wherein the repeat disease is at least one selected from the group consisting of C9orf72 ALS, C9orf72 FTLD, Huntington's disease, spinocerebellar ataxia, dentatorubral-pallidoluysian atrophy, spinal and bulbar muscular atrophy, Friedreich ataxia, fragile X-associated tremor/ataxia syndrome, and myotonic dystrophy.

**43.** A method of treating or preventing a repeat disease, comprising administering the single-stranded antisense oligonucleotide or a pharmaceutically acceptable salt thereof according to any one of claims 1 to 33 or the antisense oligonucleotide complex or a pharmaceutically acceptable salt thereof according to claim 34, to an individual.

**44.** The method of treating or preventing a repeat disease according to claim 43, wherein the repeat disease is at least one selected from the group consisting of C9orf72 ALS, C9orf72 FTLD, Huntington's disease, spinocerebellar ataxia, dentatorubral-pallidoluysian atrophy, spinal and bulbar muscular atrophy, Friedreich ataxia, fragile X-associated tremor/ataxia syndrome, and myotonic dystrophy.

**45.** The single-stranded antisense oligonucleotide or a pharmaceutically acceptable salt thereof according to any one of claims 1 to 33 or the antisense oligonucleotide complex or a pharmaceutically acceptable salt thereof according to claim 34, for use for treating or preventing C9orf72 ALS.

**46.** The single-stranded antisense oligonucleotide or a pharmaceutically acceptable salt thereof according to any one of claims 1 to 33 or the antisense oligonucleotide complex or a pharmaceutically acceptable salt thereof according to claim 34, for use for producing an agent for treating or preventing C9orf72 ALS.

FIG.1

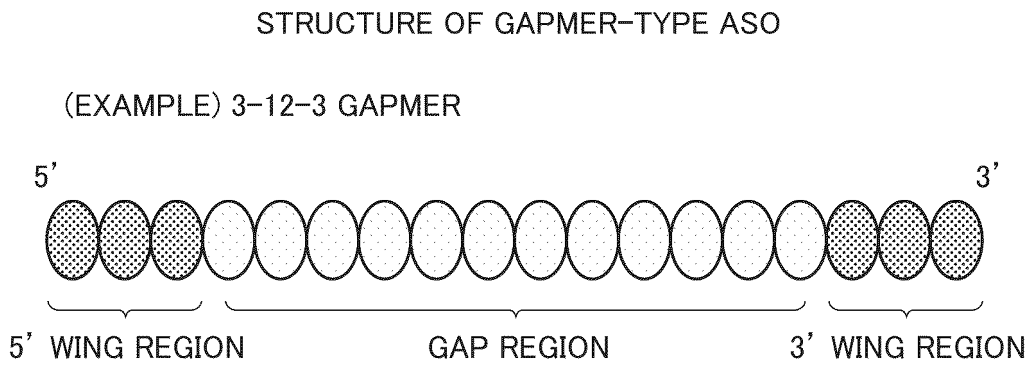
STRUCTURE OF GAPMER-TYPE ASO
(EXAMPLE) 3-12-3 GAPMER
5'
3'
5' WING REGION
GAP REGION
3' WING REGION

FIG.2

MECHANISM OF TARGET RNA DEGRADATION BY GAPMER-TYPE ASO

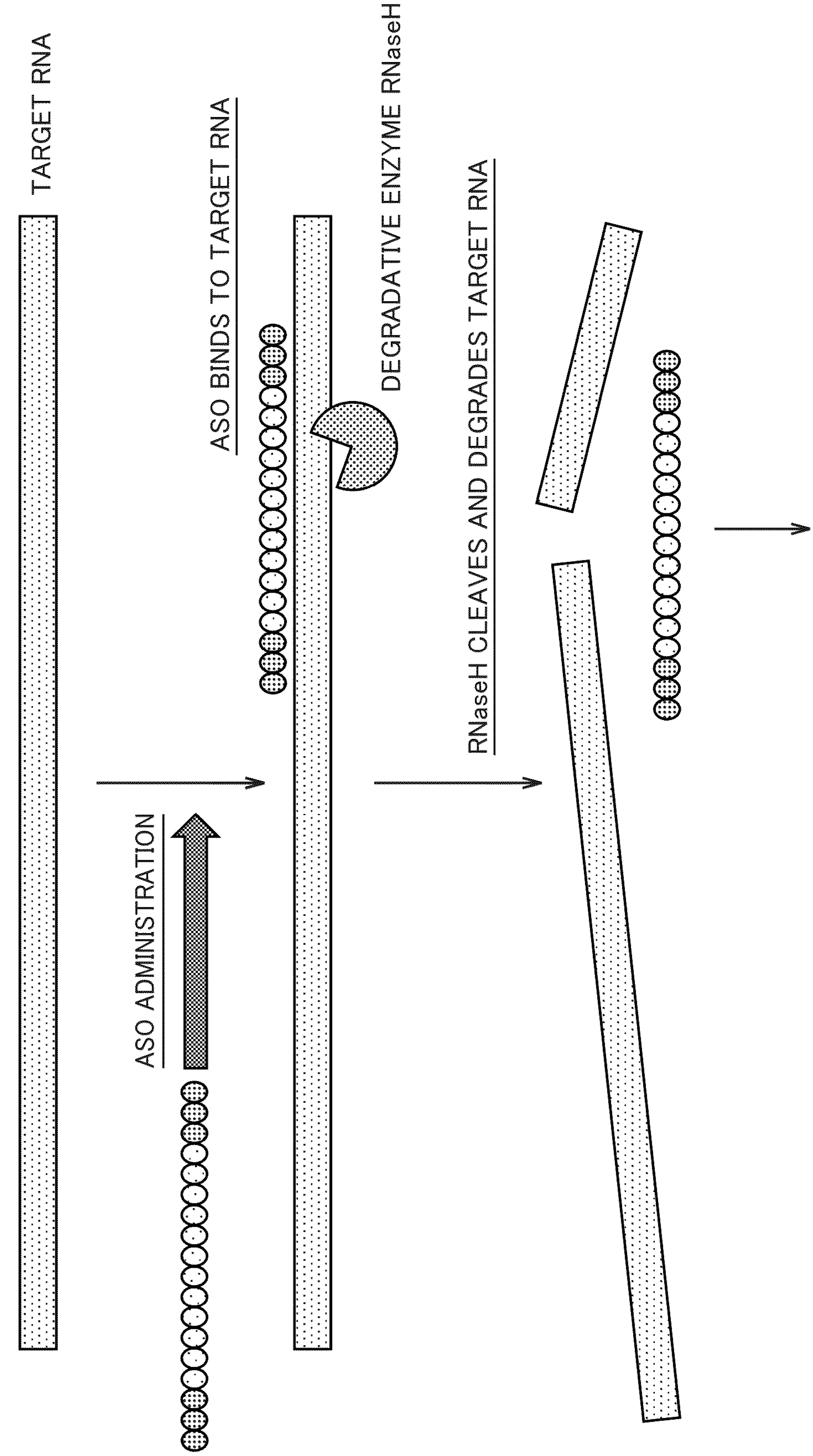

**INTERNATIONAL SEARCH REPORT**

International application No.
**PCT/JP2024/021613**

**A. CLASSIFICATION OF SUBJECT MATTER**

***C12N 15/113***(2010.01)i; ***A61K 31/7088***(2006.01)i; ***A61K 47/54***(2017.01)i; ***A61K 47/60***(2017.01)i; ***A61K 47/61***(2017.01)i; ***A61K 47/64***(2017.01)i; ***A61K 47/68***(2017.01)i; ***A61K 48/00***(2006.01)i; ***A61P 21/00***(2006.01)i; ***A61P 25/00***(2006.01)i; ***A61P 25/14***(2006.01)i; ***A61P 25/18***(2006.01)i; ***A61P 43/00***(2006.01)i

FI: C12N15/113 Z ZNA; A61K31/7088; A61K47/54; A61K47/60; A61K47/61; A61K47/64; A61K47/68; A61K48/00; A61P21/00; A61P25/00; A61P25/14; A61P25/18; A61P43/00 111

According to International Patent Classification (IPC) or to both national classification and IPC

**B. FIELDS SEARCHED**

Minimum documentation searched (classification system followed by classification symbols)

C12N15/113; A61K31/7088; A61K47/54; A61K47/60; A61K47/61; A61K47/64; A61K47/68; A61K48/00; A61P21/00; A61P25/00; A61P25/14; A61P25/18; A61P43/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

CAplus/MEDLINE/EMBASE/BIOSIS (STN), JSTPlus/JMEDPlus/JST7580 (JDreamIII), PubMed, GenBank/EMBL/DDBJ/GeneSeq, Japio-GPG/FX

**C. DOCUMENTS CONSIDERED TO BE RELEVANT**

| Category* | Citation of document, with indication, where appropriate, of the relevant passages | Relevant to claim No. |
|---|---|---|
| X | WO 2022/097727 A1 (SUMITOMO PHARMA CO., LTD.) 12 May 2022 (2022-05-12) claims 1, 17-26, SEQ ID NO: 1, examples | 1-46 |
| A | WO 2022/234855 A1 (LUXNA BIOTECH CO., LTD.) 10 November 2022 (2022-11-10) entire text, all drawings | 1-46 |
| A | YAMADA, S. et al. RPS25 is required for efficient RAN translation of C9orf72 and other neurodegenerative disease-associated nucleotide repeats. Nature Neuroscience. 2019, vol. 22, no. 9, pp. 1383-1388 entire text, all drawings | 1-46 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **19 August 2024** | **27 August 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**

International application No.
**PCT/JP2024/021613**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

**INTERNATIONAL SEARCH REPORT**
**Information on patent family members**

International application No.
**PCT/JP2024/021613**

| Patent document cited in search report | Publication date (day/month/year) | Patent family member(s) | Publication date (day/month/year) |
|---|---|---|---|
| WO 2022/097727 A1 | 12 May 2022 | EP 4252846 A1<br>claims 1, 17-26, SEQ ID NO: 1, examples<br>US 2024/0018516 A1 | |
| WO 2022/234855 A1 | 10 November 2022 | EP 4335859 A1<br>entire text, all drawings | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2019084068 A
- WO 2011052436 A
- WO 2014046212 A
- WO 2015125783 A
- WO 2020158910 A
- WO 9914226 A
- WO 2022097727 A
- WO 2016127000 A
- WO 2022234855 A


### Non-patent literature cited in the description


- *Science*, 2011, vol. 331 (6018), 730-736
- *Genes Dev.*, 2009, vol. 23 (23), 2753-2764
- *Proc. Natl. Acad. Sci. USA*, 2011, vol. 108 (1), 260-265
- *Neuron*, 2015, vol. 88, 667-677
- *Neuron*, 2020, vol. 105, 645-662
- *Nat. Neurosci.*, 2019, vol. 22 (9), 1383-1388
- *Mol. Ther. Nucleic Acids*, 2023, vol. 31, 182-196
- *BioRxiv*, 2021
- *Mol. Gen. Genet.*, 1979, vol. 169, 1-6
- *Curr. Opin. Struct. Biol.*, 2014, vol. 24, 165-169
- *J. Med. Chem.*, 2016, vol. 59, 9645-9667
- *Nature*, 2015, vol. 518 (7539), 409-412
- RPS25 gene. *Mol. Gen. Genet.*, 1979, vol. 169, 1-6
- **W. BRAD WAN**. *Al. J. Med. Chem.*, 2016, vol. 59, 9645-9667
- *Nature*, 2015, vol. 518 (7539), 409-12